# FASCICULE DE BREVET EUROPEEN

(11) **EP 3 326 229 B1**
(45) Date de publication et mention de la délivrance du brevet: **27.05.2020**
(21) Numéro de dépôt: 16753948.5
(22) Date de dépôt: 21.07.2016
(51) Int. Cl.: H01M 10/052, H01M 10/0565, C08G 65/40, C08G 65/48

(54) **PROCÉDÉ DE PRÉPARATION D'IONOMÈRES À CONDUCTION CATIONIQUE UNIPOLAIRE À PARTIR DE MONOMÈRES IONIQUES DIFLUORÉS**
VERFAHREN ZUR HERSTELLUNG VON IONOMEREN FÜR EINPOLIGE KATIONISCHE LEITFÄHIGKEIT AUS IONISCHEN DIFLUORIDMONOMEREN
PROCESS FOR PREPARING IONOMERS OF UNIPOLAR CATIONIC CONDUCTIVITY FROM DIFLUORIDE IONIC MONOMERS

(30) Priorité: 22.07.2015 FR 1556967
(43) Date de publication de la demande: 30.05.2018
(73) Titulaire: Centre National de la Recherche Scientifique, 75016 Paris (FR); Institut Polytechnique de Grenoble, 38031 Grenoble Cedex (FR)
(72) Inventeur: IOJOU, Cristina, 38210 Vourey (FR); THIAM, Amadou, 38100 Grenoble (FR); DANYLIV, Olesia, 43168 Mölndal (UA); MERCIER, Régis, 69540 Irigny (FR); SANCHEZ, Jean-Yves, 38330 Saint-Ismier (FR)
(74) Mandataire: Lavoix
(86) Numéro de dépôt international: PCT/FR2016/051905
(87) Numéro de publication internationale: WO 2017/013375

(56) Documents cités:
- WO-A1-2014/022224
- WO-A2-00/77057
- US-A1- 2008 114 183
- US-A1- 2009 163 692
- KUI XU ET AL: "Highly Conductive Aromatic Ionomers with Perfluorosulfonic Acid Side Chains for Elevated Temperature Fuel Cells", MACROMOLECULES, vol. 44, no. 12, 28 juin 2011 (2011-06-28) , pages 4605-4609, XP055137602, ISSN: 0024-9297, DOI: 10.1021/ma201188e
- JIANG RUICHUN ET AL: "Perfluorocyclobutane and poly(vinylidene fluoride) blend membranes for fuel cells", ELECTROCHIMICA ACTA, vol. 110, 2 août 2013 (2013-08-02), pages 306-315, XP028767662, ISSN: 0013-4686, DOI: 10.1016/J.ELECTACTA.2013.07.074
- ANDREW E FEIRING ET AL: "Novel Aromatic Polymers with Pendant Lithium Perfluoroalkylsulfonate or Sulfonimide Groups", MACROMOLECULES, AMERICAN CHEMICAL SOCIETY, US, vol. 33, no. 25, 1 janvier 2000 (2000-01-01), pages 9262-9271, XP009098090, ISSN: 0024-9297, DOI: 10.1021/MA000893F
- MCLOUGHLIN V C R ET AL: "A ROUTE TO FLUOROALKYL-SUBSTITUTED AROMATIC COMPOUNDS INVOLVING FLUOROALKYLCOPPER INTERMEDIATES", TETRAHEDRON, ELSEVIER SCIENCE PUBLISHERS, AMSTERDAM, NL, vol. 25, no. 24, 1 janvier 1969 (1969-01-01), pages 5921-5940, XP001205218, ISSN: 0040-4020, DOI: 10.1016/S0040-4020(01)83100-8

## Description

L'invention concerne un procédé de préparation d'ionomères à conduction cationique unipolaire à partir de monomères ioniques fluorés, lesdits ionomères à conduction cationique unipolaire, leurs utilisations, une composition électrolyte comprenant au moins un desdits ionomères à conduction cationique unipolaire et un dispositif électrochimique comprenant au moins un desdits ionomères à conduction cationique unipolaire, notamment à titre d'électrolyte.

Les dispositifs de stockage électrochimique de l'énergie tels que les batteries au lithium sont devenus des constituants indispensables dans des appareils portables tels que les téléphones mobiles, les ordinateurs et l'outillage léger, ou des appareils plus lourds tels que des moyens de transports à deux roues (bicyclettes, cyclomoteurs) ou à quatre roues (véhicules automobiles électriques ou hybrides). Ils sont également largement étudiés pour une utilisation dans le domaine du stockage stationnaire d'énergie.

Les divers constituants d'un dispositif électrochimique (e.g. matériaux d'électrodes et/ou électrolyte) sont choisis de manière à produire, au coût le plus faible possible, des dispositifs qui ont une densité d'énergie élevée, une bonne stabilité au cyclage et qui fonctionnent avec sécurité.

En termes de batteries rechargeables, les batteries au lithium sont celles dont les densités d'énergie théoriques sont les plus élevées. En outre, dans le domaine de l'électronique portable, des batteries capables de fonctionner à des températures aussi basses que -20°C (253 K) sont requises. Pour ce faire, des batteries fonctionnant avec des électrolytes liquides constituées de mélanges de carbonates cycliques, comme le carbonate d'éthylène (EC), et de carbonates acycliques comme le diméthyl-carbonate (DMC), le diéthyl-carbonate (DEC) ou l'éthyl-méthyl carbonate (EMC) ont été développées. Le sel majoritairement utilisé est l'hexafluorophosphate de lithium (LiPF₆) et est généralement utilisé à une concentration molaire de 1 mol/l environ. Plusieurs électrolytes liquides sont commerciaux tels le LP30® qui est une solution à 1M de LiPF₆ dans un mélange 1/1 de EC/DMC. Les réactions des électrolytes liquides avec le lithium métal, Li⁰, ne permettent pas d'obtenir un nombre de cycles de charge/décharge suffisant dans les batteries au lithium utilisant des électrodes négatives au Li⁰. Le remplacement de Li⁰ par le graphite lithié, LiC₆ dans le début des années 1990 a permis le développement des batteries lithium-ion. EC et LiPF₆ permettent la formation d'une SEI (Solid Electrolyte Interface) qui a pour effet de cycler efficacement l'électrode de graphite. Les électrolytes liquides précédemment cités sont alors utilisés avec des séparateurs macroporeux en polyoléfine tels que ceux commercialisés sous la référence Solupor®, à base de polyéthylène, ou Celgard® (e.g. Celgard 2400), à base de polypropylène. La phase poreuse remplie d'électrolyte liquide assure alors la conductivité ionique, tandis que la phase polymère assure la tenue mécanique empêchant des courts-circuits par contact entre les électrodes. L'introduction d'un séparateur induit une chute de conductivité ionique d'un facteur allant de 5 à 20 par rapport à celle obtenue en présence d'un électrolyte liquide seul (i.e. sans séparateur). Par ailleurs, la faible affinité de l'électrolyte liquide, de nature hydrophile, avec le séparateur, de nature hydrophobe, ne permet pas une très bonne rétention de l'électrolyte liquide dans le séparateur, ne favorisant pas la formation de batteries en film mince. En outre, bien que les électrolytes liquides soient bien adaptés aux basses températures, leur stabilité à haute température est plus problématique. Ainsi l'instabilité thermique de LiPF₆ à partir de 50-55°C est bien connue. Il est possible d'utiliser des sels de lithium beaucoup plus stables en température que LiPF₆ comme LiTFSI ou le triflate de lithium mais ils induisent une corrosion du collecteur de courant en aluminium de l'électrode positive à un potentiel supérieur ou égal à 3,6 V vs Li/Li⁺. Il est possible de pallier à cet inconvénient en recourant à des solvants partiellement fluorés qui augmentent le prix de la batterie. Des liquides ioniques à très haute stabilité thermique donnent d'une part, des performances en batterie inférieures à celles des électrolytes liquides et d'autre part, sont très coûteux. Par conséquent, le coût des batteries lithium-ion et les problèmes de sécurité compromettent actuellement le développement de véhicules électriques alimentés par de telles batteries lithium-ion.

Une seconde catégorie d'électrolytes permet d'utiliser des électrodes négatives de lithium métal Li⁰, ce sont les électrolytes polymères qui sont des solutions de sels dans des solvants macromoléculaires solvatants de type poly(oxyéthylène) ou matrice polymère polyéther. Ces électrolytes polymères, qui ne nécessitent pas l'utilisation d'un séparateur, répondent en grande partie au cahier des charges de la traction électrique et des applications stationnaires. Par ailleurs, ils peuvent être utilisés avec des électrodes au lithium métal Li⁰ tout en évitant ou en diminuant la croissance dendritique généralement observée en présence d'un électrolyte liquide. Toutefois, les batteries au lithium utilisant des électrolytes polymères sans incorporation de solvants liquides, ne sont pas adaptées aux besoins de l'électronique portable.

La valeur du nombre de transport cationique (e.g. de l'ion Li⁺ dans un accumulateur au lithium) détermine la part de courant transporté par le cation. Dans l'électrolyte, le sel de lithium qui y est partiellement dissocié assure la conductivité ionique. Cette dissociation partielle induit des équilibres entre d'une part les espèces associées (i.e. paires d'ions au contact), qui sont électriquement neutres et ne contribuent pas à la conductivité de l'électrolyte et, d'autre part, les paires d'ions séparées par le solvant et les ions libres qui déterminent la conductivité des électrolytes. Lorsqu'un courant continu est appliqué à travers l'électrolyte, le courant est initialement transporté par les anions et les cations du sel dissocié. Les électrodes étant bloquantes pour les anions, à l'état stationnaire, le déplacement des anions par diffusion contrebalance leur déplacement par migration. Seul le cation Li⁺ devient alors responsable du transport du courant. Un nombre de transport cationique proche de l'unité permet de réduire la polarisation aux électrodes pendant les étapes de charge et de décharge rapides et ainsi d'obtenir une plus grande densité d'énergie et de puissance.

Or, dans les électrolytes polymères constitués d'un sel de lithium dissous dans une matrice polymère polyéther, la fraction de la charge portée par les ions lithium est faible (<20% et de l'ordre de 10% pour les électrolytes polymères les plus conducteurs).

Doyle et al. [Electrochimica Acta, 1994, 39] ont calculé qu'un électrolyte ayant un nombre de transport égal à 1 permettait d'obtenir de meilleurs performances en batterie qu'un électrolyte ayant un nombre de transport de 0,2 ; même si sa conductivité totale chutait d'un facteur 10. Dans le cas de fortes densités de courant, la croissance dendritique débute au moment où la concentration en lithium chute à zéro à la surface de l'électrode. Puis, les dendrites croissent à une vitesse proportionnelle à la mobilité anionique [Brissot et al., J. Power Sources, 1999, 81-82, 925-929 et Rosso et al., J. Power Sources, 2001, 97-98, 804-806].

Dans les électrolytes liquides, le nombre de transport cationique est plus élevé que dans les électrolytes polymères et les liquides ioniques (il se situe majoritairement entre 0,3 et 0,4). Toutefois, il reste inférieur à 1 et est handicapant à fort régime de charge/décharge.

Il est donc essentiel de parvenir à augmenter significativement le nombre de transport cationique et de tendre vers la valeur de 1.

Ainsi, Benrabah et al. [Electrochim. Acta, 1995, 40, 2259-2264] ont décrit le greffage d'un anion sulfonate perfluoré DaaR_{F}SO₃Li (i.e. lithium N,N-diallyl-1-amido-tétrafluoroéthanesulfonate) répondant à la formule suivante : 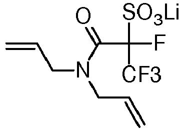 sur des polymères de type polyéthers réticulés pour former un ionomère à conduction cationique (i.e. le courant est transporté majoritairement par les cations Li⁺) ayant ainsi un nombre de transport cationique proche de 1. Toutefois, l'électrolyte polymère obtenu présente une conductivité ionique faible d'environ 4 x 10⁻⁵ S.cm⁻¹ à 60°C. D'autre part, la méthode d'introduction du sel lors de la phase de réticulation se traduit par des rendements de greffage de l'ordre de 50%, ce qui, compte-tenu du coût du sel, augmente significativement le coût de production du ionomère obtenu. Enfin, le sel qui n'a pas été greffé doit être extrait et éliminé (e.g. avec des solvants) car ses doubles liaisons allyliques et sa fonction amide réduisent sa fenêtre de stabilité électrochimique, respectivement en oxydation et en réduction, générant là encore un surcoût de production et rendant l'application industrielle problématique.

Plus récemment, Xu et al. [Chem. Mater., 2002, 14, 401-409] ont décrit un électrolyte polymère P(LiOEG_{q1}B) de la famille des oxalato-orthoborates répondant à la formule suivante :

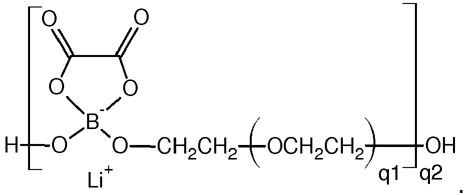

Cependant, le LIBOB est peu soluble dans les solvants classiques (0,5 mole/l alors que les électrolytes classiques utilisent généralement le sel à une concentration proche de 1 mole/l). En outre, ce sel est sensible à l'eau. Une fois incorporé au squelette polyéther par polycondensation, l'ionomère obtenu garde une forte sensibilité à l'hydrolyse. Par ailleurs, les familles d'électrolytes polymères à base d'oxalato- ou malonato- orthoborates sont généralement difficiles à purifier, les électrolytes préparés contenant toujours du bis(oxalato)borate de lithium (LiBOB) même après de multiples lavages. La conductivité ionique du P(LiOEGₙB) est d'environ 1 x 10⁻⁵ S.cm⁻¹ à température ambiante. Cependant, le calcul de cette conductivité ionique ne tient pas compte de la teneur résiduelle en bis(oxalato)borate de lithium, les résultats de conductivité obtenus sont donc très probablement surestimés et la conductivité ionique diminue nettement lorsque la valeur de n est élevée (e.g. n ≥ 16). Enfin, les ionomères obtenus n'ont aucune tenue mécanique.

WO 2014/022224 décrit des ionomères comme PAE-LiPFS utilisés dans des compositions d'électrolyte et leur fabrication, les monomères de ces ionomères comprenant -OCF₂CF₂SO₃Li et un groupe phényle au moins difluoré.

Kui Xu et al. [Macromolecules, 2011, 44, 4605-4609] décrit des ionomères utilisés dans des piles à combustible et leur fabrication, les monomères de ces ionomères comprenant -OCF₂CF₂SO₃Na et un groupe phényle tétrafluoré.

US 2009/163692 décrit des polyéthers aromatiques utilisés dans les compositions d'électrolyte et leur fabrication à partir de biphénol et des monomères en milieu basique, les monomères desdits polyéthers comprenant -SO₂-(CF₂)₂₋₄SO₃Li et un groupe phényle difluoré.

US 2008/114183 décrit des ionomères utilisés comme électrolyte polymère et leur fabrication, les monomères desdits ionomères comprenant -OCF₂CF₂SO₃Na ou -(CF₂)₂O(CF₂)SO₃H et un ou trois cycles aromatiques de 6 ou 20 atomes de carbone avec deux groupes -OH.

Jiang Ruichun et al. [Electrochimica Acta, 2013, 110, 306-315] décrit des polyéthers utilisés comme électrolyte polymère comprenant des unités structurelles -O-phényl[(CF₂)₂O(CF₂)₂SO₃H]-O- et cyclobutane perfluoré.

WO 00/77057 décrit un polyéthylène substitué avec -(CF₂)₂O(CF₂)₂SO₂N⁻SO₂CF₃ utilisé comme électrolyte polymère.

Andrew Feiring et al. [Macromolecules, 2000, 33, 9262-9271] décrit un polyéthylène glycol avec -O(CF₂)₂SO₃Li et E étant -[OCH₂CH(CH₂O-alkyl)-phényle]- ou -CO-phényle-CO- utilisé comme électrolyte polymère.

Mcloughlin et al. [Tetrahedron, 1969, 25, 5921-5940] décrit la synthèse de C₆H₅(CF₂)₃CO₂H à partir de C₆H₅I et I(CF₂)₃CO₂H.

Le but de la présente invention est de pallier tout ou partie des inconvénients de l'art antérieur précité et de fournir des ionomères à conduction cationique unipolaire présentant de bonnes propriétés en termes de conduction ionique, de nombre de transport cationique et de tenue mécanique, notamment ayant un nombre de transport cationique proche de 1, lesdits ionomères pouvant être utilisés en toute sécurité dans un dispositif électrochimique.

Ce but est atteint par l'invention qui est exposée dans le jeu de revendications joint.

Un procédé de préparation d'un ionomère divulgué comprenant au moins des unités récurrentes UP répondant à la formule (II) suivante : 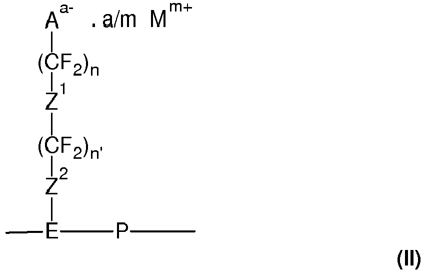 dans laquelle :
- M est un cation de métal alcalin, un cation de métal alcalino-terreux, un cation de métal de transition, un cation de métal pauvre, un ammonium, un sulfonium ou un phosphonium de valence m, avec 1 ≤ m ≤ 3, m étant un nombre entier,
- A^{a-} est un anion choisi parmi un anion sulfonate, un anion sulfonimidure de formule -SO₂-N⁻-SO₂R, un anion dérivé d'un anion sulfonimidure portant au moins deux charges négatives, et un carbanion de formule -SO₂-C⁻R'R", avec 1 ≤ a ≤ 3, a étant un nombre entier,
   * avec R représentant un atome de fluor ; un groupe alkyle éventuellement fluoré ou perfluoré, ayant de 1 à 10 atomes de carbone, ledit groupe alkyle pouvant porter au moins un groupe électroattracteur X¹ ; un groupe alkoxy éventuellement fluoré ou perfluoré, ayant de 1 à 10 atomes de carbone, ledit groupe alkoxy pouvant porter au moins un groupe électroattracteur X² ; un groupe phénoxy éventuellement substitué par un groupe électroattracteur X² ; un groupe éther de dialkyle éventuellement fluoré ou perfluoré, ayant de 1 à 10 atomes de carbone ; un groupe thiocyanate (-SCN) ; un groupe phényle éventuellement substitué ; un groupe nitrile (-CN) ; un groupe amino de formule -NR¹R², dans laquelle R¹ et R² sont choisis, indépendamment l'un de l'autre, parmi les groupes suivants : un groupe alkyle éventuellement fluoré ou perfluoré, ayant de 1 à 5 atomes de carbone, un groupe alkyle ayant de 1 à 5 atomes de carbone et portant un groupe électroattracteur X³, un groupe éther de dialkyle éventuellement fluoré ou perfluoré, ayant de 1 à 5 atomes de carbone, et un groupe électroattracteur X⁴ ; un groupe -NR³ étant choisi parmi un hétérocycle saturé ayant de 3 à 6 atomes de carbone et un hétérocycle insaturé ayant de 4 à 6 atomes de carbone ; un groupe amide de formule -NH-CO-R⁴ ou -N(CH₃)-CO-R⁴, dans laquelle R⁴ est un groupe alkyle ayant de 1 à 3 atomes de carbone ; un groupe sulfonamide de formule -NH-SO₂-R⁵ ou -N(CH₃)-SO₂-R⁵, dans laquelle R⁵ est un groupe alkyle ayant de 1 à 3 atomes de carbone ; un groupe uréthane de formule -NH-CO₂- R⁶ ou -N(CH₃)-CO₂- R⁶, dans laquelle R⁶ est un groupe alkyle ayant de 1 à 3 atomes de carbone ; un groupe cyanamide de formule -NH-CN ou -N(R⁷)-CN, dans laquelle R⁷ est un groupe alkyle ayant 1 à 3 atomes de carbone ; un groupe dicyanamide -N(CN)₂ ; un groupe tricyanométhyle -C(CN)₃ ; ou un groupe dicyanométhylène de formule -CH(CN)₂ ou -CR⁸(CN)₂, dans laquelle R⁸ est un groupe alkyle ayant 1 à 3 atomes de carbone,
   * avec R' et R" étant choisis, indépendamment l'un de l'autre, parmi les groupes monovalents suivants : un atome de fluor ; un groupe thiocyanate ; un groupe nitrile ; un groupe nitro ; un groupe nitroso de formule R⁹NO-, dans laquelle R⁹ est un groupe alkyle ayant de 1 à 3 atomes de carbone ; un groupe carbonyle de formule -COR¹⁰ dans laquelle R¹⁰ est un groupe alkyle perfluoré ayant de 1 à 5 atomes de carbone ; un groupe sulfoxyde de formule -SOR¹¹ dans laquelle R¹¹ est un groupe alkyle éventuellement fluoré ou perfluoré, ayant de 1 à 5 atomes de carbone ou un groupe éther de dialkyle éventuellement fluoré ou perfluoré, ayant de 1 à 5 atomes de carbone ; un groupe sulfonyle de formule -SO₂R¹² dans laquelle R¹² est un atome de fluor, un groupe thiocyanate, un groupe nitrile, un groupe alkoxy éventuellement fluoré ou perfluoré, ayant de 1 à 5 atomes de carbone, un groupe alkyle éventuellement fluoré ou perfluoré, ayant de 1 à 5 atomes de carbone ou un groupe éther de dialkyle éventuellement fluoré ou perfluoré, ayant de 1 à 5 atomes de carbone ; un groupe ester carboxylique de formule -COOR¹³, dans laquelle R¹³ est un groupe alkyle ayant de 1 à 5 atomes de carbone ; un groupe amide de formule -CONHR¹⁴ dans laquelle R¹⁴ est un groupe alkyle ayant de 1 à 5 atomes de carbone ; un groupe amide de formule -CONR¹⁴R¹⁵ dans laquelle R¹⁴ et R¹⁵ sont choisis, indépendamment l'un de l'autre, et R¹⁵ est un groupe alkyle ayant de 1 à 5 atomes de carbone ; un groupe phényle éventuellement substitué ; ou un groupe phénoxy éventuellement substitué, ou
   * avec R' et R" étant des groupes divalents de telle sorte que le radical carbanion -C⁻R'R" résultant forme un cycle aromatique comprenant de 5 à 6 atomes de carbone et optionnellement un ou plusieurs des hétéroatomes O ou N, ledit cycle aromatique étant éventuellement substitué par un ou plusieurs groupes nitriles,
- 1 ≤ n ≤ 4, et de préférence 1 ≤ n ≤ 2, et de préférence encore n = 2, n étant un nombre entier
- 0 ≤ n' ≤ 2, n' étant un nombre entier
- Z¹ est choisi parmi une liaison simple, un atome d'oxygène, un atome de soufre, un groupe S=O, un groupe S(=O)₂ et un groupe phényle éventuellement substitué en position ortho par rapport à l'une des fonctions (CF₂)ₙ ou (CF₂)_{n'},
- Z² est choisi parmi une liaison simple, un atome d'oxygène, un atome de soufre, un groupe S=O, un groupe S(=O)₂ et un groupe C=O, étant entendu que lorsque n' = 0, Z² est une liaison simple,
- E est un groupe aromatique comprenant de 5 à 20 atomes de carbone, et de préférence de 5 à 15 atomes de carbone, étant entendu que E comprend de 1 à 3 cycles aromatiques, et
- P est une chaîne polymère d'oxyde d'alkylène,
   ledit procédé étant caractérisé en ce qu'il comprend au moins une étape a₁) de polycondensation d'au moins un monomère ionique difluoré (I) avec au moins un polymère d'oxyde d'alkylène P¹ en milieu basique,
   ledit monomère ionique difluoré répondant à la formule (I) suivante : 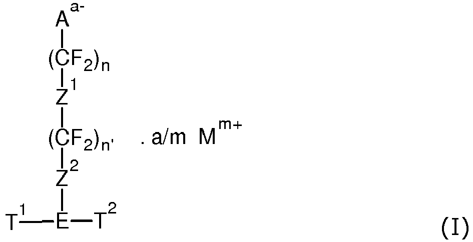 dans laquelle A, n, n', Z¹, Z², E, m, a et M sont tels que définis ci-dessus et T¹ et T² sont des atomes de fluor.

L'ionomère de l'invention peut comprendre p unités UP, de préférence au moins 2 unités UP, notamment avec 2 ≤ p ≤ 100, et de préférence encore 2 ≤ p ≤ 50.

Les monomères ioniques difluorés (I) utilisés dans le procédé de l'invention ont une forte aptitude à la dissociation et présentent l'avantage de pouvoir conduire de façon simple et économique à des ionomères à conduction cationique unipolaire, présentant notamment de bonnes performances électrochimiques et mécaniques, particulièrement en termes de conduction ionique, de nombre de transport cationique et de tenue mécanique.

Il convient de noter que la polycondensation, par comparaison avec d'autres types de polymérisation (e.g. polymérisation radicalaire), requiert généralement des monomères de départ de très grande pureté et suffisamment réactifs afin de conduire quantitativement au polymère souhaité. Par ailleurs, la polycondensation, par comparaison avec la polymérisation radicalaire, permet de former des unités récurrentes UP telles que définies ci-dessus ou en d'autres termes, un ionomère dans lequel on trouve une alternance : groupe ionique E-Z²-(CF₂)_{n'}-Z¹-(CF₂)ₙ-A^{a-} .(a/m) M^{m+} / chaine polymère d'oxyde d'alkylène / groupe ionique E-Z²-(CF₂)_{n'}-Z¹-(CF₂)ₙ-A^{a-} .(a/m) M^{m+} / chaine polymère d'oxyde d'alkylène etc... En effet, avec la polymérisation radicalaire, la différence de réactivité entre les monomères de départ ne permet pas d'obtenir une telle alternance et conduit à de l'homopolymérisation ou à l'obtention de polymères peignes, induisant la présence de groupes ioniques adjacents. Or, la présence de groupes ioniques adjacents diminue la dissociation et par conséquent, la conductivité ionique du polymère obtenu. La polycondensation permet également de connaître avec précision la position des groupes ioniques.

Dans le cas présent, la polycondensation d'un monomère ionique difluoré (I) avec un polymère d'oxyde d'alkylène P¹ est simple, facilement industrialisable et permet de conduire quantitativement à des ionomères à conduction cationique, notamment grâce à la présence des atomes de fluor très réactifs (i.e. T₁ = T₂ = F) sur le monomère ionique de départ.

En effet, l'utilisation de monomères ioniques dihydroxylés (remplacement des atomes de fluor par des groupes hydroxyles) comme précurseurs dans la polycondensation ne permet pas de conduire aux ionomères de l'invention avec de bons rendements. En effet, les monomères ioniques difluorés peuvent être directement polymérisés avec des polymères d'oxyde d'alkylène P¹ commerciaux, alors que les monomères ioniques dihydroxylés correspondants nécessitent des étapes supplémentaires, notamment de transformation dudit P¹ (terminé généralement par -OH) afin de le fonctionnaliser avec des atomes d'halogène ou d'autres fonctions capable de réagir avec les deux fonctions hydroxylates desdits monomères ioniques dihydroxylés. Elle n'est donc pas envisageable pour l'instant à l'échelle industrielle.

En particulier, les monomères ioniques (I) utilisés dans le procédé de la présente invention présentent l'avantage de disposer de trois fonctions clés sur le groupe aromatique E: une fonction ionique -Z²-(CF₂)_{n'}-Z¹-(CF₂)ₙ-A^{a-} .(a/m) M^{m+} et deux atomes de fluor (fonctions T¹ et T²). La fonction ionique permet d'améliorer leur dissociation en milieu aprotique de par leur caractère très peu basique ; et les atomes de fluor permettent leur polymérisation ou copolymérisation avec des monomères ou des oligomères, notamment à base de chaînes d'oxyde d'alkylène, et ainsi d'être d'excellents précurseurs d'ionomères à conduction cationique unipolaire à squelette polyéther solvatant présentant notamment une faible température de transition vitreuse T_{g}.

Dans la présente invention, l'expression « groupe alkyle » signifie un groupe alkyle (i.e. constitué d'atomes de carbone et d'hydrogène) linéaire, ramifié ou cyclique, et de préférence un groupe alkyle linéaire.

Dans la présente invention, l'expression « groupe alkoxy » signifie un groupe alkoxy linéaire, ramifié ou cyclique, et de préférence un groupe alkoxy linéaire.

Dans la présente invention, l'expression « groupe éther de dialkyle » signifie un groupe éther de dialkyle dont les deux groupes alkyles sont choisis, indépendamment l'un de l'autre, parmi des groupes alkyles linéaires, ramifiés et cycliques, et de préférence sont deux groupes alkyles linéaires.

Le groupe R alkyle éventuellement fluoré ou perfluoré a de préférence 1 à 5 atomes de carbone.

Selon une forme de réalisation de l'invention, le groupe R alkyle éventuellement fluoré ou perfluoré est un groupe alkyle linéaire, notamment choisi parmi :
* un groupe alkyle linéaire ayant de 1 à 5 atomes de carbone, et de préférence ayant de 1 à 3 atomes de carbone, tel que -CH₃ ou -C₂H₅,
* un groupe alkyle linéaire perfluoré ayant de 1 à 8 atomes de carbone, et de préférence ayant de 1 à 4 atomes de carbone, tel que -CF₃, -C₂F₅ ou -C₄F₉, et
* un groupe alkyle linéaire fluoré de formule -CH₂-CᵤF₂ᵤ₊₁, dans laquelle 1 ≤ u ≤ 5, et de préférence u = 1 ou 2, tel que -CH₂CF₃.

Le groupe électroattracteur X¹ peut être un groupe nitrile (-CN), un groupe -CF₃ ou un groupe nitro (-NO₂).

A titre d'exemple de groupe R alkyle éventuellement fluoré ou perfluoré, ayant de 1 à 10 atomes de carbone et portant au moins un groupe électroattracteur X¹, on peut citer un groupe alkyle linéaire ayant 1 ou 2 atomes de carbone et portant au moins un groupe nitrile ou nitro, tel que -CH₂CN, -CH₂NO₂, -CH(CN)₂ ou -C(CN)₃.

Le groupe R alkoxy éventuellement fluoré ou perfluoré a de préférence 1 à 6 atomes de carbone.

Le groupe électroattracteur X² peut être un groupe nitrile, un groupe -CF₃ ou un groupe nitro.

A titre d'exemple de groupe R alkoxy éventuellement fluoré ou perfluoré ayant de 1 à 10 atomes de carbone et portant au moins un groupe électroattracteur X², on peut citer un groupe alkoxy linéaire ayant de 2 à 4 atomes de carbone, et de préférence ayant 2 atomes de carbone, et portant au moins un groupe électroattracteur X², tel que -OC₂H₄CN ou -OC₂H₄NO₂.

Selon une forme de réalisation de l'invention, le groupe R alkoxy éventuellement fluoré ou perfluoré ayant de 1 à 10 atomes de carbone est un groupe choisi parmi :
* un groupe alkoxy linéaire ayant de 1 à 5 atomes de carbone, et de préférence ayant de 1 à 3 atomes de carbone, tel que -OCH₃ ou -OC₂H₅,
* un groupe alkoxy linéaire perfluoré ayant de 1 à 8 atomes de carbone, et de préférence ayant de 1 à 4 atomes de carbone,
* un groupe alkoxy linéaire fluoré de formule -O-CH₂-C_{w'}F_{2w'+1}, dans laquelle 1 ≤ w' ≤ 5, et de préférence w' = 1 ou 2, tel que -OCH₂CF₃, et
* un groupe alkoxy ramifié fluoré de formule -O-CH(CₜF₂ₜ₊₁)(C_{t'}F_{2t'+1}), dans laquelle 1 ≤ t ≤ 3, et de préférence t = 1 ou 2, 1 ≤ t' ≤ 3, et de préférence t' = 1 ou 2, tel que -OCH(CF₃)₂.

Le groupe R phénoxy éventuellement substitué par un groupe électroattracteur X² peut répondre à la formule -OC₆H₅ ou à la formule -OC₆H₄X² dans laquelle X² est tel que défini dans la présente invention, ledit groupe phényle étant substitué par le groupe X² en position para, ortho ou méta, et de préférence en position para ou ortho.

Le groupe R éther de dialkyle éventuellement fluoré ou perfluoré, a de préférence 1 à 5 atomes de carbone, notamment lorsque les groupes alkyles sont linéaires ou ramifiés.

Selon une forme de réalisation de l'invention, le groupe R éther de dialkyle éventuellement fluoré ou perfluoré, ayant de 1 à 10 atomes de carbone est un groupe éther de dialkyle linéaire, notamment choisi parmi :
* un groupe éther de dialkyle linéaire de formule -CᵥH₂ᵥOC_{w}H_{2w+1} dans laquelle 1 ≤ v ≤ 5, et de préférence 2 ≤ v ≤ 4, et 1 ≤ w ≤ 4, et de préférence w = 1 ou 2, et
* un groupe éther de dialkyle linéaire perfluoré de formule -C_{u'}F_{2u'}OC_{v'}F_{2v'+1}, dans laquelle 1 ≤ u' ≤ 5, et de préférence 2 ≤ u' ≤ 4, et 1 ≤ v' ≤ 4, et de préférence v' = 1 ou 2, tel que -CF₂CF₂OCF₂CF₃.

A titre d'exemple de groupe R phényle substitué, on peut citer un groupe phényle substitué par un groupe nitro, un groupe nitrile, un atome de fluor ou un groupe méthane sulfonyle (-SO₂CH₃), ledit groupe phényle étant substitué en para, et/ou ortho et/ou méta.

Dans un mode de réalisation préféré de l'invention, le groupe R¹ (respectivement le groupe R²) alkyle éventuellement fluoré ou perfluoré, ayant de 1 à 5 atomes de carbone est un groupe alkyle linéaire, notamment un groupe méthyle ou un groupe éthyle.

Le groupe électroattracteur X³ peut être un groupe nitrile, un groupe nitro ou un groupe thiocyanate, et plus généralement un substituant ayant des sigma para ou méta de Hammett positifs et de préférence supérieurs à 0,3.

Le groupe R¹ (respectivement le groupe R²) alkyle ayant de 1 à 5 atomes de carbone et portant un groupe électroattracteur X³ est de préférence un groupe de formule -(CH₂)ᵣCN ou -(CH₂)ᵣNO₂, dans laquelle r = 1 ou 2.

Le groupe R¹ (respectivement le groupe R²) éther de dialkyle éventuellement fluoré ou perfluoré, ayant de 1 à 5 atomes de carbone est de préférence un groupe -(CH₂)₂OCH₃.

Le groupe électroattracteur X⁴ peut être un groupe nitrile ou un groupe nitro.

Dans un mode de réalisation avantageux de l'invention, le groupe amino -NR¹R² est de préférence choisi parmi les groupes suivants : -N(CH₃)C₂H₅, -N(CH₃)₂, -N(C₂H₅)₂, -N[(CH₂)₂CN]₂, -N(CH₂CN)₂, -N[(CH₂)₂CN][CH₂CN], -N[(CH₂)₂OCH₃]₂, -N[(CH₂)₂OCH₃][CH₂CN], -N[(CH₂)₂OCH₃][(CH₂)₂CN], -N(CH₃)CN, -N(C₂H₅)CN, -N(CN)₂, -N[(CH₂)₂NO₂]₂, -N(CH₂NO₂)₂, -N[(CH₂)₂NO₂][CH₂NO₂], -N[(CH₂)₂OCH₃][CH₂NO₂], -N[(CH₂)₂OCH₃][(CH₂)₂NO₂], -N(CH₃)NO₂, -N(C₂H₅)NO₂ et -N(NO₂)₂.

A titre d'hétérocycle saturé -NR³ ayant de 3 à 6 atomes, on peut citer l'aziridine, l'azétidine, la pyrrolidine ou la pipéridine.

A titre d'hétérocycle insaturé -NR³ ayant de 4 à 6 atomes, on peut citer la pyrrolidone, le pyrrole, l'imidazole, le pyrazole, le triazole, le tétrazole, le succinimide ou le maléimide.

Le groupe amide de formule NH-CO-R⁴ ou -N(CH₃)-CO-R⁴ peut être cyclique ou linéaire.

Selon une forme de réalisation préférée de l'invention, le groupe R est un groupe alkyle perfluoré, tel que -CF₃.

Le groupe R¹⁰ est de préférence un groupe alkyle linéaire perfluoré, tel que -CF₃ ou -C₂F₅.

Le groupe R¹¹ est de préférence un groupe alkyle linéaire éventuellement perfluoré, tel que -CH₃, -C₂H₅, -CF₃ ou -C₂F₅.

Le groupe R¹² est de préférence un groupe alkyle linéaire éventuellement perfluoré, tel que -CH₃, -C₂H₅, -CF₃ ou -C₂F₅; ou un groupe éther de dialkyle linéaire perfluoré tel que -CF₂OCF₃, -CF₂OC₂F₅, -C₂F₄OC₂F₅ ou -C₂F₄OCF₃ ; ou un groupe alkoxy linéaire tel que -OCH₃ ou -OC₂H₅.

A titre d'exemple de groupe R' ou R" phényle substitué, on peut citer un groupe phényle mono-, di- ou tri-substitué par un ou plusieurs des groupes suivants : un atome de fluor, un groupe nitrile, un groupe nitro, un groupe nitroso ou un groupe carbonyle de formule -C(=O)R¹⁰ tel que défini précédemment, un groupe ester carboxylique de formule -COOR¹³ tel que défini précédemment, un groupe amide de formule -CONHR¹⁴ tel que défini précédemment, un groupe amide de formule -CONR¹⁴R¹⁵ tel que défini précédemment, ou un groupe de formule -SO₂X⁵ dans laquelle X⁵ est un atome de fluor, -CF₃, -SCN ou -CH₃. Le groupe R' ou R" phényle peut être substitué en position ortho et/ou méta et/ou para, et de préférence en position para.

A titre d'exemple de groupe R' ou R" phénoxy substitué, on peut citer un groupe phénoxy mono-, di- ou tri-substitué par un ou plusieurs des groupes suivants : un atome de fluor, un groupe nitrile, un groupe nitro, un groupe nitroso, un groupe de formule -SO₂X⁵ tel que défini précédemment, ou un groupe carbonyle de formule -C(=O)R¹⁰ tel que défini précédemment. Le groupe R' ou R" phénoxy peut être substitué en position ortho et/ou méta et/ou para, et de préférence en position para.

Le groupe -CR'R" formant un cycle aromatique comprenant de 5 à 6 atomes de carbone et optionnellement un ou plusieurs des hétéroatomes O ou N, ledit cycle aromatique étant éventuellement substitué par un ou plusieurs groupes nitriles, peut être un des groupes suivants :

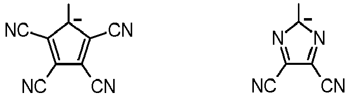

Lorsque ces carbanions sont fixés sur le groupe SO₂, on obtient un composé très acide dont la base conjuguée est doublement stabilisée : 1) par l'aromaticité de l'anion, et 2) par l'extension de la conjugaison aux groupes nitriles. L'acide formé est donc très acide et facilement transformable en sel.

Un anion dérivé d'un anion sulfonimidure portant au moins deux charges négatives (i.e. a = 2 ou 3) est un anion sulfonimidure -SO₂-N⁻-SO₂-Y dans lequel Y est un groupe chimique portant une ou deux charges négatives.

L'anion dérivé d'un anion sulfonimidure portant au moins deux charges négatives (i.e. a = 2 ou 3) peut être choisi parmi les anions de formules suivantes : -SO₂-N⁻-SO₂-N⁻-SO₂CF₃, -SO₂-N⁻-SO₂-N⁻-(CF₂)_{n"}-SO₃⁻ avec 2 ≤ n" ≤ 4, -SO₂-N⁻-SO₂-Ar-SO₃⁻ avec Ar étant un groupe phényle éventuellement substitué par 2, 3 ou 4 atomes de fluor et de préférence 4 atomes de fluor, -SO₂-N⁻-SO₂-Ar-SO₂-N⁻-SO₂CF₃, -SO₂-N⁻-SO₂-N⁻-SO₂-C⁻R'R", avec R' et R" étant tels que définis dans l'invention.

Avec un tel anion portant au moins deux charges négatives, on peut multiplier la CEI d'un facteur 2 ou 3 sans augmenter la concentration en monomère ionique, tout en augmentant modérément sa masse molaire.

Selon une forme de réalisation particulièrement préférée de l'invention, l'anion A^{a-} est un sulfonate ou un sulfonimidure (i.e. avec a = 1), et de préférence encore un sulfonimidure.

En effet, les monomères dans lesquels A^{a-} est un sulfonimidure conduisent à des ionomères dont la stabilité à l'oxydation et la conductivité ionique sont améliorées.

Le groupe aromatique E peut comprendre des éléments non aromatiques (dits « fonctionnels ») présents sur un ou plusieurs des cycles aromatiques ou permettant de lier plusieurs cycles aromatiques entre eux. Ces éléments non aromatiques peuvent être des groupes alkyle, alcényle, alcynyle, haloalkyle, diényle conjugué, esters, amide, etc...

Par exemple, le groupe benzophénone est un groupe aromatique E en C₁₃ (i.e. comprenant 13 atomes de carbone) comprenant deux cycles aromatiques (i.e. deux groupes phényles) et un élément non aromatique (i.e. groupe cétone) ; le groupe benzyle est un groupe aromatique E en C₇ (i.e. comprenant 7 atomes de carbone) comprenant un cycle aromatique (i.e. un groupe phényle) et un élément non aromatique (i.e un groupe méthylène).

Dans la présente invention, le groupe aromatique E peut comprendre des hétératomes tels qu'un ou plusieurs atomes d'azote, de soufre ou d'oxygène, ou être uniquement constitué d'atomes de carbone et d'hydrogène.

Dans la présente invention, le terme « cycle aromatique » signifie une structure cyclique ayant [4r'+2] électrons délocalisés, avec r' = 1.

De préférence, le groupe aromatique E comprend au moins un cycle aromatique qui est un groupe phényle, un groupe thiényle, un groupe pyridyle, un groupe furanyle, ou un groupe pyrazolyle, et de préférence un groupe phényle.

Les cycles aromatiques du groupe aromatique E peuvent être choisis, indépendamment les uns des autres, parmi un groupe phényle, un groupe thiényle, un groupe pyridyle, un groupe furanyle et un groupe pyrazolyle.

E comprend de préférence un ou deux cycles aromatiques.

Le ou les cycles aromatiques du groupe aromatique E sont de préférence des groupes phényles.

Lorsque E comprend deux cycles aromatiques, il peut être un groupe azulényle, un groupe benzophénone, un groupe diphényle, un groupe diphényl sulfure, un groupe diphényl sulfone ou un groupe naphthyle, et de préférence un groupe benzophénone, un groupe diphényl sulfure ou un groupe diphényl sulfone.

Lorsque E comprend trois cycles aromatiques, il peut être un anthracényle ou un groupe phénanthrényle.

De préférence, E est un groupe aromatique générant peu d'encombrement stérique, c'est-à-dire qu'il ne comprend pas de carbone tri-substitué par des cycles aromatiques tel qu'un groupe triphénylméthyle (groupe trityle).

Les atomes de fluor T¹ et T² sont de préférence sur au moins un cycle aromatique du groupe aromatique E, et de préférence encore sur un seul cycle aromatique ou deux cycles aromatiques différents.

En d'autres termes, les atomes de fluor T¹ et T² sont des substituants d'au moins un cycle aromatique du groupe aromatique E, et de préférence encore d'un seul cycle aromatique ou deux cycles aromatiques différents.

En particulier, lorsque E comprend plusieurs cycles aromatiques, les groupes T¹ et T² peuvent être (i.e. comme substituants) soit sur le même cycle aromatique, soit sur des cycles aromatiques différents du groupe E.

Ainsi, lorsque les atomes de fluor T¹ et T² sont des substituants de cycle(s) aromatique(s) du groupe aromatique E, le procédé de l'invention conduit à un ionomère dans lequel la chaîne polymère d'oxyde d'alkylène est directement liée au(x) cycle(s) aromatique(s) du groupe E (par l'intermédiaire des groupes terminaux du polymère d'oxyde d'alkylène P¹ ou des hétéroatomes terminaux de la chaîne polymère d'oxyde d'alkylène).

Cette liaison directe permet d'améliorer la conductivité du ionomère obtenu et d'obtenir de très bons rendements.

Dans un mode de réalisation préféré de l'invention, la fonction -Z²-(CF₂)_{n'}-Z¹-(CF₂)ₙ-A^{a-}.(a/m) M^{m+} est un substituant d'un cycle aromatique du groupe E.

Dans un mode de réalisation particulièrement préféré de l'invention, au moins deux des trois fonctions -Z²-(CF₂)_{n'}-Z¹-(CF₂)ₙ-A^{a-} .(a/m) M^{m+}, T¹ et T² sont sur un même cycle aromatique du groupe E (i.e. comme substituants).

Dans un mode de réalisation préféré de l'invention, E est choisi parmi un groupe phényle, un groupe benzophénone, un groupe diphényl sulfure et un groupe diphényl sulfone.

Dans le monomère ionique difluoré de formule (I), le métal de transition M peut être un métal de transition de la période 4 ou 5 du tableau périodique des éléments, en particulier un métal de transition choisi parmi le fer, le cuivre, le zinc, le cobalt, le nickel et l'argent.

Le fer peut être trivalent ou divalent (m = 3 ou 2) ; le cuivre le zinc, le cobalt et le nickel sont divalents (m = 2) et l'argent est monovalent (m = 1).

Le métal pauvre peut être l'aluminium. L'aluminium est trivalent (m = 3).

M est de préférence un cation de métal alcalin, tel que Li⁺, Na⁺ ou K⁺ (m = 1) ou un cation de métal alcalino-terreux, tel que Mg²⁺ ou Ca²⁺, et de préférence encore un cation de métal alcalin, tel que Li⁺ ou Na⁺.

A titre de cation ammonium, M peut être un composé de formule ⁺NR¹⁶R^{16'}R^{16"}R^{16"'} ou ⁺NHR¹⁶R^{16'}R^{16"} dans laquelle R¹⁶, R^{16'}, R^{16"} et R^{16'"} sont des groupes alkyles, identiques ou différents, ayant de 1 à 4 atomes de carbone.

Les ionomères porteurs de cations ammonium ⁺NHR¹⁶R^{16'}R^{16"} peuvent être avantageusement imprégnés de liquides ioniques.

A titre de cation sulfonium, M peut être un composé de formule ⁺SR¹⁶R^{16'}R^{16"} ou ⁺SHR¹⁶R^{16'} dans laquelle R¹⁶, R^{16'} et R^{16"} sont tels que définis précédemment.

A titre de cation phosphonium, M peut être un composé de formule ⁺PR¹⁶R^{16'}R^{16"}R ^{16"'} ou ⁺PHR¹⁶R^{16'}R^{16"} dans laquelle R¹⁶, R^{16'}, R^{16"} et R^{16'"} sont tels que définis précédemment.

Selon une forme de réalisation de l'invention, n' ≠ 0.

Le monomère ionique difluoré (I) de l'invention peut être tel que Z¹ est un atome d'oxygène et Z² est un atome de soufre ou une liaison simple.

Selon une forme de réalisation particulièrement préférée de l'invention, une ou plusieurs des conditions suivantes s'appliquent au monomère ionique difluoré (I) de l'invention :
* n = n' = 2,
* Z¹ est un atome d'oxygène,
* Z² est un atome de soufre ou une liaison simple.

A titre d'exemples de tels monomères ioniques difluorés (I), on peut citer les monomères répondant aux formules suivantes : 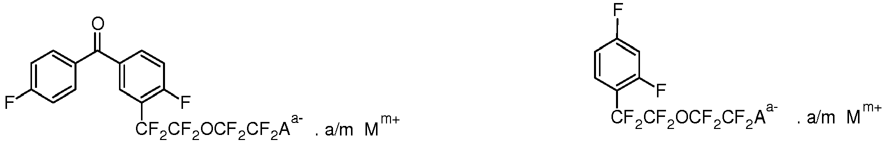 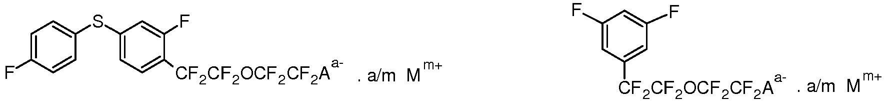 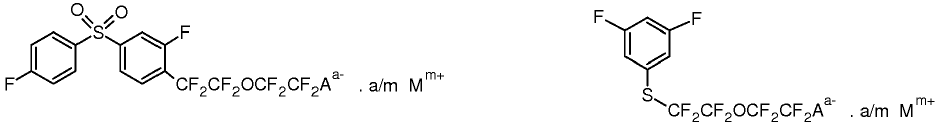 dans lesquelles M, A, a et m sont tels que définis dans l'invention.

L'étape a₁) de polycondensation permet de moduler la distance entre les fonctions ioniques et ainsi, de contrôler la viscosité et/ou la masse molaire du ionomère souhaité. Par exemple, l'augmentation de l'espace entre les fonctions ioniques permet d'améliorer la conductivité ionique dudit ionomère. Par conséquent, une certaine longueur de chaîne solvatante (i.e. de chaîne polymère d'oxyde d'alkylène P) est préférable afin que chaque fonction ionique soit convenablement solvatée.

La chaîne polymère d'oxyde d'alkylène P de l'unité UP peut comprendre des hétéroatomes terminaux de type atome d'oxygène (-O-), atome de soufre (-S-), amine secondaire (-NH-) ou tertiaire (-NR¹⁷-, R¹⁷ = -CH₃ ou -C₂H₅), et de préférence de type atome d'oxygène ou amine secondaire.

De ce fait, le polymère d'oxyde d'alkylène P¹ peut comprendre des groupes terminaux de type hydroxyle (-OH), thiol (-SH), amine primaire (-NH₂) ou secondaire (-NHR¹⁷, R¹⁷ = -CH₃ ou -C₂H₅), et de préférence de type hydroxyle ou amine primaire.

Le polymère d'oxyde d'alkylène P¹ utilisé dans l'étape a) peut être choisi parmi les polymères de formules suivantes :
* H-[O-(CH₂)ₓ]_{y}-OH, dans laquelle 2 ≤ x ≤ 4, 1 ≤ y ≤ 50, de préférence 2 ≤ y ≤ 40, et de préférence encore 8 ≤ y ≤ 34,
* H-[O-CH₂-CHR¹⁸]_{y}-OH, dans laquelle R¹⁸ est un groupe alkyle ayant de 1 à 8 atomes de carbone, et de préférence un groupe méthyle, ou un groupe alkoxy ayant de 1 à 8 atomes de carbone, et de préférence un groupe méthoxy, et 1 ≤ y ≤ 50, de préférence 2 ≤ y ≤ 40, et de préférence encore 8 ≤ y ≤ 34,
* H-[O-(CH₂)ₓᵢ-O-(CH₂-CHR¹⁹)ₓᵢᵢ]_{y}-OH, dans laquelle 1 ≤ xi ≤ 4, et de préférence xi = 1 ; 1 ≤ xii ≤ 2, et de préférence xii = 1 ; R¹⁹ est un atome d'hydrogène ou un groupe alkyle ayant de 1 à 8 atomes de carbone, et de préférence un atome d'hydrogène ou un groupe méthyle ; 1 ≤ y ≤ 50, de préférence 2 ≤ y ≤ 40, et de préférence encore 8 ≤ y ≤ 34,
* NH₂-CHR²⁰-CH₂-[O-CH₂-CHR²⁰]_{y}-NH₂, dans laquelle R²⁰ est un groupe alkyle ayant de 1 à 8 atomes de carbone, et de préférence un groupe méthyle ; et 1 ≤ y ≤ 50, de préférence 2 ≤ y ≤ 40, et de préférence encore 8 ≤ y ≤ 34, ces oligomères étant commercialisés sous le nom de Jeffamines® dans une large gamme de y lorsque R²⁰ est un CH₃, et
* NH₂-CHR²¹-CH₂-O-[CH₂-CH₂-O]_{y}-CH₂-CHR²¹-NH₂, dans laquelle R²¹ est un groupe alkyle ayant de 1 à 8 atomes de carbone, et de préférence un groupe méthyle ; et 1 ≤ y ≤ 50, de préférence 2 ≤ y ≤ 40, et de préférence encore 8 ≤ y ≤ 34.

On peut aussi utiliser à titre de P¹ un copolymère tribloc répondant à la formule suivante :

NH₂-CHR²²-CH₂-[(O-CH₂-CHR²²)_{x"}-(O-CH₂-CH₂)_{x"'}-(O-CH₂-CHR²²)ₓᵢᵢᵢ]_{y}-NH₂

dans laquelle R²² est un groupe alkyle ayant de 1 à 8 atomes de carbone, et de préférence un groupe méthyle ; 1 ≤ x" ≤ 50, et de préférence 5 ≤ x" ≤ 24 ; 1 ≤ x'" ≤ 50, et de préférence 8 ≤ x'" ≤ 34 ; 1 ≤ xiii ≤ 50, et de préférence 5 ≤ xiii ≤ 24 ; 1 ≤ y ≤ 50, de préférence 2 ≤ y ≤ 40, et de préférence encore 8 ≤ y ≤ 34.

Le polymère d'oxyde d'alkylène P¹ utilisé dans l'étape a₁) est de préférence un polymère répondant à l'une des formules suivantes telles que définies dans l'invention : H-[O-(CH₂)ₓ]_{y}-OH, H-[O-CH₂-CHR¹⁸]_{y}-OH ou H-[O-(CH₂)ₓᵢ-O-(CH₂-CHR¹⁹)ₓᵢᵢ]_{y}-OH.

Dans ce mode de réalisation particulier, la chaîne polymère d'oxyde d'alkylène P de l'unité UP répondant à la formule (II) peut répondre alors à l'une quelconque des formules suivantes :
* -[O-(CH₂)ₓ]_{y}-O-, dans laquelle 2 ≤ x ≤ 4, 1 ≤ y ≤ 50, de préférence 2 ≤ y ≤ 40, et de préférence encore 8 ≤ y ≤ 34,
* -[O-CH₂-CHR¹⁸]_{y}-O, dans laquelle R¹⁸ est un groupe alkyle ayant de 1 à 8 atomes de carbone, et de préférence un groupe méthyle, ou un groupe alkoxy ayant de 1 à 8 atomes de carbone, et de préférence un groupe méthoxy, et 1 ≤ y ≤ 50, de préférence 2 ≤ y ≤ 40, et de préférence encore 8 ≤ y ≤ 34,
* -[O-(CH₂)ₓᵢ-O-(CH₂-CHR¹⁹)ₓᵢᵢ]_{y}-O-, dans laquelle 1 ≤ xi ≤ 4, et de préférence xi = 1 ; 1 ≤ xii ≤ 2, et de préférence xii = 1 ; R¹⁹ est un atome d'hydrogène ou un groupe alkyle ayant de 1 à 8 atomes de carbone, et de préférence un atome d'hydrogène ou un groupe méthyle ; 1 ≤ y ≤ 50, de préférence 2 ≤ y ≤ 40, et de préférence encore 8 ≤ y ≤ 34,
* -NH-CHR²⁰-CH₂-[O-CH₂-CHR²⁰]_{y}-NH-, dans laquelle R²⁰ est un groupe alkyle ayant de 1 à 8 atomes de carbone, et de préférence un groupe méthyle ; et 1 ≤ y ≤ 50, de préférence 2 ≤ y ≤ 40, et de préférence encore 8 ≤ y ≤ 34, ou
* -NH-CHR²¹-CH₂-O-[CH₂-CH₂-O]_{y}-CH₂-CHR²¹-NH₂, dans laquelle R²¹ est un groupe alkyle ayant de 1 à 8 atomes de carbone, et de préférence un groupe méthyle ; et 1 ≤ y ≤ 50, de préférence 2 ≤ y ≤ 40, et de préférence encore 8 ≤ y ≤ 34.

On peut également citer une chaîne polymère d'oxyde d'alkylène P répondant à la formule suivante :

-NH-CHR²²-CH₂-[(O-CH₂-CHR²²)_{x"}-(O-CH₂-CH₂)_{x'"}-(O-CH₂-CHR²²)ₓᵢᵢᵢ]_{y}-NH-

dans laquelle R²² est un groupe alkyle ayant de 1 à 8 atomes de carbone, et de préférence un groupe méthyle ; 1 ≤ x" ≤ 50, et de préférence 5 ≤ x" ≤ 24 ; 1 ≤ x'" ≤ 50, et de préférence 8 ≤ x'" ≤ 34 ; 1 ≤ xiii ≤ 50, et de préférence 5 ≤ xiii ≤ 24 ; 1 ≤ y ≤ 50, de préférence 2 ≤ y ≤ 40, et de préférence encore 8 ≤ y ≤ 34.

La chaîne polymère d'oxyde d'alkylène P est de préférence une chaîne répondant à l'une des formules telles que définies ci-dessus : -[O-(CH₂)ₓ]_{y}-O-, -[O-CH₂-CHR¹⁸]_{y}-O- ou -[O-(CH₂)ₓᵢ-O-(CH₂-CHR¹⁹)ₓᵢᵢ]_{y}-O-.

Dans un mode de réalisation de l'invention, l'étape a₁) met en oeuvre la polycondensation d'un monomère ionique difluoré (I) avec un polymère d'oxyde d'alkylène en milieu basique. Dans ce cas, on obtient un ionomère de formule (III-a) ou (III-a') tel que défini ci-après.

L'étape a₁) peut mettre en oeuvre la polycondensation d'un monomère ionique difluoré (I) avec plusieurs (e.g. deux ou trois) polymères d'oxyde d'alkylène différents en milieu basique.

Par exemple, le monomère ionique difluoré (I) peut réagir avec P¹, P^{1'} et éventuellement P^{1"}, avec P^{1'} et P^{1"} ayant la même définition que P¹, et P¹, P^{1'}, P^{1"} étant différents.

Dans ce cas, on obtient un ionomère statistique (e.g. ionomère de formule (III-b₁) tel que défini ci-après) comprenant des unités récurrentes UP, UP', et éventuellement UP", avec P' et P" ayant la même définition que P, et P, P', P" différents. Les unités UP, UP' et UP" sont réparties de manière statistique dans l'ionomère formé.

L'étape a₁) peut mettre en oeuvre la polycondensation de plusieurs monomères ioniques difluorés (tels que définis dans l'invention) différents avec un polymère d'oxyde d'alkylène P¹ en milieu basique.

Par exemple, le polymère P¹ peut réagir avec un monomère ionique difluoré (I), un monomère ionique difluoré (I') et éventuellement un monomère ionique difluoré (I'), les formules (I') et (I") ayant la même définition que celle de la formule (I), et les trois monomères étant différents.

Dans ce cas, on obtient un ionomère statistique (e.g. ionomère de formule (III- b₂) tel que défini ci-après) comprenant des unités récurrentes UP_{I}, UP'_{I}, et éventuellement UP"_{I}, avec (I') et (I") ayant la même définition que celle de (I), et les trois monomères étant différents. Les unités UP_{I}, UP'_{I} et UP"_{I} sont réparties de manière statistique dans l'ionomère formé.

L'étape a₁) peut être suivie de plusieurs autres étapes mettant en oeuvre : soit d'autres monomères ioniques difluorés différents du monomère ionique difluoré de l'étape a₁), soit d'autres polymères d'oxyde d'alkylène différents du polymère d'oxyde d'alkylène P¹ de l'étape a₁). Ces étapes sont détaillées ci-dessous.

Le procédé peut comprendre en outre après l'étape a₁), plusieurs autres étapes aᵢ) distinctes de polycondensation du monomère ionique difluoré (I) utilisé dans l'étape a) avec plusieurs polymères d'oxyde d'alkylène différents de P¹, (e.g. avec P^{1'}, et éventuellement P¹" tels que définis précédemment).

Par exemple, le monomère ionique difluoré (I) peut être polycondensé avec le polymère d'oxyde d'alkylène P¹ selon l'étape a₁), puis avec P^{1'} tel que défini précédemment selon l'étape a₂), puis éventuellement avec P^{1"} tel que défini précédemment selon l'étape a₃).

Dans ce cas, des blocs d'unités récurrentes (UP)ₚ, (UP')_{p'} et (UP")_{p"} sont formés séparément selon les étapes distinctes a₁), a₂) et a₃), puis lesdits blocs sont polycondensés selon un étape a') postérieure à la dernière étape aᵢ) (i.e. a₃) dans le cas présent) pour former un ionomère à blocs (e.g. ionomère de formule (III-c₁) tel que défini ci-après).

Le procédé peut comprendre en outre après l'étape a₁), plusieurs autres étapes Aᵢ) distinctes de polycondensation du polymère d'oxyde d'alkylène P¹ utilisé dans l'étape a₁) avec plusieurs monomères ioniques difluorés tels que définis dans l'invention et différents de celui utilisé dans l'étape a₁) (e.g. avec le monomère de formule (I'), et éventuellement le monomère de formule (I") tels que définis précédemment).

Par exemple, le polymère d'oxyde d'alkylène P¹ peut être polycondensé avec le monomère ionique difluoré (I) selon l'étape a₁), puis avec le monomère ionique difluoré (I') selon l'étape A₂), puis éventuellement avec le monomère ionique difluoré (I") selon l'étape A₃).

Dans ce cas, des blocs d'unités récurrentes (UP_{I})ₚ, (UP'_{I})_{p'} et (UP"_{I})_{p"} sont formés séparément selon les étapes distinctes a₁), A₂) et A₃), puis lesdits blocs sont polycondensés selon un étape A') postérieure à la dernière étape Aᵢ) (i.e. A₃) dans le cas présent) pour former un ionomère à blocs (e.g. ionomère de formule (III-c₂) tel que défini ci-après).

Le procédé peut comprendre en outre après l'étape a₁), une étape α) de polycondensation du ionomère issu de l'étape a₁), avec un polymère d'oxyde d'alkylène P^{1'} tel que défini précédemment. On obtient alors un ionomère de formule (III- d₁) tel que défini ci-après.

Le procédé peut comprendre en outre après l'étape a), une étape α') de polycondensation du ionomère issu de l'étape a₁), avec un monomère ionique difluoré (I') tel que défini précédemment. On obtient alors un ionomère de formule (III-d₂) tel que défini ci-après.

Les ionomères de l'invention obtenus (statistiques, alternés ou à blocs) peuvent ainsi comprendre deux ou trois chaînes polymères d'oxyde d'alkylène différentes, par exemple des chaînes polymères d'oxyde d'éthylène de formule -[O-CH₂-CH₂]_{y}-O- et/ou des chaînes d'oxyde de triméthylène -[O-CH₂-CH₂-CH₂]_{y}-O- et/ou des chaînes d'oxyde de propylène -[O-CH₂-CHCH₃]_{y}-O-.

Selon une première variante du procédé de l'invention, le procédé peut comprendre en outre après l'étape a₁), ou éventuellement après l'une quelconque des étapes a₁), a'), Aᵢ), A'), α) ou α') si elle existe, au moins une étape b) de mise en contact du ionomère avec un composé G comprenant au moins deux fonction F¹ aptes à se polycondenser avec ledit ionomère et éventuellement au moins une fonction F² post-polymérisable.

L'étape b) est par conséquent une étape de polycondensation du ionomère avec le composé G. Elle permet notamment de conduire au ionomère de formule (III-e) ou (III-e') tel que défini ci-après.

La fonction F¹ peut être un atome d'halogène tel qu'un atome de chlore, une fonction isocyanate ou une fonction carboxylate.

A titre d'exemple, le composé G comprenant au moins deux fonction F¹ peut être un diisocyanate, un triisocyanate, un composé di- ou trihalogéné, ou tout autre composé comprenant deux ou plusieurs fonctions F¹ capables de réagir avec la chaîne polymère d'oxyde d'alkylène P du ionomère de l'étape a₁) ou éventuellement de l'une quelconque des étapes aᵢ), a'), Aᵢ), A'), α) ou α') si elle existe, et notamment avec les groupes terminaux de ladite chaîne, selon une polycondensation.

Le diisocyanate peut être un alkylène diisocyanate tel que l'hexaméthylène diisocyanate (HMDI), un toluène diisocyanate (TDI) (e.g. 2,4-diisocyanate, toluène 2,6-diisocyanate), un oligo(oxyalkylène) α,ω-diisocyanate (par exemple un PEG α,ω-diisocyanate ou un PTHF α,ω-diisocyanate).

En particulier, les composés G de type diisocyanate ou triisocyanate peuvent se polycondenser avec la chaîne polymère d'oxyde d'alkylène P pour conduire à des polyuréthanes (lorsque les groupes terminaux sont des alcools) ou des polyurées (lorsque les groupes terminaux sont des amines primaires).

Dans la présente invention, le terme « fonction post-polymérisable F²» signifie une fonction qui ne réagit pas avec la chaîne polymère d'oxyde d'alkylène P du ionomère de l'étape a₁) ou éventuellement de l'une quelconque des étapes aᵢ), a'), Aᵢ), A'), α) ou α') si elle existe, et notamment avec les groupes terminaux de ladite chaîne, mais qui peut permettre la mise en oeuvre d'une polymérisation postérieure (e.g. polymérisation ionique, polymérisation radicalaire ou polycondensation).

A titre d'exemple de composé G comprenant au moins deux fonction F¹ et une fonction post-polymérisable F², on peut citer le 3-chloro-2-chloroprop-1-ène ou les isomères Z et E (i.e. *cis* et *trans*) du 1,4-dibromo-but-2-ène.

Le 3-chloro-2-chloroprop-1-ène à titre de composé G réagit avec la chaîne polymère d'oxyde d'alkylène P par l'intermédiaire de ses fonctions -Cl (fonctions F¹) en position allylique selon une polycondensation (étape b)) et il comprend en outre une fonction post-polymérisable F² de type alcényle.

La fonction post-polymérisable F² peut être polymérisable chimiquement, thermiquement ou photochimiquement, et de préférence photochimiquement.

Les fonctions post-polymérisables F² peuvent être des fonctions alkoxysilane, alcényle, alcynyle, vinyl éther, acrylate ou méthacrylate.

Lorsque le composé G comprend une fonction post-polymérisable F², le procédé de l'invention peut comprendre en outre une étape c) de post-polymérisation (e.g. polymérisation radicalaire, ionique ou hydrolyse-polycondensation) du ionomère obtenu à l'issue de l'étape b).

La post-polymérisation c) (i.e. réticulation) peut permettre de former un ionomère réticulé, c'est-à-dire un réseau tridimensionnel favorable pour atteindre un nombre de transport cationique proche de 1.

Dans l'ionomère réticulé, des liaisons chimiques se sont formées suivant toutes les directions de l'espace afin de conduire à la formation d'un réseau tridimensionnel.

L'étape de post-polymérisation c) peut accroître la constante diélectrique de l'ionomère et ainsi augmenter la dissociation des paires d'ions de l'ionomère.

En particulier, un ionomère porteur d'une fonction alcényle peut être obtenu à l'issue de l'étape b). Puis, l'ionomère obtenu peut réagir avec un carbonate cyclique insaturé tel que le carbonate de vinylène selon une étape b'). Enfin, l'ionomère obtenu à l'issu de b') peut subir une étape c) de post-polymérisation (e.g. amorçage radicalaire, thermique ou photochimique) afin de permettre la copolymérisation entre le carbonate de vinylène et les fonctions alcényles de l'ionomère.

Selon une deuxième variante du procédé de l'invention, le procédé peut comprendre en outre, après l'étape a₁), ou éventuellement après l'une quelconque des étapes aᵢ), a'), Aᵢ), A'), α) ou α') si elle existe, au moins une étape d) de mise en contact du ionomère avec un composé H comprenant une fonction F¹ apte à se condenser avec ledit ionomère et éventuellement au moins une fonction F² post-polymérisable.

Les fonctions F¹ et F² sont telles que définies précédemment.

L'étape d) est par conséquent une étape de condensation du ionomère avec le composé H. Elle permet notamment de conduire au ionomère de formule (III-f) ou (III-f') tel que défini ci-après.

A titre d'exemple, le composé H comprenant une fonction F¹ peut être un isocyanatopropyltrialkoxysilane ou tout autre composé comprenant une seule fonction F¹ capable de réagir avec la chaîne polymère d'oxyde d'alkylène du ionomère de l'étape a₁), ou éventuellement de l'une quelconque des étapes aᵢ), a'), Aᵢ), A'), α) ou α') si elle existe, et notamment avec les groupes terminaux de ladite chaîne, selon une condensation.

Lorsque le composé H est un isocyanatopropyl trialkoxysilane, il peut se condenser avec la chaîne polymère d'oxyde d'alkylène P pour donner un seul lien uréthane ou urée.

Lorsque le composé H comprend une fonction post-polymérisable F², le procédé de l'invention peut comprendre en outre une étape e) de post-polymérisation (e.g. polymérisation radicalaire, ionique ou d'hydrolyse-polycondensation) du ionomère obtenu à l'issue de l'étape d).

En particulier, lorsque le composé H est un isocyanatopropyl trialkoxysilane, l'ionomère obtenu à l'issue de l'étape de condensation d) peut subir une étape e) d'hydrolyse-polycondensation par méthode sol-gel afin de former un polysiloxane.

Selon une troisième variante du procédé de l'invention, le procédé peut comprendre en outre, après l'étape a₁), ou éventuellement après l'une quelconque des étapes aᵢ), a'), Aᵢ), A'), α) ou α') si elle existe, au moins une étape f) de mise en contact du ionomère avec un composé J apte à réagir avec ledit ionomère selon une polymérisation radicalaire ou ionique, et de préférence radicalaire.

Le composé J peut être choisi parmi le styrène, l'alphaméthylstyrène, la vinylpyridine, l'acénaphtylène, l'acide acrylique, l'acide méthacrylique, un acrylate d'alkyle (e.g. acrylate de méthyle), un méthacrylate d'alkyle (e.g. méthacrylate de méthyle), l'acrylonitrile, le méthacrylonitrile, un N-alkyl-acrylamide, un N-alkyl-methacrylamide ou le carbonate de vinylène. En effet, les groupes terminaux hydroxyles (-OH) ou amine primaire (-NH₂) de la chaîne polymère d'oxyde d'alkylène peuvent être utilisés pour amorcer une polymérisation permettant d'accéder à des polymères di-blocs ou tri-blocs à base de polystyrène, de polyalphaméthylstyrène, de polyvinylpyridine, de polyacénaphtylène, de polyacrylate d'alkyle, de polyméthacrylate d'alkyle, de polyacrylonitrile ou de polyméthacrylonitrile.

L'étape f) permet notamment de conduire au ionomère de formule (III-g) tel que défini ci-après.

A titre d'exemple, les groupes terminaux hydroxyles (-OH) peuvent être transformés en alcoolate de sodium par traitement avec NaH. Ces groupes alcoolate peuvent ensuite amorcer la polymérisation anionique de l'acrylonitrile, du méthacrylonitrile ou des acrylates et méthacrylates d'alkyle.

Les groupes terminaux amines peuvent être transformées en amidure de sodium par traitement avec du Na métal. Ces groupes amidures peuvent ensuite amorcer la polymérisation des styrènes.

Il est également possible de préparer un polymère de type polyéthersulfone ou polyéther-éther-cétone terminé respectivement par un groupe diphénylsulfone porteur d'un chlore terminal ou par un groupe benzophénone porteur d'un fluor terminal. Séparément, l'ionomère terminé par un -OH ou par un -NH₂ est transformé respectivement en alcoolate ou en amidure de sodium, puis réagit avec le chlore ou le fluor terminal du polymère préparé précédemment pour former un copolymère di-bloc ou tri-bloc.

On peut aussi, par écart maîtrisé à la stœchiométrie, préparer un polyéther-sulfone ou un polyéther-éther-cétone terminé aux 2 extrémités respectivement par des chlores ou des fluors. La polycondensation avec les ionomères sous forme dialcoolate ou diamidure de sodium conduit alors à un polymère alternant des blocs de polymère polyethersulfone ou/et de polyéther-éther-cétone rigides et hydrophobes et des blocs flexibles et hydrophiles d'ionomères, les premiers blocs assurant une excellente tenue mécanique et les seconds les propriétés de conduction ionique. En modulant la taille des blocs rigides, on peut utiliser ces copolymères di, tri et multi-blocs sans addition de solvant. Les polymères résultants auront de remarquables propriétés mécaniques mais seront moins conducteurs que les ionomères à base exclusive de polyéthers. Par contre ces copolymères à blocs seront très adaptés à la formation de gels, les blocs rigides et hydrophobes assurant une excellente tenue mécanique et empêchant un gonflement excessif par les solvants liquides (e.g. solvants carbonates).

Le procédé peut comprendre en outre après l'une quelconque des étapes a₁), aᵢ), a'), Aᵢ), A'), α), α), b), b'), c), d), e) ou f), une étape g) d'échange cationique afin de remplacer le cation M du ionomère par un autre cation M souhaité.

Les ionomères de l'invention sont hydrosolubles, ils sont par conséquent facilement purifiables par ultra-filtration selon une étape h) postérieure à l'une quelconque des étapes a₁), aᵢ), a'), Aᵢ), A'), α), α'), b), b'), c), d), e), f) ou g).

Le monomère ionique difluoré (I) tel que défini dans l'invention peut être préparé selon un procédé comprenant au moins une étape i) de réaction d'un composé (I-a) avec un composé (I-b) selon le schéma réactionnel suivant : 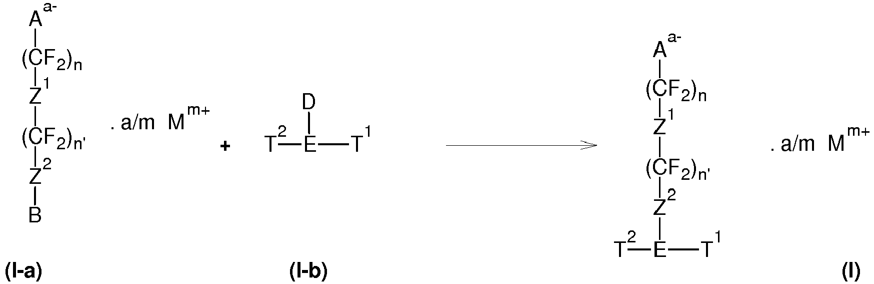 ou au moins une étape i') de réaction d'un composé (I'-a) avec un composé (I'-b) selon le schéma réactionnel suivant : 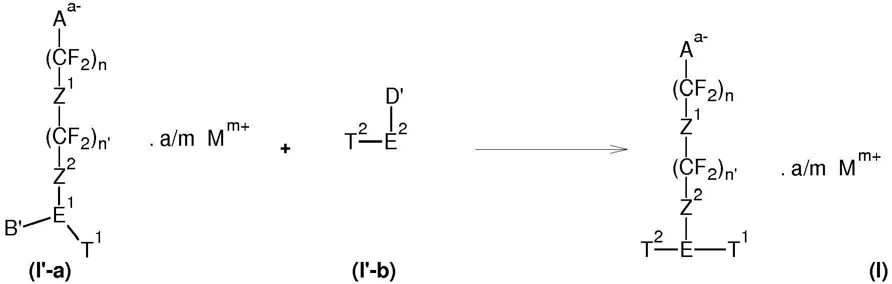 ou au moins une étape i") de réaction d'un composé (I"-a) avec un composé (I"-b) selon le schéma réactionnel suivant : 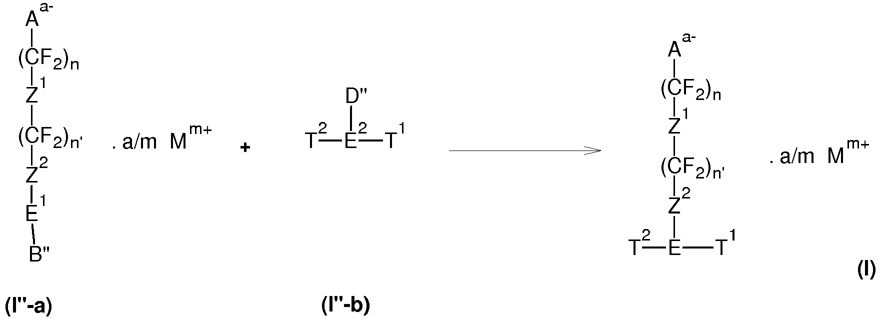
avec Z¹, Z², n, n', T¹, T², E, A, a, m et M étant tels que définis dans l'invention, et
les groupes B et D, les groupes B' et D' et les groupes B" et D" étant choisis de façon appropriée pour pouvoir réagir entre eux et E¹ et E² étant choisis de façon appropriée pour pouvoir former le groupe aromatique E.

L'étape i) est généralement utilisée lorsque E comprend un cycle aromatique, tandis que l'étape i') ou l'étape i") est préférée lorsque E comprend deux ou trois cycles aromatiques. En outre, si T¹ et T² ne sont pas sur le même cycle aromatique, l'étape i') est utilisée et si T¹ et T² sont sur le même cycle aromatique, l'étape i") est utilisée.

Lorsque Z² est un atome d'oxygène, l'étape i) est de préférence une réaction de substitution nucléophile.

En particulier, l'étape i) est conduite en présence du composé de formule (I-b) dans laquelle D est un atome d'iode, de brome ou de chlore, du composé de formule (I-a) dans laquelle B est un atome d'hydrogène, et d'au moins un réactif (base) choisi parmi le carbonate de sodium, le carbonate de potassium, le carbonate de lithium, l'hydrure de sodium, l'hydrure de potassium et l'hydrure de lithium.

Lorsque Z² est un atome de soufre, l'étape i) est de préférence une réaction de substitution nucléophile.

En particulier, l'étape i) est conduite en présence du composé de formule (I-b) dans laquelle D est un atome d'iode, de brome ou de chlore, du composé de formule (I-a) dans laquelle B est un atome d'hydrogène, et d'au moins un réactif (base) tel que l'hydrure de sodium ou l'hydrure de lithium.

Lorsque Z² est un groupe S=O ou un groupe S(=O)₂, l'étape i) comprend de préférence une première sous-étape permettant d'accéder au monomère ionique difluoré dans lequel Z² est un atome de soufre, ladite sous-étape étant telle que définie précédemment, et une deuxième sous-étape d'oxydation.

En particulier, l'étape i) est conduite selon une première sous-étape en présence du composé de formule (I-b) dans laquelle D est un atome d'iode, de brome ou de chlore, du composé de formule (I-a) dans laquelle B est un atome d'hydrogène, et d'au moins un réactif (base) tel que l'hydrure de sodium, l'hydrure de lithium, le carbonate de sodium, le carbonate de potassium ou le carbonate de lithium , et selon une deuxième sous-étape en présence d'au moins un péroxyde organique tel que l'acide méta-chloropéroxybenzoique (mCPBA).

Les réactions telles que décrites ci-dessus pour Z² = O, S, S=O ou S(=O)₂ sont également applicables lorsque n' = 0 (et par conséquent Z² est une liaison simple) et Z¹ = O, S, S=O ou S(=O)₂.

Lorsque Z² est un groupe C=O, l'étape i) est de préférence une réaction de Friedel et Craft.

En particulier, l'étape i) est conduite en présence du composé de formule (I-a) dans laquelle B est un atome de chlore, du composé de formule (I-b) dans laquelle D est un atome d'hydrogène positionné directement sur un cycle aromatique du groupe E et ledit cycle aromatique ayant en position ortho de l'hydrogène un atome de fluor (T¹ ou T²) ou un groupe méthoxy (-OMe), et de réactifs de type AlCl₃.

Lorsque Z² est une liaison simple et n' ≠ 0, ou lorsque Z¹ est une liaison simple et n' = 0 (et par conséquent Z² est une liaison simple), l'étape i) est de préférence une réaction de couplage de type Ullmann.

En particulier, l'étape i) est conduite en présence du composé de formule (I-a) dans laquelle B est un atome d'halogène tel qu'un atome d'iode, du composé de formule (I-b) dans laquelle D est un atome d'halogène tel qu'un atome de brome ou d'iode, et de cuivre (0) dans un solvant tel que le diméthylsulfoxyde (DMSO) ou du diméthylacétamide (DMAc).

Le procédé peut comprendre en outre une étape i₀) préalable à l'étape i) de préparation du composé de formule (I-a) à partir du composé répondant à la formule (I-c) suivante : 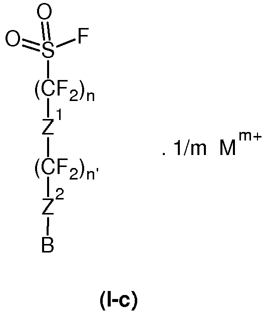 dans laquelle Z¹, n, m et M sont tels que définis dans l'invention, Z² est une liaison simple et n' ≠ 0 ou Z¹ est une liaison simple et n' = 0, et le groupe B est un atome d'halogène tel qu'un atome d'iode.

Selon une première variante de l'étape i₀), le composé (I-c) est mis en présence d'un hydroxyde de métal M dans un solvant organique tel que du tétrahydrofurane, M étant tel que défini dans l'invention. Par conséquent, l'hydrolyse du fluorure de sulfonyle (i.e. du composé (I-c)) conduit directement au composé de formule (I-a) dans laquelle A^{a-} est un anion sulfonate tel que défini dans l'invention.

Selon une deuxième variante de l'étape i₀), le composé (I-c) est mis en présence d'un sulfonamide de formule RSO₂NH₂ en milieu basique, notamment en présence de triéthylamine, dans un solvant organique tel que de l'acétonitrile, R étant tel que défini dans l'invention ; puis le composé obtenu est mis en présence d'un hydroxyde de métal M en milieu aqueux, M étant tel que défini dans l'invention. Par conséquent, la réaction de substitution nucléophile du fluorure de sulfonyle (i.e. du composé (I-c)) par un sulfonamide de formule RSO₂NH₂ puis la formation du sulfonimidure, conduisent directement au composé de formule (I-a) dans laquelle A^{a-} est un anion sulfonimidure tel que défini dans l'invention.

Selon une troisième variante de l'étape i₀), le composé (I-c) est mis en présence d'un sel de formule M'⁺(CHR'R")⁻ dans un solvant organique tel que l'acétonitrile, M'⁺ étant un cation monovalent tel que Na⁺, K⁺ ou Li⁺, et R' et R" étant tels que définis dans l'invention ; puis le composé obtenu est mis en présence d'un hydroxyde de métal M en milieu aqueux, M étant tel que défini dans l'invention. Par conséquent, la réaction de substitution nucléophile du fluorure de sulfonyle (i.e. du composé (I-c)) par un sel de formule M'⁺(CHR'R")⁻ puis la formation du carbanion, conduisent directement au composé de formule (I-a) dans laquelle A^{a-} est un carbanion tel que défini dans l'invention.

L'étape i') [respectivement l'étape i")] peut être effectuée par une réaction de substitution nucléophile, notamment en milieu basique, d'un composé de formule (I'-b) [respectivement de formule (I"-b)] dans laquelle D' [respectivement D"] est un atome d'halogène tel qu'un atome de fluor, de chlore ou d'iode, par un composé de formule (I'-a) [respectivement de formule (I"-a)] dans laquelle B' [respectivement B"] est choisi parmi un groupe thiol, un groupe alcool, un groupe acide carboxylique, un groupe amide et un groupe amine.

Les définitions de B' et D' ou de B" et D" peuvent être inversées, c'est-à-dire que l'étape i') [respectivement l'étape i")] peut être effectuée par une réaction de substitution nucléophile, notamment en milieu basique, d'un composé de formule (I'-a) [respectivement de formule (I"-a)] dans laquelle B' [respectivement B"] est un atome d'halogène tel qu'un atome de fluor, de chlore ou d'iode, par un composé de formule (I'-b) [respectivement de formule (I"-b)] dans laquelle D' [respectivement D"] est choisi parmi un groupe thiol, un groupe alcool, un groupe acide carboxylique, un groupe amide et un groupe amine.

Selon ces modes de réalisation, le groupe aromatique E obtenu comprend au moins un élément fonctionnel qui permet de relier les groupes aromatiques E¹ et E².

E¹ (respectivement E²) est un groupe aromatique comprenant de 5 à 15 atomes de carbone, et de préférence de 5 à 10 atomes de carbone, étant entendu que E¹ (respectivement E²) comprend au moins un cycle aromatique et au plus deux cycles aromatiques.

Le groupe aromatique E¹ (respectivement E²) peut comprendre des éléments non aromatiques (dits « fonctionnels ») présents sur un ou plusieurs des cycles aromatiques ou permettant de lier plusieurs cycles aromatiques entre eux. Ces éléments non aromatiques peuvent être des groupes alkyles, alcényles, alcynyles, haloalkyles, diényles conjugués, alcools, éthers, acide carboxyliques, cétones, esters, amides, amines, nitro, etc...

Le groupe aromatique E¹ (respectivement E²) peut comprendre des hétératomes tels qu'un ou plusieurs atomes d'azote, de soufre ou d'oxygène, ou être uniquement constitué d'atomes de carbone et d'hydrogène.

Le terme « cycle aromatique » est tel que défini dans l'invention.

Selon un mode de réalisation préféré, E¹ et E² sont des groupes phényles.

Lorsque le groupe B' ou D' [respectivement B" ou D"] est un groupe thiol, le procédé peut comprendre après l'étape i') ou l'étape i"), une étape d'oxydation du groupe thiol en sulfoxyde ou en sulfone.

Le procédé peut comprendre en outre avant l'étape i'), une étape i₀') de préparation du composé (I'-a) par réaction du composé (I-a) tel que défini précédemment avec un composé (I'-d) de formule suivante : 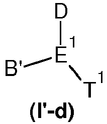 dans laquelle B', D, E¹ et T¹ sont tels que définis dans la présente invention.

L'étape i₀') peut être effectuée selon l'une quelconque des réactions précitées dans la présente invention et qui permet de faire réagir le groupe B avec le groupe D, notamment selon la nature de Z² ou de Z¹ et la valeur de n' dans le composé (I-a).

Le composé (I-a) peut être obtenu à partir du composé (I-c) tel que défini dans la présente invention, selon l'une des variantes telles que décrites dans la présente invention. La variante utilisée sera notamment choisie en fonction de la nature de l'anion A^{a-} souhaité.

Le procédé peut comprendre en outre avant l'étape i"), une étape i₀") de préparation du composé (I"-a) par réaction du composé (I-a) tel que défini précédemment avec un composé (I"-d) de formule suivante : 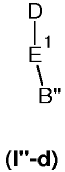 dans laquelle B", D et E¹ sont tels que définis dans la présente invention.

L'étape i₀") peut être effectuée selon l'une quelconque des réactions précitées dans la présente invention et qui permet de faire réagir le groupe B avec le groupe D, notamment selon la nature de Z² ou de Z¹ et la valeur de n' dans le composé (I-a).

Le composé (I-a) peut être obtenu à partir du composé (I-c) tel que défini dans la présente invention, selon l'une des variantes telles que décrites dans la présente invention. La variante utilisée sera notamment choisie en fonction de la nature de l'anion A^{a-} souhaité.

Ainsi, le procédé de préparation des monomères ioniques difluorés (I) est simple, économique. En particulier, la présence des deux atomes de fluor à titre de groupes T¹ et T² est avantageuse puisqu'elle permet d'obtenir rapidement et quantitativement, les monomères ioniques difluorés (I). En effet, d'une part, les monomères ioniques difluorés obtenus sont facilement purifiables, et d'autre part le procédé évite toutes les étapes de protection et déprotection qui seraient nécessaires si des groupes de type hydroxyle, amine, acide carboxylique, ester ou thiol étaient utilisés à la place des atomes de fluor T¹ et T² de l'invention.

Par ailleurs, les monomères ioniques difluorés (I) présentent l'avantage de réagir avec des oligomères ou polymères d'oxyde d'alkylène en formant des liaisons éther qui sont plus stables, notamment à l'hydrolyse, que des liaisons esters et amides qui seraient obtenues si l'on partait de monomères ioniques difonctionnalisés par des groupes acide carboxylique ou ester. Cette meilleure stabilité permet un lavage à l'eau sans dégradation dudit monomère ionique difluoré (I).

L'invention a pour deuxième objet un ionomère, caractérisé en ce qu'il comprend au moins des unités récurrentes UP répondant à la formule (II) suivante : 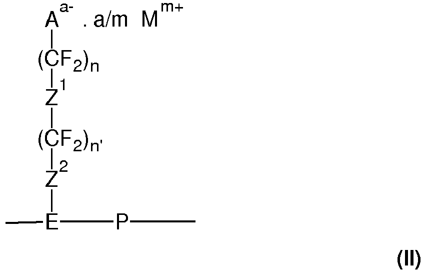 dans laquelle A, n, n', Z¹, Z², E, m, a et M sont tels que définis dans le premier objet de l'invention, et P est une chaîne polymère d'oxyde d'alkylène.

P est tel que définie dans le premier objet de l'invention.

L'ionomère peut comprendre p unités UP, de préférence au moins deux unités UP, notamment avec 2 ≤ p ≤ 100, et de préférence 2 ≤ p ≤ 50.

L'ionomère peut être obtenu selon le procédé conforme au premier objet de l'invention.

Comme expliqué ci-dessus, le procédé de l'invention est une polycondensation qui permet de former des unités récurrentes UP telles que définies ci-dessus ou en d'autres termes, un ionomère dans lequel on trouve une alternance : groupe ionique E-Z²-(CF₂)_{n'}-Z¹-(CF₂)ₙ-A^{a-} .(a/m) M^{m+} / chaine polymère d'oxyde d'alkylène / groupe ionique E-Z²-(CF₂)_{n'}-Z¹-(CF₂)ₙ-A^{a-} .(a/m) M^{m+} / chaine polymère d'oxyde d'alkylène etc... Une telle alternance permet d'éviter la présence de groupes ioniques adjacents qui diminue la dissociation et par conséquent, la conductivité ionique du polymère obtenu. La polycondensation permet également d'obtenir un ionomère dans lequel la position des groupes ioniques est précisément connue.

Dans un mode de réalisation particulièrement préféré de l'invention, la chaîne polymère d'oxyde d'alkylène P du ionomère est directement liée au(x) cycle(s) aromatique(s) du groupe E (par l'intermédiaire des groupes terminaux du polymère d'oxyde d'alkylène P¹ ou des hétéroatomes terminaux de la chaîne polymère d'oxyde d'alkylène). Ce ionomère est obtenu par le procédé de l'invention lorsque les atomes de fluor T¹ et T² sont des substituants de cycle(s) aromatique(s) du groupe aromatique E.

Dans un mode de réalisation particulier, l'ionomère comprend uniquement des unités récurrentes UP.

Il répond donc à la formule (III-a) suivante : 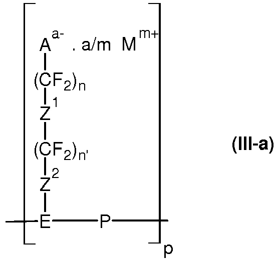 dans laquelle A, n, n', Z¹, Z², E, m, a, M, P et p sont tels que définis dans le premier objet de l'invention.

Selon une forme de réalisation préférée de l'invention, l'ionomère de l'invention répond à l'une quelconque des formules (III-a') suivantes : 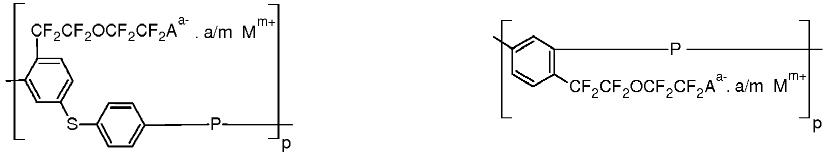 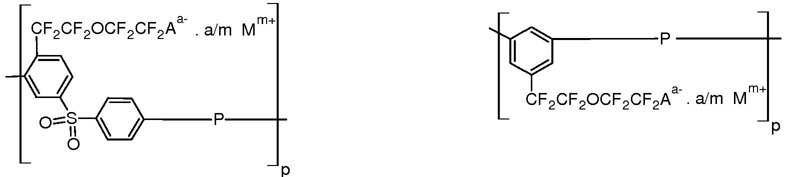 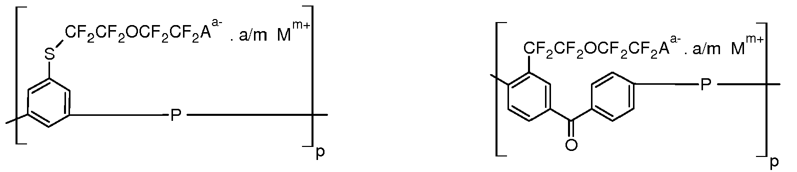 dans laquelle A, m, a, M, P et p sont tels que définis dans le premier objet de l'invention.

Dans un autre mode de réalisation, l'ionomère comprend en outre des chaînes polymères d'oxyde d'alkylène P' ayant la même définition que celle des chaînes polymères d'oxyde d'alkylène P, avec P' différente de P.

Dans ce cas, l'ionomère peut répondre à la formule (III-b₁), (III-c₁) ou (III-d₁) suivante : 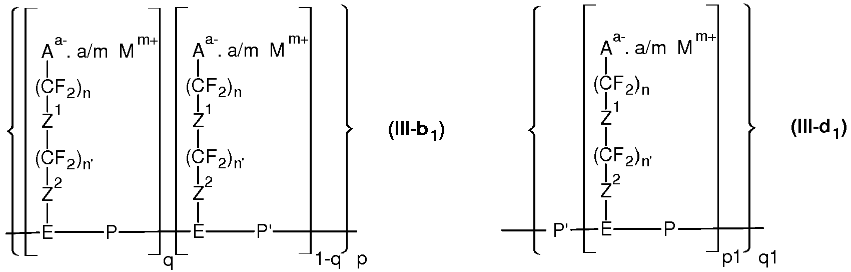 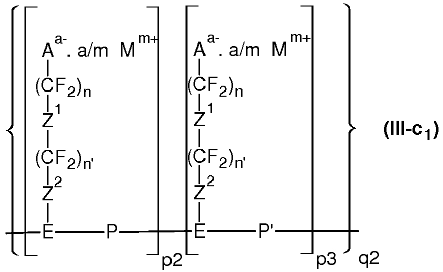 dans laquelle A, n, n', Z¹, Z², E, m, a, M, P, P' et p sont tels que définis dans le premier objet de l'invention, q, q₁, q₂, p₁, p₂ et p₃ ont la même définition que p.

Dans un autre mode de réalisation, l'ionomère comprend en outre des monomères ioniques difluorés (I') ayant la même définition que celle des monomères ioniques difluorés (I), avec les monomères étant différents.

Dans ce cas, l'ionomère peut répondre à la formule (III-b₂), (III-c₂) ou (III-d₂) suivante : 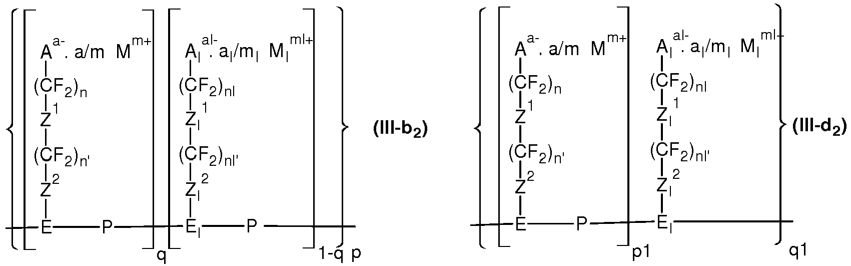 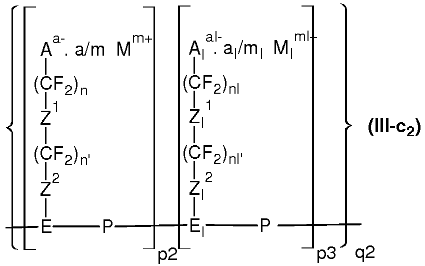 dans laquelle A, n, n', Z¹, Z², E, m, a, M, P et p sont tels que définis dans le premier objet de l'invention, q, q₁, q₂, p₁, p₂ et p₃ ont la même définition que p, et A_{I}, n_{I}, n_{I}', Z_{I}¹, Z_{I}², E_{I}, m_{I}, a_{I} et M_{I} ont respectivement la même définition que A, n, n', Z¹, Z², E, m, a et M, étant entendu que A_{I} différent de A et/ou n_{I} différent de n et/ou n_{I}' différent de n' et/ou Z_{I}¹ différent de Z¹ et/ou Z_{I}² différent de Z² et/ou E_{I} différent de E et/ou M_{I} différent de M.

Dans un mode de réalisation particulier, les parties ioniques ne diffèrent que par l'anion (i.e. A_{I}^{aI-} différent de A^{a-} seulement). Par exemple, A^{a-} peut être un anion sulfonate et A_{I}^{aI-} un anion sulfonimidure.

La chaîne polymère d'oxyde d'alkylène P peut être polycondensée grâce à ses groupes terminaux, avec un composé G tel que défini dans le troisième objet de l'invention (cf. 1^{ère} variante du procédé de l'invention).

Dans ce cas, l'ionomère de l'invention peut répondre à la formule (III-e) suivante : 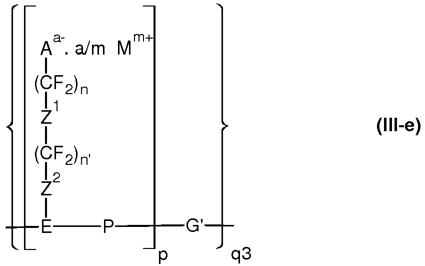 dans laquelle A, n, n', Z¹, Z², E, m, a, M, P et p sont tels que définis dans le premier objet de l'invention, q₃ a la même définition que p, et G' résulte de la polycondensation du composé G avec les groupes terminaux de P.

Par exemple, lorsqu'un composé G est un diisocyanate Ph(NCO)₂Me (diisocyanate de toluène) l'ionomère obtenu peut répondre à la formule (III-e') suivante : 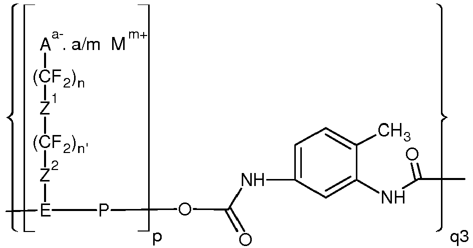 dans laquelle A, n, n', Z¹, Z², E, m, a, M, P et p sont tels que définis dans le premier objet de l'invention, et q₃ a la même définition que p.

Dans cet exemple, le diisocyanate de toluène est polycondensé avec la chaîne de polymère d'oxyde d'alkylène P par l'intermédiaire de ses groupes terminaux alcools pour former des ponts uréthanes.

Lorsque le composé G comprend une fonction post-polymérisable F² telle que définie dans le troisième objet de l'invention, un ionomère réticulé peut être obtenu, c'est-à-dire que des liaisons chimiques se sont formées suivant toutes les directions de l'espace afin de conduire à la formation d'un réseau trididmensionnel.

Dans un autre mode de réalisation, la chaîne polymère d'oxyde d'alkylène P peut être condensée grâce à ses groupes terminaux, avec un composé H tel que défini dans le troisième objet de l'invention (cf. 2^{ème} variante du procédé de l'invention).

Dans ce cas, l'ionomère obtenu est fonctionnalisée en bout de chaîne par le composé H et l'ionomère répond à la formule (III-f) suivante : 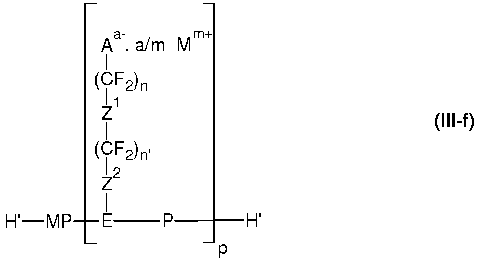 dans laquelle A, n, n', Z¹, Z², E, m, a, M, P et p sont tels que définis dans le premier objet de l'invention, MP correspond au monomère de la chaîne polymère P, et H' résulte de la polycondensation du composé H avec les groupes terminaux de la chaîne polymère P.

Par exemple, lorsqu'un composé H est un isocyanatopropyltriéthoxysilane de formule (EtO)₃Si(CH₂)₃(NCO), l'ionomère obtenu peut répondre à la formule (III-f') suivante : 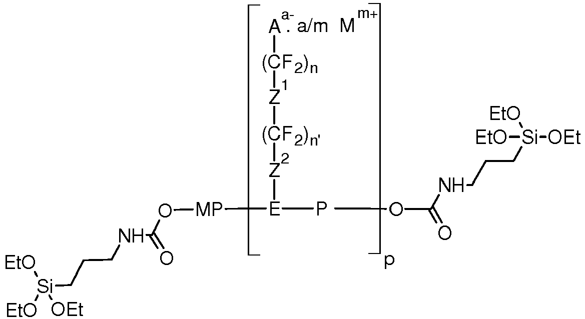 dans laquelle A, n, n', Z¹, Z², E, m, a, M, P et p sont tels que définis dans le premier objet de l'invention, et MP correspond au monomère de la chaîne polymère P.

Dans cet exemple, l'isocyanate est condensé avec un ionomère par l'intermédiaire de ses groupes terminaux alcools pour former des ponts uréthanes. L'ionomère obtenu est fonctionnalisé en bout de chaîne par le composé H et comprend des unités récurrentes UP (p unités).

Dans un autre mode de réalisation, les groupes terminaux, notamment les groupes alcool (OH) ou amine primaire (NH₂), de la chaîne polymère d'oxyde d'alkylène P peuvent être utilisés pour amorcer une polymérisation permettant d'accéder à des polymères di-blocs ou tri-blocs, notamment à base de polystyrène, de polyalphaméthylstyrène, de polyvinylpyridine, de polyacénaphtylène, de polyacrylate, de polyméthacrylate, de polyacrylonitrile ou de polyméthacrylonitrile (cf. 3^{ème} variante du procédé de l'invention).

Dans ce cas, l'ionomère peut répondre à la formule (III-g) suivante : 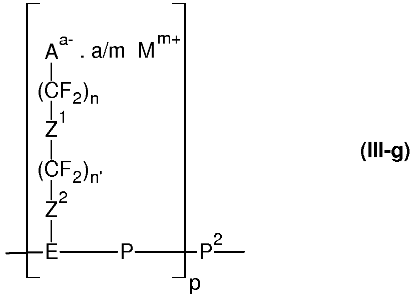 dans laquelle A, n, n', Z¹, Z², E, m, a, M, P et p sont tels que définis dans le premier objet de l'invention, et P₂ est un polymère choisi parmi un polystyrène, un polyalphaméthylstyrène, une polyvinylpyridine, un polyacénaphtylène, un polyacrylate, un polyméthacrylate, un polyacrylonitrile et un polyméthacrylonitrile.

Les ionomères de l'invention ont une grande aptitude à la dissociation en milieu aprotique.

De préférence, ils ont une conductivité ionique unipolaire supérieure ou égale à 10⁻⁶ S.cm⁻¹ à température ambiante, et de préférence supérieure ou égale à 10⁻⁵ S.cm⁻¹ à température ambiante.

L'ionomère de l'invention peut présenter une température de transition vitreuse T_{g} inférieure ou égale à -20°C environ, et de préférence inférieure ou égale à -40°C environ.

Les fonctions ioniques du ionomère de l'invention sont choisies de façon à optimiser la dissociation en milieu aprotique, tout en contrariant la cristallisation de la matrice polymère à base d'oxyde de polyalkylène de façon à optimiser la conductivité à température ambiante.

L'ionomère de l'invention a de préférence une masse molaire moyenne M_{w} (masse molaire moyenne en masse) allant de 1000 g/mol à 300 000 g/mol environ, et de préférence encore de 20 000 g/mol à 300 000 g/mol environ.

Dans un mode de réalisation particulier, l'ionomère présente un nombre de transport cationique supérieur ou égal à 0,9 environ, et de préférence égale à 1.

Dans l'ionomère de l'invention, seuls les cations M^{m+} transportent le courant lorsqu'il est utilisé dans un dispositif électrochimique.

L'ionomère peut atteindre un nombre de transport ionique proche de 1 du fait de l'immobilité des anions A^{a-}, obtenu grâce au greffage desdits anions dans un réseau tridimensionnel ou sur de longues chaînes de polymère unidimensionnel dont les longueurs sont largement supérieures au seuil d'enchevêtrement.

L'ionomère de l'invention peut comprendre en outre des fonctions post-polymérisables permettant l'obtention d'un polymère solvatant à conduction cationique tridimensionnel (ionomère réticulé). Un tel ionomère réticulé peut avoir un module de conservation allant de 1 à 5 MPa environ, et de préférence allant de 3 à 5 MPa environ.

L'invention a pour troisième objet une composition électrolyte, caractérisée en ce qu'elle comprend au moins un ionomère conforme au deuxième objet de la présente invention ou obtenu selon le procédé conforme au premier objet de la présente invention.

La composition électrolyte peut être uniquement constituée du ionomère de l'invention.

La composition électrolyte peut comprendre en outre un ou plusieurs solvants organiques, notamment choisis parmi les carbonates (e.g. des mélanges de carbonate de propylène, de carbonate d'éthylène et de diméthyl-carbonate en proportions variables), la N-méthylacétamide, la γ-butyrolactone, le diméthylformamide, la N-méthylpyrrolidone, les tétraalkylsulfamides, un liquide ionique à base d'ammonium quaternaire, et les éthers diméthyliques de polyéthylène glycol de masse comprise entre 90 et 2000.

Le ou les solvants organiques exercent : 1) un effet dissociant du fait de leur constante diélectrique élevée, et 2) un effet plastifiant favorisant la mobilité du cation M.

La quantité de solvants organiques est de préférence inférieure à 70% en masse, de préférence comprise entre 25% et 50% en masse. On obtient alors un électrolyte polymère gélifié. Dans ce cas, l'ionomère devra comporter un taux de fonction ionique élevé, c'est-à-dire avec des espaceurs polyéther n'excédant pas 400 g/mole. De préférence, on utilisera un ionomère de type copolymère à blocs pour assurer une bonne propriété mécanique.

La composition électrolyte peut comprendre en outre un ou plusieurs additifs choisis parmi les charges minérales, les sels de métal alcalin tels que les sels de lithium conventionnellement utilisés dans les électrolytes liquides (e.g. LiTFSI, LiClO₄ ou LiPF₆), les charges organiques telles que des charges organiques cellulosiques, les agents complexants, les agents ignifugeants et un de leurs mélanges.

L'agent complexant est spécifique du cation M tel que défini dans l'invention et peut favoriser la dissociation des paires d'ions et accroître la mobilité du cation M. Cet agent complexant, qui dépend de la nature du cation M, peut être un éther-couronne ou un cyclame, et peut être solide ou liquide.

Dans le cas des ionomères au lithium, on peut utiliser le tétraméthyl-tétraaza cyclotétradécane (solide) ou la sparteïne liquide qui bout à très haute température.

La charge minérale peut être de la silice.

La charge organique cellulosique peut être choisie parmi les fibres de cellulose, les nanofibres de cellulose et les microfibrilles de cellulose, lesdites fibres, nanofibres ou microfibrilles étant éventuellement fonctionnalisées par des groupes de type sulfonate -SO₃⁻ ou sulfate-ester -O-SO₃⁻.

Selon une forme de réalisation de l'invention, la composition électrolyte ne comprend pas de solvant et comprend de préférence au plus 10% en masse environ de sel(s) de métal alcalin (e.g. sel de lithium). On obtient ainsi une composition électrolyte « sans solvant » comprenant de faibles concentrations massiques de sel. Cette composition électrolyte présente l'avantage d'avoir une conductivité améliorée. Le nombre de transport cationique sera certes diminué mais la présence de l'ionomère s'opposera à la création d'un gradient de concentration en sel.

La composition électrolyte peut comprendre en outre un ou plusieurs polymères non-ioniques tel le poly(oxyéthylène).

Ce mode de réalisation est particulièrement avantageux pour fabriquer des électrodes composites dans lesquelles la composition électrolyte joue le rôle de liant.

Dans la composition électrolyte, l'ionomère peut être utilisé sous sa forme polycondensat saturé, sous sa forme polycondensat insaturé ou sous forme de copolymères à bloc incorporant le polycondensat saturé.

La composition électrolyte peut être mise en forme par pressage à chaud, par extrusion ou par enduction d'un substrat pour conduire à un électrolyte sous la forme d'un film, notamment lorsque la composition électrolyte comprend une charge organique cellulosique.

Lorsqu'une composition électrolyte selon l'invention est préparée sous la forme d'un film ou sous la forme d'un électrolyte polymère gélifié, elle est directement utilisable en tant que film d'électrolyte ou électrolyte polymère gélifié pour un dispositif électrochimique.

Dans le cas où l'on souhaite former un film, la préparation du film d'électrolyte peut comprendre la préparation d'une solution d'un ionomère de l'invention, et éventuellement d'un ou plusieurs solvants organiques et/ou d'un ou plusieurs additifs dans un solvant volatil, le dégazage de la solution obtenue, puis une étape au cours de laquelle on coule ladite solution sur un substrat, et le séchage du film sous vide par évaporation du solvant.

Comme solvant volatil tel que par exemple l'eau, l'acétonitrile, le diméthylformamide ou le dichlorométhane. On utilise de préférence un substrat dont la surface est revêtue d'une couche d'un matériau inerte et anti-adhésif tel que le polytétrafluoroéthylène. La surface du film coulé peut être délimitée par un anneau de verre collé sur la surface du substrat.

Lorsque l'ionomère comporte des doubles liaisons post-réticulables (fonctions F²), on peut avantageusement additionner à une solution aqueuse du ionomère, un amorceur thermique ou un photoamorceur radicalaire hydrosoluble, couler le film, le sécher, puis le réticuler soit par chauffage (amorçage thermique), soit par irradiation UV (photoamorceur).

Les procédés de fabrication dans l'eau sont très prisés des fabricants de batteries.

Lorsque l'ionomère réticulé ou non-réticulé est destiné à être renforcé par des charges organiques de type cellulosique, on peut disperser ces charges dans la solution aqueuse d'ionomère (e.g. obtenue après ultrafiltration) éventuellement en présence d'un amorceur thermique ou d'un photoamorceur radicalaire hydrosoluble, couler le film, le sécher, puis éventuellement le réticuler. On obtient ainsi un film renforcé par charges organiques de type cellulosique directement utilisable dans un dispositif électrochimique tel qu'une batterie.

Dans le cas où l'on souhaite fabriquer une électrode composite à l'aide de la composition électrolyte de l'invention en tant que liant, il convient de réaliser une encre comportant le matériau actif (e.g. LFP pour l'électrode positive et graphite ou LTO pour l'électrode négative), la composition électrolyte comprenant au moins un ionomère et un ou plusieurs polymères non-ioniques, et du carbone tel que du noir de carbone ou des nanotubes de carbone.

Le matériau actif peut également être un matériau actif organique tel qu'un polymère organique rédox (e.g. poly(phénothiazine) ou PPT) ou un matériau actif mixte organique/inorganique tel qu'un mélange PPT/LFP.

Lorsque le polycondensat est insaturé, l'encre comprend en outre un amorceur, à décomposition thermique ou photochimique (UV) pour réticuler l'encre une fois séchée. Il est donc possible de réticuler séparément l'ionomère avant la préparation de l'encre ou de réticuler l'encre directement, permettant ainsi d'améliorer les interfaces matériau actif/composition électrolyte/carbone au sein de l'électrode composite.

L'invention a pour quatrième objet l'utilisation d'un ionomère conforme au deuxième objet de la présente invention ou obtenu selon le procédé conforme au premier objet de la présente invention, pour la fabrication d'un électrolyte, notamment sous la forme d'un film d'électrolyte ou d'un électrolyte polymère gélifié, pour un dispositif électrochimique tel qu'une batterie (e.g. batteries lithium, lithium-ion, lithium-soufre, lithium-air, sodium, sodium-ion, magnésium, calcium, aluminium), un supercondensateur, un vitrage électrochrome ou une cellule solaire.

L'invention a pour cinquième objet l'utilisation d'un ionomère conforme au deuxième objet de la présente invention ou obtenu selon le procédé conforme au premier objet de la présente invention, comme liquide ionique.

Dans cette utilisation, la masse molaire du ionomère est inférieure ou égale à 20 000 g/mol environ, et de préférence inférieure ou égale à 2000 g/mole environ.

A l'inverse des liquides ioniques conventionnels à base d'ammonium quaternaire, les liquides ioniques de l'invention participent directement aux réactions électrochimiques et ne nécessitent pas d'additionner un sel de lithium (batteries au lithium) ou un sel de sodium, magnésium, calcium (batteries au Na, Mg, Ca).

Les liquides ioniques conventionnels sont très difficiles à purifier car ils sont amorphes, liquides à basse température et ne peuvent être ni distillés ni sublimés, ce qui rend très coûteux les liquides ioniques de grade batterie. En choisissant des liquides ioniques de l'invention de masse molaire > 1000 g/mole, on peut aisément les purifier par ultrafiltration dans l'eau.

Les ionomères de l'invention en tant que liquides ioniques peuvent être utilisés en mélange avec une membrane dense à base de polymère de type POE, ou avec une membrane macroporeuse de type Celgard® ou PVdF. L'ionomère utilisé comme liquide ionique remplit alors les pores de la membrane macroporeuse. Si l'ionomère n'est pas réticulé, il va avoir un nombre de transport cationique d'environ 0,7. Une fois les pores de la membrane macroporeuse remplis par l'ionomère, celui-ci peut être réticulé pour former un polyliquide ionique présentant un nombre de transport cationique égal à 1.

Les ionomères de l'invention en tant que liquides ioniques peuvent être également utilisés en mélange avec des polymères neutres de forte masse molaire tels que le POE 300000 pour réaliser des électrodes composites.

L'invention a pour sixième objet l'utilisation d'un ionomère conforme au deuxième objet de la présente invention ou obtenu selon le procédé conforme au premier objet de la présente invention, pour la réalisation d'électrodes composites, c'est-à-dire comme constituant d'une d'électrode composite, en particulier comme liant conducteur d'une d'électrode composite.

L'utilisation d'ionomères comme liant d'électrode permet de lui conférer une conduction ionique, l'électrode composite possédant alors une conduction électronique assurée par l'agent de conduction (e.g. noir de carbone), une conduction ionique assurée par l'ionomère et un matériau actif. Le fait d'utiliser des ionomères de longueur de chaîne soit très supérieure au seuil d'enchevêtrement (ionomère non-réticulé), soit infinie (ionomère réticulé) garantit que les ionomères ne migreront pas lors des cycles de charge/décharge, évitant ainsi un appauvrissement en sel de l'électrode composite.

L'ionomère de l'invention a ainsi une quadruple fonction : de solvant macromoléculaire, de conducteur cationique unipolaire, de séparateur et de liant ionique d'électrode.

L'ionomère selon la présente invention peut également être utilisé comme constituant de membranes sélectives ou de membranes de référence dans les capteurs à membrane.

L'invention a pour septième objet un dispositif électrochimique comprenant au moins une électrode négative et au moins une électrode positive séparées par une composition électrolyte, caractérisé en ce que la composition électrolyte est conforme au troisième objet de l'invention.

Dans un mode de réalisation particulier, l'électrode positive (respectivement l'électrode négative) est une électrode composite et elle comprend une composition électrolyte conforme au cinquième objet de l'invention, notamment à titre de liant conducteur.

Un tel dispositif électrochimique peut être une batterie électrique, un supercondensateur, un vitrage électrochrome ou une cellule solaire, et de préférence une batterie lithium, lithium-polymère lithium-ion, lithium-soufre, lithium-air, sodium, magnésium ou calcium.

### EXEMPLES

Sauf indications contraires, toutes les matières premières des exemples ont été utilisées tels que reçues des fabricants.

Les matériaux préparés (e.g. monomères et/ou ionomères) ont été caractérisés par :
- résonance magnétique nucléaire du proton et/ou du fluor (RMN),
- mesure de la conductivité ionique par spectrométrie d'impédance électrochimique,
- calorimétrie différentielle à balayage (bien connue sous l'anglicisme « *Differential Scanning Calorimetry* » ou DSC),
- analyse thermomécanique (bien connue sous l'anglicisme « *Dynamic Mechanic Analysis* » ou DMA),
- mesure du nombre de transport cationique par spectrométrie d'impédance à basse fréquences,
- mesure de la masse molaire par chromatographie d'exclusion stérique à l'aide d'une HPLC Waters 515 couplée à un détecteur multiangulaire à diffusion de la lumière Wyatt Dawn EOS à 690 nm environ (bien connue sous l'anglicisme « *Size Exclusion Chromatography coupled to Multi-Angle Laser Light Scattering* » ou SEC-MALLS).

Les analyses de résonance magnétique nucléaire du fluor et de l'hydrogène ont été effectuées à l'aide d'un appareil vendu sous la dénomination commerciale AVANCE III HD, par la société Bruker, avec les paramètres suivants : fréquences 400,15 MHz pour la RMN du proton (¹H RMN) et 376,52 MHz pour la RMN du fluo (¹⁹F RMN).

Les mesures de conductivité ionique ont été effectuées à l'aide d'un impédancemètre HP 4192A, vendu par la société Hewlett Packard et fonctionnant dans la gamme de fréquences 5 Hz - 13 MHz, l'amplitude du signal sinusoïdal étant fixée à +/-10 mV. Les mesures ont été effectuées à une température allant de 20°C à 90°C dans une étuve thermostatée. Les mesures ont été effectuées tous les 10°C après une stabilisation en température de 1 heure, notamment lors de la descente en température. Les échantillons ont été placés en boîte à gant sous argon dans des cellules Swagelok®. Chaque mesure a été réalisée deux fois afin de s'assurer de la reproductibilité des conductivités ioniques déterminées.

Les analyses par calorimétrie différentielle à balayage ont été effectuées avec un appareil vendu sous la dénomination commerciale DSC 1 STARe system par la société Mettler Toledo. Elles permettent d'obtenir la température de transition vitreuse des ionomères obtenus.

Les analyses thermomécaniques (i.e. mesure du module de conservation) ont été effectuées à l'aide d'un appareil vendu sous la dénomination commerciale DMA Q800 par la société TA instruments (Waters).

La chromatographie d'exclusion stérique a été effectuée sur des colonnes C₁₈ Agilent 2xPLgel-Mixed-D en utilisant comme solvant d'élution du nitrate de sodium à 0,1M dans du diméthylformamide. La vitesse d'élution du solvant était de 1 ml/min environ.

Le nombre de transport cationique a été déterminé par la méthode décrite par Sorensen et al. [Electrochimica Acta, 1982, 27, 12, 1671-1675] qui utilise la spectroscopie d'impédance électrochimique à basse fréquence. Cette impédance de diffusion peut-être représentée par une impédance de Warburg comprise dans un circuit électrique équivalent. Le nombre de transport cationique a été mesuré à 70°C environ dans une cellule souple symétrique lithium/électrolyte/lithium de type « *coffee bag ».*

### Exemple 1 : préparation d'un monomère ionique difluoré M¹

Le schéma de synthèse pour conduire au monomère ionique difluoré **M¹** est le suivant :

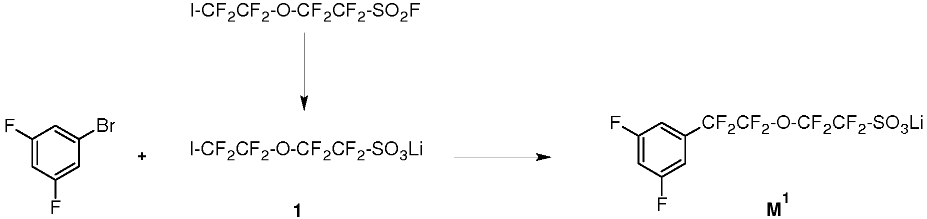

### 1.1) Première étape : synthèse du composé 1 (5-iodooctafluoro-3-oxapentanesulfonate de lithium)

Le composé **1** a été obtenu par hydrolyse du fluorure de 5-iodooctafluoro-3-pentanesulfonyl dans un solvant organique en présence de LiOH (lithine).

En particulier, 10,22 g (0,024 mole) de fluorure de 5-iodooctafluoro-3-pentanesulfonyl ont été solubilisés dans 48 ml de tétrahydrofurane (THF). Puis, 2,22 g (0,053 mole) de lithine ont été introduits dans la solution de THF et le milieu réactionnel résultant a été maintenu sous agitation vigoureuse pendant 12h à température ambiante et sous atmosphère inerte.

La disparition du produit de départ dans le milieu réactionnel (i.e. à la fin de l'hydrolyse) a été suivie et contrôlée par analyse RMN du fluor (¹⁹F RMN) grâce à la disparition du pic se trouvant à 52 ppm et correspondant au fluor dans le motif « SO₂F ».

Le milieu réactionnel a été filtré afin d'éliminer l'excès de lithine et le THF a été évaporé. Le résidu obtenu a été solubilisé dans de l'acétonitrile, puis filtré sur un filtre ayant un seuil de filtration de 0,2 µm environ. Le composé 1 a été obtenu avec un rendement de 85% environ sous la forme d'une poudre blanche, après évaporation de l'acétonitrile puis séchage pendant 24h à 80°C environ sous pression réduite. Il a été stocké à l'abri de l'air sous atmosphère inerte.

Le composé **1** a été caractérisé par RMN du fluor :
¹⁹F RMN : δ (ppm, acétone-d₆) = -118,6 (*s*, CF₂SO₃Li) ; -86,52 (*m*, CF₂O) ; -82,95 (*t*, CF₂O) ; -69,06 (s, ICF₂).

Un procédé similaire a été utilisé pour préparer le 5-iodooctafluoro-3-oxapentanesulfonate de sodium (rendement de 98%, composé **2**) et le 5-iodooctafluoro-3-oxapentanesulfonate de potassium (rendement de 98%, composé **3**).

Le composé **3** a été caractérisé par RMN du fluor :
¹⁹F RMN : δ (ppm, acétone-d₆) = -118,6 (*s*, CF₂SO₃K) ; -86,52 (*m*, CF₂O) ; -82,94 (*t*, CF₂O) ; -69,10 (s, ICF₂).

### 1.2) Deuxième étape : synthèse du composé M¹" (3,5-(difluorophényl)octafluoro-3-oxapentanesulfonate de potassium)

Une solution comprenant 8,25 g (0,130 mole) de cuivre (0) et 7,5 ml (0,065 mole) de 1-bromo-3,5-difluorobenzene dans 10 ml de diméthylsulfoxyde distillé (DMSO) a été préparée. Le milieu réactionnel résultant a été maintenu sous agitation pendant 1h30 à 115-120°C environ.

Une solution comprenant 15,0 g (0,032 mole) de composé **3** (forme K⁺) dans 17 ml de DMSO a été préparée séparément puis ajoutée au milieu réactionnel. Le milieu réactionnel résultant a été maintenu sous agitation pendant 3h à 125°C environ puis refroidi à température ambiante, filtré sur de la Célite®545 afin d'enlever l'excès de cuivre, et versé dans 200 ml d'une solution aqueuse saturée de chlorure de sodium (saumure). Le composé a été extrait 3 fois avec 200 ml d'acétate d'éthyle. Puis, les phases organiques ont été réunies, lavées une fois avec 400 ml d'eau, séchées sur Na₂SO₄, filtrées et les solvants ont été évaporés. Le résidu obtenu (solide jaunâtre) a été lavé avec de l'hexane, puis du toluène et du dichlorométhane jusqu'à ce que le solvant de lavage soit transparent. Le monomère **M¹"** a été obtenu avec un rendement de 75% environ, sous la forme d'un solide blanc.

Le monomère **M¹"** a été caractérisé par RMN du fluor et du proton :
¹H RMN : δ (ppm, acétone-d₆) = 7,34 (*t-t*, 1 H_{Ar}) ; 7,50 *(d-d,* 2 H_{Ar}).
¹⁹F RMN : δ (ppm, acétone-d₆) = -119,11 (s, CF₂SO₃K) ; -114,34 (*t*, CF₂Ar) ; -108,70 (*t*, 2 F_{Ar}); -88,38 (m, CF₂O) ; -83,48 (*m*, CF₂O).

Un procédé similaire a été utilisé pour préparer le monomère 3,5-(difluorophényl)octafluoro-3-oxapentanesulfonate de lithium (monomère **M¹)** et le monomère 3,5-(difluorophényl)octafluoro-3-oxapentanesulfonate de sodium (monomère **M¹')** avec des rendements respectifs de 76% et 78%.

### Exemple 2 : préparation d'un monomère ionique difluoré M²

Le schéma de synthèse pour conduire au monomère ionique difluoré **M²** est le suivant :

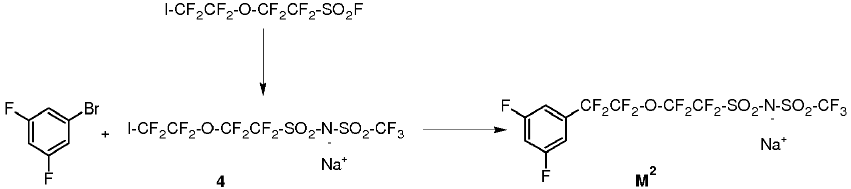

### 2.1) Première étape : synthèse du composé 4 (5-iodooctafluoro-3-oxapentane trifluométhane de sulfonimidure de sodium)

Dans un ballon bicol, 25,82 mmole de trifluorométhanesulfonamide et 49,30 mmoles de triéthylamine ont été dissous dans 20 ml d'acétonitrile (ACN), fraîchement distillé à partir d'hydrure de calcium, et le mélange résultant a été mis sous agitation. Puis, 23,47 mmoles de fluorure de 5-iodooctafluoro-3-oxapentanesulfonyl ont été ajoutées et le mélange résultant a été chauffé à 40°C environ pendant 36 à 40h.

La disparition du produit de départ dans le milieu réactionnel (i.e. à la fin de l'hydrolyse) a été suivie et contrôlée par analyse RMN du fluor (¹⁹F RMN) grâce à la disparition du pic se trouvant à 52 ppm et correspondant au fluor dans le motif « SO₂F ».

Le solvant a été évaporé sous pression réduite à 40 °C environ. Puis le résidu obtenu a été dissous dans du dichlorométhane, lavé avec 1000 ml d'eau distillée, et séché sur du sulfate de magnésium. Les solvants ont été évaporés sous pression réduite à 40 °C environ. Puis, le résidu obtenu a été dissous dans une solution aqueuse d'hydroxyde de sodium (NaOH) de manière à avoir un excès molaire en NaOH de 5% environ. Après agitation pendant 15 minutes, l'eau a été éliminée par lyophilisation. NaOH a permis de faire l'échange entre l'ammonium et le sodium. Une huile visqueuse a été obtenue puis a été dissoute dans de l'acétonitrile, séchée sur du sulfate de magnésium, filtrée et les solvants ont été évaporés sous pression réduite à 40 °C environ. Le résidu obtenu a été recristallisé dans de l'anisole pour conduire au composé **4.**

### 2.2) Deuxième étape : synthèse du monomère M² (3,5-(difluorophényl)octafluoro-3-oxapentane trifluorométhane sulfonimidure de sodium)

Dans un ballon muni d'un réfrigérant et d'un thermomètre, 4 équivalents de cuivre (0) (2,265 g, 35,64 mmole) et 2 équivalents de 1-bromo-2,5-difluorobenzène (3,44 g, 17,82 mmole) ont été dissous dans 10 ml de DMSO non distillé. Le milieu réactionnel résultant a été maintenu sous agitation pendant 1h30 à une température allant de 115 à 120°C environ et sous atmosphère inerte. Puis, la température a été abaissée à 70°C environ.

Une solution comprenant 1 équivalent de composé **4** (5 g, 8,91 mmole) dans 7 ml de DMSO a été préparée séparément et a été ajoutée au milieu réactionnel. La température du mélange résultant a été portée à 127°C environ. A la fin de la réaction, le mélange résultant a été filtré sur de la Célite®545 pour éliminer l'excès de cuivre, puis versé dans 200 ml d'une solution aqueuse saturée de chlorure de sodium. Le composé a été extrait 3 fois avec 200 ml d'acétate d'éthyle. Puis, les phases organiques ont été réunies, séchées sur Na₂SO₄, filtrées et les solvants ont été évaporés. Le résidu obtenu a été lavé avec de l'hexane, puis du toluène et du dichlorométhane jusqu'à ce que le solvant de lavage soit transparent. Le monomère **M²** a été obtenu avec un rendement de 70% environ.

Le monomère **M²** a été caractérisé par RMN du fluor et du carbone :
¹³C RMN : δ (ppm, acétone-d₆) = 120,7 (q, J = 321,5Hz, C9) ; 112,8 (tt, J = 294,6Hz, J = 34,6Hz, C6) ; 117,6 (tt, J = 287,5Hz, J = 31,3Hz, C8) ; 117,9 (tt, J = 287,5Hz, J = 37,1Hz, C7) ; 163,9 (dd, J = 12,6Hz, J =250,5Hz, C2) ; 132,5 (septuplet, J = 9,8Hz, C4) ; 111,4 (dt, J = 27,7Hz, J = 7,4Hz, C3) ; 108,4 (t, J = 24.7Hz, C1) ; 113,7 (tt, J = 255,4Hz, J = 33,1Hz, C5).
¹⁹F RMN : δ (ppm, acétone-d₆) = -79,87 (s, 3F, F₉) ; -82,16 (s, 2F, F₆) ; -88,15 (s, 2F, F₇) ; -108,53 (s, 2F, F₂) ; 114,66 (s, 2F, F₅) ; -117,27 (s, 2F, F₈).

### Exemple 3 : préparation d'un monomère ionique difluoré M³

Le schéma de synthèse pour conduire au monomère ionique difluoré **M³** est le suivant :

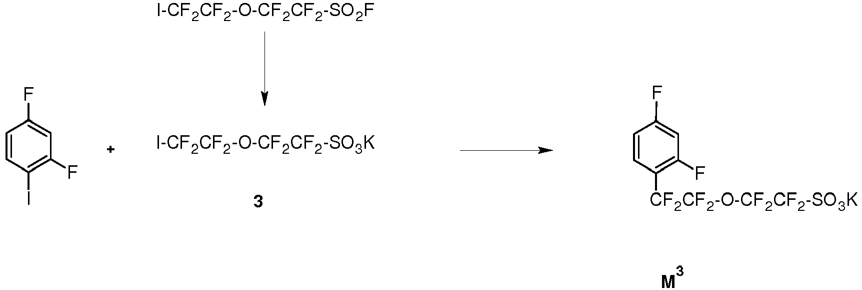

### 3.1) Première étape : synthèse du composé 3 (5-iodooctafluoro-3-oxapentanesulfonate de potassium)

Le composé **3** a été obtenu par hydrolyse du fluorure de 5-iodooctafluoro-3-pentanesulfonyl dans un solvant organique en présence de KOH selon le procédé tel que décrit dans l'exemple 1.1).

### 3.2) Deuxième étape : synthèse du composé M³ (2,4-difluorophényl)octafluoro-3-oxapentanesulfonate de potassium)

Dans un ballon tricol de 50 ml équipé d'un réfrigérant, sous atmosphère d'azote et sous agitation, ont été placés 1,0 g de 2,4-difluoro-1-iodobenzène (4,08 mmoles), 2,23 g de cuivre bronze (numéro CAS : 158113-12-3, alliage de cuivre et d'étain comprenant 90% en masse de cuivre et 10% en masse d'étain, 12,2 mmoles), 0,064 g de bipyridine (0,41 mmole) dans 5 ml de DMSO. La solution résultante a été chauffée à 80°C environ, puis 0,94 g de composé **3** (2,04 mmoles) ont été ajoutés. La température du milieu réactionnel a été augmentée à 130°C environ et maintenue pendant 5h. Le milieu réactionnel a été refroidi et versé dans de l'eau déionisée. La solution a ensuite été filtrée sur de la Célite®545 afin de conduire à un filtrat clair. Les solvants ont été évaporés puis le résidu obtenu a été extrait avec de l'acétate d'éthyle pendant 48h à l'aide d'un montage Soxhlet. La phase organique a été lavée 3 fois avec une solution aqueuse à 2M d'acide chlorhydrique (HCl), une solution de bicarbonate de sodium et 3 fois avec de l'eau déionisée. La phase organique résultante a été séchée sur du sulfate de sodium et les solvants ont été évaporés sous vide. Le résidu obtenu a été séché sous vide pendant 24h puis placé dans un dessiccateur contenant du P₂O₅. Le monomère ionique **M³** a été obtenu avec un rendement de 70% environ.

Le monomère **M³** a été caractérisé par RMN du fluor et du proton :
¹H RMN : δ (ppm, DMSO-d₆) = 7,8 (*d-d,* 1 H_{Ar}) ; 7,55 *(d-d,* 1 H_{Ar}) ; 7,3 (d-d, 1H_{Ar}).
¹⁹F RMN : δ (ppm, DMSO-d₆) = -118 (*s*, CF₂SO₃K) ; -111,5 (m, CF₂Ar) ; -108,4 (*m*, 1 F_{Ar}); -102,6(*m*, 1 F_{Ar}); -87,5 (m, CF₂O) ; -82,3 (*m*, CF₂O).

### Exemple 4 : préparation d'un monomère ionique difluoré M⁴

Le schéma de synthèse pour conduire au monomère ionique difluoré **M⁴** est le suivant :

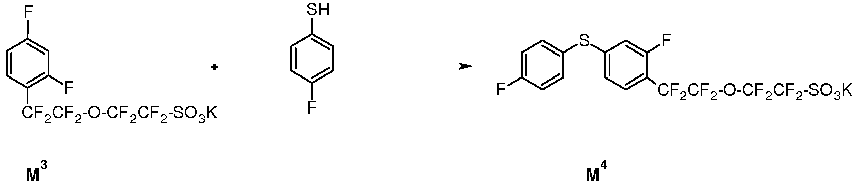

### 4.1) Première étape : synthèse du monomère M³

Le monomère **M³** a été obtenu selon le procédé tel que décrit dans l'exemple 3.

### 4.2) Deuxième étape : synthèse du composé M⁴ (3-{1,1,2,2-tétrafluoro-2-[1,1,2,2-tétrafluoro-2(potassiumoxysulfonyl)éthoxyléthyl}-4,4'-difluorodiphénylsulfide)

Dans un ballon tricol de 50 ml équipé d'un réfrigérant, sous atmosphère d'azote et sous agitation, ont été placés 0,64 g de fluorothiophénol (4,9 mmoles), 2 g de monomère **M³** (4,46 mmoles), 2,18 g de Cs₂CO₃ (6,69 mmoles) dans 8 ml de DMSO. La solution résultante a été chauffée à 65°C environ pendant 16h, puis le milieu réactionnel résultant a été dilué dans de l'eau et extrait avec de l'acétate d'éthyle. La phase organique a été lavée avec de l'eau puis séchée sur du sulfate de magnésium. Les solvants ont été évaporés sous vide pour conduire à un résidu. Ledit résidu a été purifié par chromatographie en utilisant une colonne de silice de type C₁₈ et un éluant méthanol/eau (v/v, 55/45). Le monomère ionique **M⁴** a été obtenu avec un rendement de 65% environ.

Le monomère **M⁴** a été caractérisé par RMN du fluor et du proton :
¹H RMN : δ (ppm, DMSO-d₆) = 7,8 *(d-d,* 1 H_{Ar}) ; 7,7- 7,5 (*m*, 4 H_{Ar}) ; 7,3 (d-d, 2H_{A}r).
¹⁹F RMN : δ (ppm, DMSO-d₆) = -118 (*s*, CF₂SO₃K) ; -112 (m, CF₂Ar) ; -113,4 (*m*, 1 F_{Ar}); -111,2 (*m*, 1 F_{Ar}); -87,5 (m, CF₂O) ; -82,3 (*m*, CF₂O).

### Exemple 5 : préparation d'un monomère ionique difluoré M⁵ conforme au premier objet de l'invention

Le schéma de synthèse pour conduire au monomère ionique difluoré **M⁵** est le suivant :

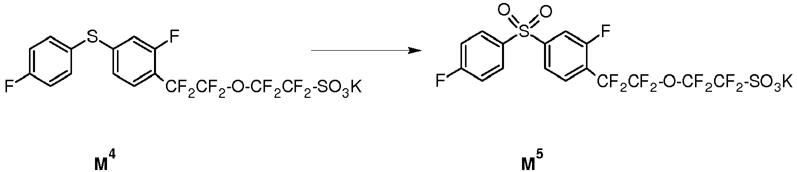

### 5.1) Première étape : synthèse du monomère M⁴

Le monomère **M⁴** a été obtenu selon le procédé tel que décrit dans l'exemple 4.

### 5.2) Deuxième étape : synthèse du composé M⁵ (4-{1,1,2,2-tétrafluoro-2-[1,1,2,2-tétrafluoro-2(potassiumoxysulfonyl)éthoxy]éthyl}-3,4'-difluorodiphényl sulfone)

Dans un ballon ont été placés 2 g de monomère **M⁴** (3,59 mmoles) dans 78 ml de méthanol. A la solution résultante a été ajoutée une solution comprenant 4,42 g d'oxone (hydrogénopersulfate de potassium, 7,18 mmoles) dans 4 ml d'eau. Le milieu réactionnel résultant a été maintenu sous agitation à température ambiante pendant 5h. Le milieu réactionnel a ensuite été versé dans une solution aqueuse à 1M d'HCl et extrait avec de l'acétate d'éthyle. Les phases organiques ont été réunies et lavées avec une solution aqueuse à 1M d'HCI et une solution saturée en NaCl puis séchées sur du sulfate de sodium. Les solvants ont été évaporés sous vide pour conduire à un résidu. Ledit résidu a été purifié par chromatographie en utilisant une colonne de silice de type C₁₈ et un éluant méthanol/eau (v/v, 55/45). Le monomère ionique **M⁵** a été obtenu avec un rendement de 60% environ.

Le monomère **M⁵** a été caractérisé par RMN du fluor et du proton :
¹H RMN : δ (ppm, DMSO-d₆) = 8,2 (*m*, 3 H_{Ar}) ; 7,95 (*s et d,* 2 H_{Ar}) ; 7,5 (d-d, 2H_{Ar}).
¹⁹F RMN : δ (ppm, DMSO-d₆) = -118 (s, CF₂SO₃K) ; -111,3 (m, CF₂Ar) ; -108,7 (*m*, 1 F_{Ar}); -103,4 (*m*, 1 F_{Ar}); -86,9 (m, CF₂O) ; -82,2 (*m*, CF₂O).

### Exemple 6 : préparation d'un monomère ionique difluoré M⁶

Le schéma de synthèse pour conduire au monomère ionique difluoré **M⁶** est le suivant :

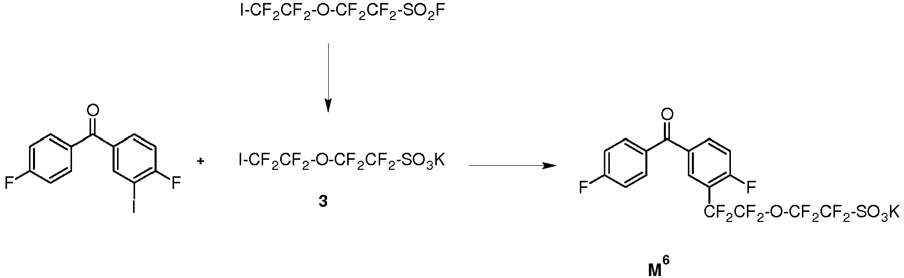

### 6.1) Première étape : synthèse du composé 3 (5-iodooctafluoro-3-oxapentanesulfonate de potassium)

Le composé **3** a été obtenu par hydrolyse du fluorure de 5-iodooctafluoro-3-pentanesulfonyl dans un solvant organique en présence de KOH selon le procédé tel que décrit dans l'exemple 1.1).

### 6.2) Deuxième étape : synthèse du composé M⁶ (3-{1,1,2,2-tétrafluoro-2-[1,1,2,2-tétrafluoro-2(potassiumoxysulfonyl)éthoxyléthyl}-4,4'-difluorobenzophénone)

Dans un ballon tricol de 250 ml équipé d'un réfrigérant, sous atmosphère d'azote et sous agitation, ont été placés 6,5 g de 4,4'-difluoro-3-iodobenzophénone (18,89 mmoles), 13,24 g de cuivre bronze (72,65 mmoles), 0,45 g de bipyridine (2,9 mmoles) dans 30 ml de DMSO. La solution résultante a été chauffée à 80°C environ, puis 6,7 g de composé **3** (14,53 mmoles) ont été ajoutés. En même temps, la température du milieu réactionnel a été augmentée à 130°C environ et maintenue pendant 6h. Le milieu réactionnel a été refroidi et versé dans de l'eau déionisée. La solution a ensuite été filtrée sur de la Célite®545 afin de conduire à un filtrat clair. Les solvants ont été évaporés puis le résidu obtenu a été extrait avec de l'acétate d'éthyle pendant 48h à l'aide d'un montage Soxhlet. Les solvants ont été évaporés sous vide et le monomère ionique **M⁶** a été obtenu avec un rendement de 70% environ.

Le monomère **M⁶** a été caractérisé par RMN du fluor et du proton :
¹H RMN : δ (ppm, DMSO-d₆) = 8,1 (*m*, 1 H_{Ar}) ; 7,92 *(d-d,* 1 H_{Ar}) ; 7,7 (2 d, 2H_{Ar}) ; 7,34 (d-d,1H_{Ar}) ; 7,4 (2 d, 2H_{Ar}).
¹⁹F RMN : δ (ppm, DMSO-d₆) = -118 (s, CF₂SO₃K) ; -112,4 (m, CF₂Ar) ; -107,2 (*m*, 1 F_{Ar}); -103,2 (*m*, 1 F_{Ar}); -87,5 (m, CF₂O) ; -82,3 (*m*, CF₂O).

### Exemple 7 : préparation d'ionomères à conduction cationique I¹ et I^{1'} conformes au deuxième objet de l'invention

Le schéma de synthèse pour conduire aux ionomères **I^{1'}** et **I¹** est le suivant :

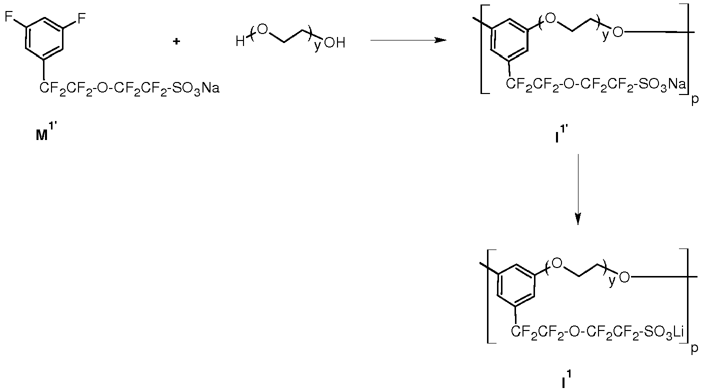

Dans un ballon tricol sous atmosphère d'argon, 0,268 g de NaH (10,65 mmoles, 2,3 équivalents) ont été placés. Dans un autre ballon, une solution de 4,62 g de polyéthylène glycol de masse molaire 1000 g/mol (PEG1000, y = 22,7) (4,62 mmoles, 1 équivalent) dans 10 ml de diglyme a été préparée. Puis, 2 à 3 ml de diglyme ont été ajoutés dans le ballon contenant le NaH et la solution résultante a été ajoutée lentement à la solution contenant le PEG1000. Le milieu réactionnel résultant a été chauffé à 65°C environ pendant 3h30 sous atmosphère d'argon.

Dans un autre ballon, une solution contenant 2 g du monomère **M^{1'}** 3,5-(difluorophényl)octafluoro-3-oxapentanesulfonate de sodium tel que préparé dans l'exemple 1 (4,62 mmole, 1 équivalent) dans 5 ml de diglyme a été préparée et la solution a été ajoutée lentement au milieu réactionnel. Le milieu réactionnel résultant a été chauffé à 140°C environ pendant 24h. Puis, il a été refroidi à température ambiante et précipité dans le pentane pour conduire à un solide contenant l'ionomère **I^{1'}**. Le solide a été filtré puis dissous dans de l'acétonitrile. La solution obtenue a été filtrée afin d'éliminer les sels inorganiques. Le filtrat a été évaporé au rotavapor afin d'obtenir l'ionomère sous la forme d'un liquide visqueux. Celui-ci a été séché sous vide à 100°C pendant 48h.

L'échange du cation sodium par le cation lithium a été effectué par ultrafiltration. L'ionomère **I^{1'}** a été dissous dans une solution aqueuse à 1M de LiCl, puis filtré sous pression avec une membrane vendue par la société Millipore sous la référence commerciale Ultracel® 3kDa par la société Sodipro ayant un seuil de coupure de 1000 g/mol environ (membrane d'ultrafiltration en cellulose). Le NaCl formé était aussi soluble dans l'eau mais passait à travers la membrane. Lorsque la solution est devenue visqueuse, plusieurs solutions aqueuses à 1M de LiCI ont été ajoutées afin de saturer le milieu en ions lithium pour mieux favoriser l'échange entre les cations. Un lavage à l'eau afin d'éliminer le maximum de NaCl formé a été effectué plusieurs fois. Cette opération a été effectuée pendant au moins 24h puis la solution résultante a été lyophilisée. Le résidu obtenu a été dissous dans l'acétonitrile et filtré avec un papier filtre puis avec des microfiltres de 0,2 µm environ afin d'éliminer l'excès de LiCI et le NaCl restant. Les solvants ont été évaporés sous pression réduite (10⁻² bars) et l'ionomère **I¹** obtenu a été séché sous vide à 60°C environ 24 h (rendement après lavage de 71% environ).

L'ionomère **I¹** a été caractérisé par RMN du fluor, du carbone et du proton :
¹H RMN : δ (ppm, acétone-d₆) = 6,92 (s, 2H, H₃) ; 6,74 (s, 1H, H₁) ; 4,25 (t, 4H, H₉, J=3,9Hz,) ; 3,84 (t, 4H, H₁₀, J=3,9Hz) ; 3,62 (s, 90H, H₁₁, -O-(CH₂CH₂-O)-).
¹³C RMN : δ (ppm, acétone-d₆) = 162,1 (s, 2C, C₂) ; 132,5 (t, 1C, C₄, J = 24,9Hz) ; 107,3 (t, 2C, C₃, J = 5,9Hz) ; 107,1 (s, 1C, C₁); 113,9 (m, 1C, C₆) ; 119,4 (m, 1C, C₈) ; 122,1 (m, 1C, C₇) ; 116,7 (m, 1C, C₅) ; 73,0 (s, 2C, C₉) ; 71,8 (s, 45C, C₁₁, (CH₂CH₂-O)ₙ-) ; 69,9 (s, 2C, C₁₀).
¹⁹F RMN : δ (ppm, acétone-d₆) = -83,37 (s, 2F, F₆) ; -88,08 (s, 2F, F₇) ; -113,88 (s, 2F, F₅) ; -118,80 (s, 2F, F₈).

La masse molaire moyenne en nombre Mₙ du ionomère **I¹** était de 21000 g/mol environ et sa masse molaire moyenne en masse M_{w} était de 36700 g/mol environ.

L'ionomère **I¹** avait une valeur p de 15 environ et une valeur y de 22,7 environ.

### Exemple 8 : préparation d'ionomères à conduction cationique I^{2'}, I², I^{3'} et I³ conformes au deuxième objet de l'invention

Le schéma de synthèse pour conduire aux ionomères **I^{2'}, I², I^{3'}** et **I³** est le suivant :

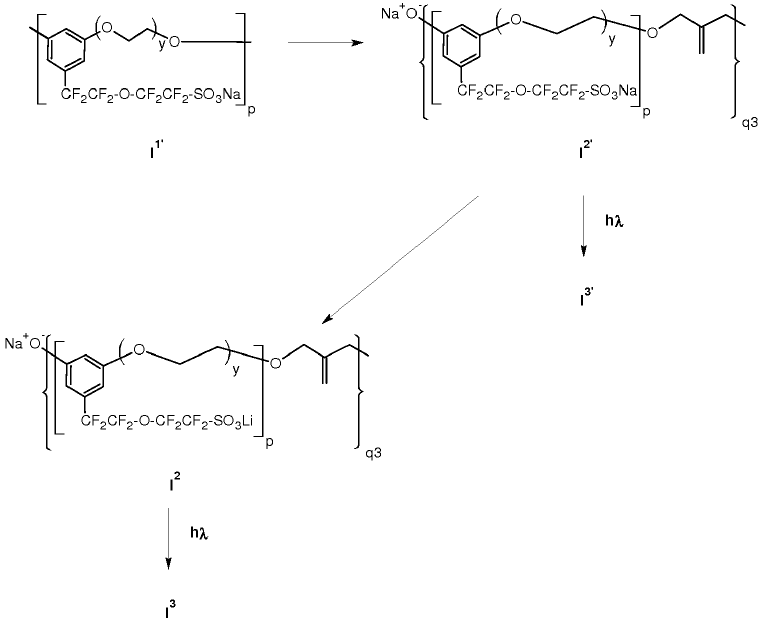

Dans un ballon tricol sous atmosphère d'argon, 0,315 g de NaH (12,5 mmoles, 2,7 équivalents) ont été placés. Dans un autre ballon, une solution de 5,32 g de polyéthylène glycol de masse molaire 1000 g/mol (PEG1000, n = 22,7) (5,32 mmoles, 1,15 équivalents) dans 6 ml de diglyme a été préparée. Puis, 2 à 3 ml de diglyme ont été ajoutés dans le ballon contenant le NaH et la solution résultante a été ajoutée lentement à la solution contenant le PEG1000. Le milieu réactionnel résultant a été chauffé à 65°C environ pendant 3h30 sous atmosphère d'argon.

Dans un autre ballon, une solution contenant 2 g du monomère **M¹'** 3,5-(difluorophényl)octafluoro-3-oxapentanesulfonate de sodium tel que préparé dans l'exemple 1 (4,62 mmole, 1 équivalent) dans 3 ml de diglyme a été préparée et la solution a été ajoutée lentement au milieu réactionnel. Le milieu réactionnel résultant a été chauffé à 140°C environ pendant 24h. Puis, il a été refroidi à 60°C environ et 7,18 mmoles de NaOH broyé ont été ajoutés. Le milieu réactionnel résultant a été laissé sous agitation pendant 2h environ.

Ensuite, 0,7 mmole de 3-chloro-2-chloro-prop-1-ène a été ajouté et le milieu réactionnel résultant a été maintenu sous agitation pendant 12h environ. Le solide a été filtré, puis dissous dans de l'acétonitrile. La solution obtenue a été filtrée afin d'éliminer les sels inorganiques. Le filtrat a été évaporé au rotavapor afin d'obtenir l'ionomère sous la forme d'un liquide visqueux. Celui-ci a été séché sous vide à 100°C pendant 48h (rendement en ionomère **I^{2'}** de 79% environ).

La masse molaire moyenne en nombre Mₙ du ionomère **I^{1'}** obtenu était de 7600 g/mol environ et sa masse molaire moyenne en masse M_{w} était de 13100 g/mol environ.

L'ionomère **I^{1'}** avait une valeur p de 5,5 environ et une valeur y de 22,7 environ.

L'ionomère **I^{2'}** a été caractérisé par RMN du fluor, du carbone et du proton :
¹H RMN : δ (ppm, acétone-d₆) = 6,92 (s, 2H, H₃) ; 6,74 (s, 1H, H₁) ; 5,16 (s, 2H, H₁₄) ; 4,26 (s, 4H, H₉) ; 4,00 (s, 4H, H₁₂) ; 3,84 (s, 4H, H₁₀) ; 3,62 (s, 90H, H₁₁, -O-(CH₂CH₂-O)-).
¹³C RMN : δ (ppm, acétone-d₆) = 162,1 (s, 1C, C₂) ; 145,5 (s, 1C, C₁₃) ; 132,5 (t, 1C, C₄, J = 24,9Hz) ; 107,3 (t, 2C, C₃, J = 5,9Hz) ; 107,1 (s, 1C, C₁) ; 113,9 (m, 1C, C₆) ; 119,4 (m, 1C, C₈) ; 122,1 (m, 1C, C₇) ; 116,7 (m, 1C, C₅) ; 73,0 (s, 4C, C₉) ; 71,8 (s, 45C, C₁₁, (CH₂CH₂-O)ₙ-) ; 71,1 (s, 4C, C₁₂) ; 69,9 (s, 4C, C₁₀).
¹⁹F RMN : δ (ppm, acétone-d₆) = -83,28 (s, 2F, F₆); -87,95 (s, 2F, F₇) ; -113,58 (s, 2F, F₅) ; -118,68 (s, 2F, F₈).

L'ionomère **I²** (sous forme lithiée) a été obtenu par échange de cation du ionomère **I^{2'}** selon le protocole d'ultrafiltration suivant :
L'ionomère **I^{2'}** a été dissous dans une solution aqueuse à 1M de LiCl, puis filtré sous pression avec une membrane d'ultrafiltration en cellulose ayant un seuil de coupure de 1000 g/mol environ telle que définie dans l'exemple 7. Le NaCl formé était aussi soluble dans l'eau mais passait à travers la membrane. Lorsque la solution est devenue visqueuse, plusieurs solutions aqueuses à 1M de LiCI ont été ajoutées afin de saturer le milieu en ions lithium pour mieux favoriser l'échange entre les cations. Un lavage à l'eau afin d'éliminer le maximum de NaCl formé a été effectué plusieurs fois. Cet opération a été effectuée pendant au moins 24h puis la solution résultante a été lyophilisée. Le résidu obtenu a été dissous dans l'acétonitrile et filtré avec un papier filtre puis avec des microfiltres de 0,2 µm environ afin d'éliminer l'excès de LiCI et le NaCl restant. Les solvants ont été évaporés sous pression réduite (10⁻² bars) et l'ionomère **I²** obtenu a été séché sous vide à 60°C environ 24h (rendement en ionomère **I²** de 79% environ).

La masse molaire moyenne en nombre Mₙ du ionomère **I²** était de 22100 g/mol environ et sa masse molaire moyenne en masse M_{w} était de 38100 g/mol environ.

L'ionomère **I²** avait une valeur y de 22 environ, une valeur p de 5,5 environ, et une valeur q₃ de 3 environ.

Les ionomères **I^{2'}** et **I²** ont ensuite été réticulés selon le protocole suivant :
1 g d'ionomère **I^{2'}** ou **I²** a été dissous dans 10 ml d'acétonitrile contenant 0,02 g d'irgacure®2959. La solution résultante a été laissée sous agitation à l'abri de la lumière environ 2h. Ensuite, la solution a été dégazée et versée dans une boite de Petri. Le solvant a été évaporé à température ambiante. Puis, l'ionomère a été réticulé par deux irradiations durant 30 secondes chacune, avec une minute d'attente entre les deux irradiations, à l'aide d'une lampe UV. L'ionomère réticulé **I^{3'}** ou **I³** obtenu a été séché sous vide à 70°C environ pendant au moins 72h et stocké dans une boite à gants.

### Exemple 9 : préparation d'un ionomère à conduction cationique I^{4'} conforme au deuxième objet de l'invention

Le schéma de synthèse pour conduire au ionomère **I^{4'}** est le suivant :

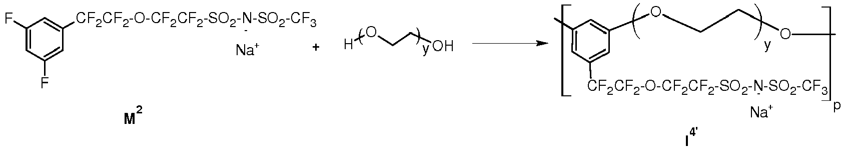

Dans un ballon tricol sous atmosphère d'argon, 0,075 g de NaH (2,45 mmoles, 2,3 équivalents) ont été placés. Dans un autre ballon, une solution de 1,285 g de polyéthylène glycol de masse molaire 1000 g/mol (PEG1000, n = 22,7) (1,285 mmoles, 1 équivalent) dans 4 ml de diglyme a été préparée. Puis, 3 ml de diglyme ont été ajoutés dans le ballon contenant le NaH et la solution résultante a été ajoutée lentement à la solution contenant le PEG1000. Le milieu réactionnel résultant a été chauffé à 65°C environ pendant 3h30 sous atmosphère d'argon.

Dans un autre ballon, une solution contenant 0,725 g du monomère **M²** tel que préparé dans l'exemple 2 (1,287 mmole, 1 équivalent) dans 3 ml de diglyme a été préparée et la solution a été ajoutée lentement au milieu réactionnel. Le milieu réactionnel résultant a été chauffé à 140°C environ pendant 24h. Puis, il a été refroidi à température ambiante et précipité dans le pentane pour conduire à un solide contenant l'ionomère **I^{4'}**. Le solide a été filtré puis dissous dans de l'acétonitrile. La solution obtenue a été filtrée afin d'éliminer les sels inorganiques. Le filtrat a été évaporé au rotavapor afin d'obtenir l'ionomère **I^{4'}** sous la forme d'un liquide visqueux. Celui-ci a été séché sous vide à 100°C pendant 48h (rendement en ionomère **I^{4'}** de 62% environ).

L'ionomère **I^{4'}** a été caractérisé par RMN du fluor et du proton :
¹H RMN : δ (ppm, acétone-d₆) = 6,83 (s, 2H, H₃) ; 6,79 (s, 1H, H₁) ; 4,21 (t, 4H, H₁₀,) ; 3,83 (t, 4H, H₁₁) ; 3,60 (s, 90H, H₁₂, -O-(CH₂CH₂-O)-).
¹⁹F RMN : δ (ppm, acétone-d₆) = -79,65 (s, 3F, F₉) ; -82,05 (s, 2F, F₆) ; -87,74 (s, 2F, F₇) ; -113,85 (s, 2F, F₅) ; -117,20 (s, 2F, F₈).

### Exemple 10 : préparation d'ionomères à conduction cationique I^{5'} et I^{6'} conformes au deuxième objet de l'invention

Le schéma de synthèse pour conduire aux ionomères **I^{5'}** et **I^{6'}** est le suivant :

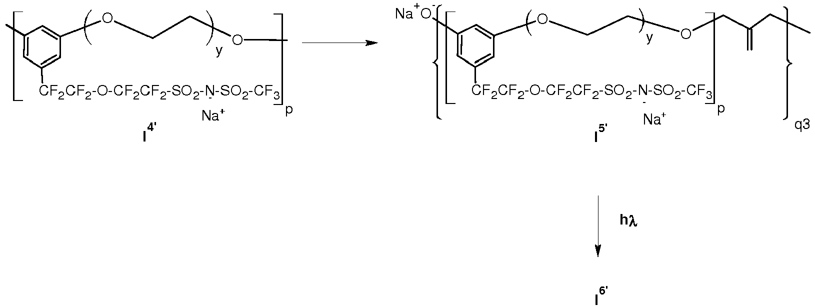

Dans un ballon tricol sous atmosphère d'argon, 0,075 g de NaH (2,45 mmoles, 2,3 équivalents) ont été placés. Dans un autre ballon, une solution de 1,285 g de polyéthylène glycol de masse molaire 1000 g/mol (PEG1000, n = 22,7) (1,285 mmoles, 1 équivalent) dans 4 ml de diglyme a été préparée. Puis, 3 ml de diglyme ont été ajoutés dans le ballon contenant le NaH et la solution résultante a été ajoutée lentement à la solution contenant le PEG1000. Le milieu réactionnel résultant a été chauffé à 65°C environ pendant 3h30 sous atmosphère d'argon.

Dans un autre ballon, une solution contenant 0,725 g du monomère **M²** tel que préparé dans l'exemple 2 (1,287 mmoles, 1 équivalent) dans 3 ml de diglyme a été préparée et la solution a été ajoutée lentement au milieu réactionnel. Le milieu réactionnel résultant a été chauffé à 140°C environ pendant 24h.

Le milieu réactionnel a été refroidi à 60°C environ et 1 mmoles de NaOH broyé ont été ajoutés. Le milieu réactionnel résultant a été laissé sous agitation pendant 2h environ. Ensuite, 0,213 mmole de 3-chloro-2-chloro-prop-1-ène a été ajouté et le milieu réactionnel résultant a été maintenu sous agitation pendant 12h environ. Le solide a été filtré, puis dissous dans de l'acétonitrile. La solution obtenue a été filtrée afin d'éliminer les sels inorganiques. Le filtrat a été évaporé au rotavapor afin d'obtenir l'ionomère sous la forme d'un liquide visqueux. Celui-ci a été séché sous vide à 100°C pendant 48h (rendement en ionomère **I^{5'}** de 88% environ).

L'ionomère **I^{5'}** a été caractérisé par RMN du fluor et du proton :
¹H RMN : δ (ppm, acétone-d₆) = 6,82 (s, 2H, H₃) ; 6,76 (s, 1H, H₁) ; 5,16 (s, 2H, H₁₅) ; 4,23 (s, 4H, H₁₀) ; 4,00 (s, 4H, H₁₃) ; 3,83 (s, 4H, H₁₁) ; 3,60 (s, 90H, H₁₂, -O-(CH₂CH₂-O)-).
¹⁹F RMN : δ (ppm, acétone-d₆) = -79,77 (s, 3F, F₉) ; -82,14 (s, 2F, F₆) ; -87,78 (s, 2F, F₇) ; -113,93 (s, 2F, F₅) ; -117,30 (s, 2F, F₈).

L'ionomère **I^{5'}** a ensuite été réticulé selon le protocole de l'exemple 8.

### Exemple 11 : préparation de compositions électrolytes conformes au troisième objet de l'invention

Plusieurs compositions électrolytes ont été analysées par calorimétrie différentielle à balayage :
- une composition électrolyte **C^{3'}** constituée du ionomère **I^{3'}** de l'exemple 8,
- une composition électrolyte **C³** constituée du ionomère **I³** de l'exemple 8, et
- une composition électrolyte **C^{3'}-A** constituée de 90% en masse du ionomère **I^{3'}** de l'exemple 8 et de 10% en masse de nanofibres de cellulose fonctionnalisées par des groupes sulfonates de sodium -SO₃⁻Na⁺ (NCC).

Les NCC ont été fournies par la société FP Innovation, Canada. Elles sont issues du bois dur (« hardwood »).

La composition électrolyte **C^{3'}-A** a été préparée de la façon suivante :

Dans un récipient, 4 g de NCC ont été dispersées dans 100 ml d'eau distillée. La dispersion résultante a été soumise à 4 cycles de 5 minutes d'homogénéisation à l'aide d'un disperseur, en imposant une vitesse de 13 000 tours/minute, notamment avec un appareil vendu sous la dénomination commerciale IKA® Ultra-Turrax. Puis, la dispersion résultante a été soumise à des ultrasons à l'aide d'une sonde à ultrason vendu sous la dénomination commercial VCX130 par la société Sonics & Materials, Inc., plongée directement dans la dispersion. La durée du cycle d'ultrasons était de 15 minutes environ, avec une impulsion d'intensité étant de 6 sur 9. Le récipient contenant la dispersion était placé dans un bain froid pour éviter un échauffement de ladite dispersion. Typiquement, pour atteindre une dispersion homogène, une dizaine de cycles d'ultrasons étaient nécessaires pour les NCC utilisées.

0,9 g d'ionomère **I^{2'}** a été dissous dans 10 ml d'eau contenant 0,02 g d'irgacure®2959. A la solution résultante, 2,5 ml (i.e. 0,1 g de NCC) de la dispersion de NCC dans l'eau telle que préparée précédemment ont été ajoutés. La dispersion résultante a été laissée sous agitation à l'abri de la lumière environ 2h. Ensuite, la dispersion a été dégazée et versée dans une boite de Petri. Le solvant a été évaporé à température ambiante. Puis, l'ionomère a été réticulé par deux irradiations durant 30 secondes chacune, avec une minute d'attente entre les deux irradiations à l'aide d'une lampe UV. La composition électrolyte **C^{3'}-A** obtenue a été séchée sous vide à 70°C environ pendant au moins 72h et stockée dans une boite à gants.

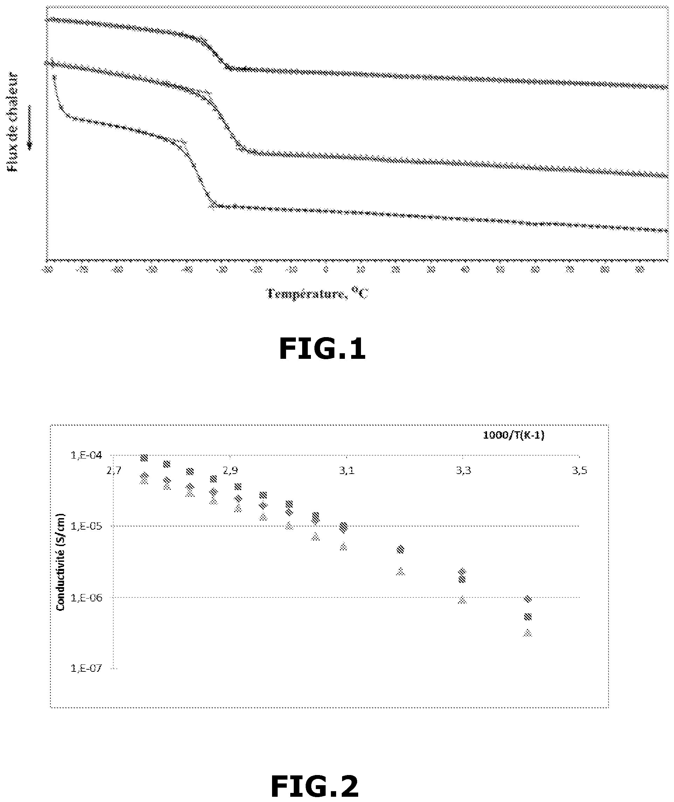

Les analyses de DSC sont reportées sur la figure 1 (flux de chaleur en fonction de la température) et ont montré que les ionomères des compositions **C³** (courbe avec les croix), **C^{3'}** (courbe avec les triangles) et **C^{3'}-A** (courbe avec les ronds) étaient entièrement amorphes puisqu'aucun pic de fusion n'a été observé. Par ailleurs, les températures de transition vitreuse T_{g} desdits ionomères étaient du même ordre de grandeur que celles obtenues avec un électrolyte de l'art antérieur POE/LiTFSI (i.e. de l'ordre de -34 à -40°C).

Des tests de conductivité ionique entre 20 et 90°C sont reportés sur la figure 2 et ont montré qu'à 50°C, la conductivité de la composition **C³** (losanges pleins) et de la composition **C^{3'}** (carrés pleins) était de l'ordre de 1 x 10⁻⁵ S.cm⁻¹. Celle de la composition **C^{3'}-A** (triangles pleins) était légèrement inférieure mais la stabilité mécanique de ladite composition **C^{3'}-A** était améliorée de par la présence des nanofibres de cellulose fonctionnalisées NCC.

Les NCC permettent également de diminuer la croissance dendritique.

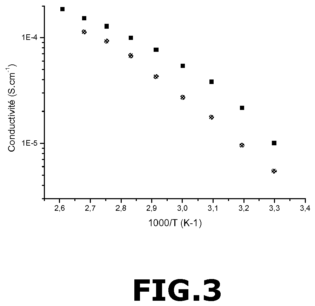

Des tests de conductivité ionique entre 20 et 90°C ont également été effectués sur des compositions électrolytes **C^{1'}** et **C^{4'}** respectivement constituées du ionomère **I^{1'}** et du ionomère **I^{4'}** et sont reportés sur la figure 3.

Ils ont montré qu'à 50°C, la conductivité de **C^{4'}** (carrés pleins) est de 4 x 10⁻⁵ S.cm⁻¹ et celle de **C^{1'}** (ronds pleins) est de 1,8 x 10⁻⁵ S.cm⁻¹.

Par ailleurs, les nombres de transport ionique des ionomères réticulés **I³, I^{3'}, I⁶** (i.e. de formule similaire à celle de **I^{6'}** mais sous la forme lithiée au lieu de la forme sodée) et **I^{6'}** étaient de 1.

## Revendications

1. Procédé de préparation d'un ionomère comprenant au moins des unités récurrentes UP répondant à la formule (II) suivante : 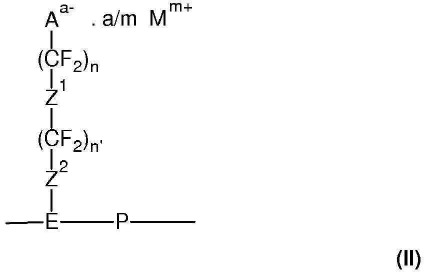 dans laquelle
- M est un cation de métal alcalin, un cation de métal alcalino-terreux, un cation de métal de transition, un cation de métal pauvre, un ammonium, un sulfonium ou un phosphonium de valence m, avec 1 ≤ m ≤ 3, m étant un nombre entier,
- A^{a-} est un anion choisi parmi un anion sulfonate, un anion sulfonimidure de formule -SO₂-N⁻-SO₂R et un carbanion de formule -SO₂-C⁻ R'R", avec 1 ≤ a ≤ 3, a étant un nombre entier,
* avec R représentant un atome de fluor ; un groupe alkyle éventuellement fluoré ou perfluoré, ayant de 1 à 10 atomes de carbone ; un groupe alkoxy éventuellement fluoré ou perfluoré, ayant de 1 à 10 atomes de carbone ; un groupe phénoxy éventuellement substitué par un groupe électroattracteur X² ; un groupe éther de dialkyle éventuellement fluoré ou perfluoré, ayant de 1 à 10 atomes de carbone ; un groupe thiocyanate ; un groupe phényle éventuellement substitué ; un groupe nitrile ; un groupe amino de formule -NR¹R², dans laquelle R¹ et R² sont choisis, indépendamment l'un de l'autre, parmi les groupes suivants : un groupe alkyle éventuellement fluoré ou perfluoré, ayant de 1 à 5 atomes de carbone, un groupe alkyle ayant de 1 à 5 atomes de carbone et portant un groupe électroattracteur X³, un groupe éther de dialkyle éventuellement fluoré ou perfluoré, ayant de 1 à 5 atomes de carbone, et un groupe électroattracteur X⁴ ; un groupe -NR³ étant choisi parmi un hétérocycle saturé ayant de 3 à 6 atomes de carbone et un hétérocycle insaturé ayant de 4 à 6 atomes de carbone ; un groupe amide de formule -NH-CO-R⁴ ou -N(CH₃)-CO-R⁴, dans laquelle R⁴ est un groupe alkyle ayant de 1 à 3 atomes de carbone ; un groupe sulfonamide de formule -NH-SO₂-R⁵ ou -N(CH₃)-SO₂-R⁵, dans laquelle R⁵ est un groupe alkyle ayant de 1 à 3 atomes de carbone ; un groupe uréthane de formule -NH-CO₂- R⁶ ou -N(CH₃)-CO₂- R⁶, dans laquelle R⁶ est un groupe alkyle ayant de 1 à 3 atomes de carbone ; un groupe cyanamide de formule -NH-CN ou -N(R⁷)-CN, dans laquelle R⁷ est un groupe alkyle ayant 1 à 3 atomes de carbone ; un groupe dicyanamide -N(CN)₂ ; un groupe tricyanométhyle -C(CN)₃ ; ou un groupe dicyanométhylène de formule -CH(CN)₂ ou -CR⁸(CN)₂, dans laquelle R⁸ est un groupe alkyle ayant 1 à 3 atomes de carbone,
* avec R' et R" étant choisis, indépendamment l'un de l'autre, parmi les groupes monovalents suivants : un atome de fluor ; un groupe thiocyanate ; un groupe nitrile ; un groupe nitro ; un groupe nitroso de formule de formule R⁹NO-, dans laquelle R⁹ est un groupe alkyle ayant de 1 à 3 atomes de carbone ; un groupe carbonyle de formule -COR¹⁰ dans laquelle R¹⁰ est un groupe alkyle perfluoré ayant de 1 à 5 atomes de carbone ; un groupe sulfoxyde de formule -SOR¹¹ dans laquelle R¹¹ est un groupe alkyle éventuellement fluoré ou perfluoré, ayant de 1 à 5 atomes de carbone ou un groupe éther de dialkyle éventuellement fluoré ou perfluoré, ayant de 1 à 5 atomes de carbone ; un groupe sulfonyle de formule -SO₂R¹² dans laquelle R¹² est un atome de fluor, un groupe thiocyanate, un groupe nitrile, un groupe alkoxy éventuellement fluoré ou perfluoré, ayant de 1 à 5 atomes de carbone, un groupe alkyle éventuellement fluoré ou perfluoré, ayant de 1 à 5 atomes de carbone ou un groupe éther de dialkyle éventuellement fluoré ou perfluoré, ayant de 1 à 5 atomes de carbone ; un groupe ester carboxylique de formule -COOR¹³, dans laquelle R¹³ est un groupe alkyle ayant de 1 à 5 atomes de carbone ; un groupe amide de formule -CONHR¹⁴ dans laquelle R¹⁴ est un groupe alkyle ayant de 1 à 5 atomes de carbone ; un groupe amide de formule -CONR¹⁴R¹⁵ dans laquelle R¹⁴ et R¹⁵ sont choisis, indépendamment l'un de l'autre, et R¹⁵ est un groupe alkyle ayant de 1 à 5 atomes de carbone ; un groupe phényle éventuellement substitué ; ou un groupe phénoxy éventuellement substitué, ou
* avec R' et R" étant des groupes divalents de telle sorte que le radical carbanion -CR'R" résultant forme un cycle aromatique comprenant de 5 à 6 atomes de carbone et optionnellement un ou plusieurs des hétéroatomes O ou N, ledit cycle aromatique étant éventuellement substitué par un ou plusieurs groupes nitriles,
- 1 ≤ n ≤ 4, n étant un nombre entier,
- 0 ≤ n' ≤ 2, n' étant un nombre entier,
- Z¹ est choisi parmi une liaison simple, un atome d'oxygène, un atome de soufre, un groupe -S=O, un groupe -S(=O)₂ et un groupe phényle éventuellement substitué en position ortho par rapport à l'une des fonctions (CF₂)ₙ ou (CF₂)n',
- Z² est choisi parmi une liaison simple, un atome d'oxygène, un atome de soufre, un groupe -S=O, un groupe -S(=O)₂ et un groupe -C=O, étant entendu que lorsque n' = 0, Z² est une liaison simple,
- E est un groupe phényle, un groupe thiényle, un groupe pyridyle, un groupe furanyle, un groupe pyrazolyle, un groupe benzophénone, un groupe diphényl sulfure ou un groupe diphényl sulfone, et
- P est une chaîne polymère d'oxyde d'alkylène, répondant à l'une des formules suivantes :
* -[O-(CH₂)ₓ]_{y}-O-, dans laquelle 2 ≤ x ≤ 4 et 1 ≤ y ≤ 50,
* -[O-CH₂-CHR¹⁸]_{y}-O-, dans laquelle R¹⁸ est un groupe alkyle ayant de 1 à 8 atomes de carbone, ou un groupe alkoxy ayant de 1 à 8 atomes de carbone, et 1 ≤ y ≤ 50, ou
* -[O-(CH₂)ₓᵢ-O-(CH₂-CHR¹⁹)ₓᵢᵢ]_{y}-O-, dans laquelle 1 ≤ xi ≤ 4 ; R¹⁹ est un atome d'hydrogène ou un groupe alkyle ayant de 1 à 8 atomes de carbone et 1 ≤ y ≤ 50,
ledit procédé étant **caractérisé en ce qu'**il comprend au moins une étape a) de polycondensation d'au moins un monomère ionique difluoré (I) avec au moins un polymère d'oxyde d'alkylène P¹ en milieu basique, ledit monomère ionique difluoré répondant à la formule (I) suivante : 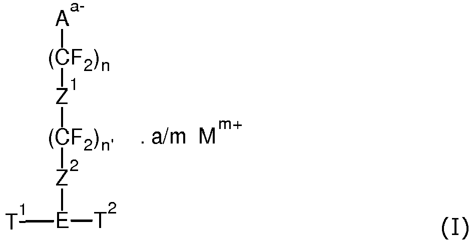
dans laquelle :
- A, n, n', Z¹, Z², E, m, a et M sont tels que définis ci-dessus, et
- T¹ et T² sont des atomes de fluor,
et ledit polymère d'oxyde d'alkylène P¹ utilisé dans l'étape a) est choisi parmi les polymères de formules suivantes :
* H-[O-(CH₂)ₓ]_{y}-OH, dans laquelle 2 ≤ x ≤ 4, 1 ≤ y ≤ 50,
* H-[O-CH₂-CHR¹⁸]_{y}-OH, dans laquelle R¹⁸ est un groupe alkyle ayant de 1 à 8 atomes de carbone ou un groupe alkoxy ayant de 1 à 8 atomes de carbone, et 1 ≤ y ≤ 50, et
* H-[O-(CH₂)ₓᵢ-O-(CH₂-CHR¹⁹)ₓᵢᵢ]_{y}-OH, dans laquelle 1 ≤ xi ≤ 4 ; 1 ≤ xii ≤ 2 ; R¹⁹ est un atome d'hydrogène ou un groupe alkyle ayant de 1 à 8 atomes de carbone ; 1 ≤ y ≤ 50.

2. Procédé selon la revendication 1, **caractérisé en ce qu'**il comprend en outre après l'étape a), au moins une étape b) de mise en contact du ionomère avec un composé G comprenant au moins deux fonctions F¹ aptes à se polycondenser avec ledit ionomère et éventuellement au moins une fonction F² post-polymérisable.

3. Procédé selon la revendication 2, **caractérisé en ce que** le composé G comprend une fonction post-polymérisable F² et le procédé comprend en outre une étape c) de post-polymérisation du ionomère obtenu à l'issue de l'étape b).

4. Procédé selon la revendication 1, **caractérisé en ce qu'**il comprend en outre après l'étape a), au moins une étape d) de mise en contact du ionomère avec un composé H comprenant une fonction F¹ apte à se condenser avec ledit ionomère et éventuellement au moins une fonction F² post-polymérisable.

5. Procédé selon la revendication 1, **caractérisé en ce qu'**il comprend en outre après l'étape a), au moins une étape f) de mise en contact du ionomère avec un composé J apte à réagir avec ledit ionomère selon une polymérisation radicalaire ou ionique.

6. Procédé selon l'une quelconque des revendications précédentes, **caractérisé en ce que** le monomère ionique difluoré (I) est préparé selon un procédé comprenant au moins une étape i) de réaction d'un composé (I-a) avec un composé (I-b) selon le schéma réactionnel suivant : 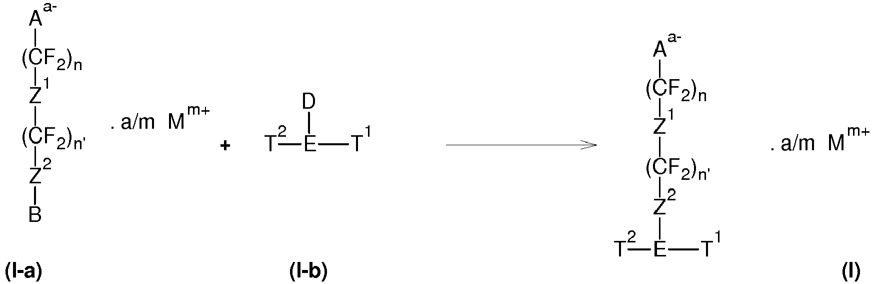 ou au moins une étape i') de réaction d'un composé (I'-a) avec un composé (I'-b) selon le schéma réactionnel suivant : 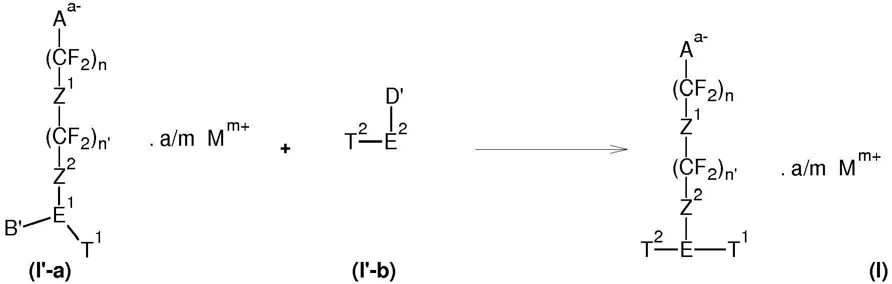 ou au moins une étape i") de réaction d'un composé (I"-a) avec un composé (I"-b) selon le schéma réactionnel suivant : 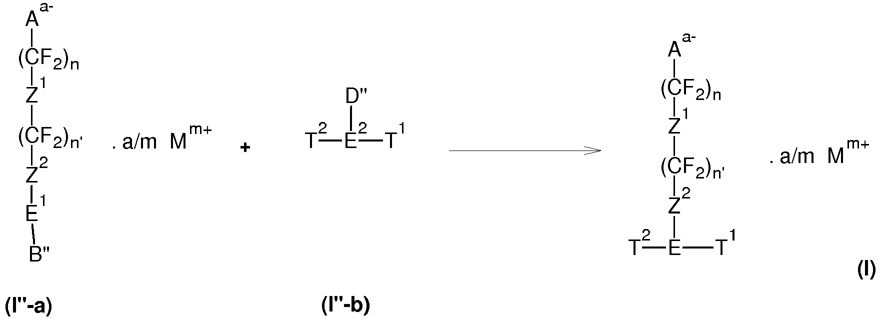
avec Z¹, Z², n, n', T¹, T², E, A, a, m et M étant tels que définis dans la revendication 1, et
les groupes B et D, les groupes B' et D' et les groupes B" et D" étant choisis de façon appropriée pour pouvoir réagir entre eux et E¹ et E² étant choisis de façon appropriée pour pouvoir former le groupe aromatique E.

7. Procédé selon l'une quelconque des revendications précédentes, **caractérisé en ce que** le monomère ionique difluoré (I) répondant à l'une quelconque des formules suivantes : 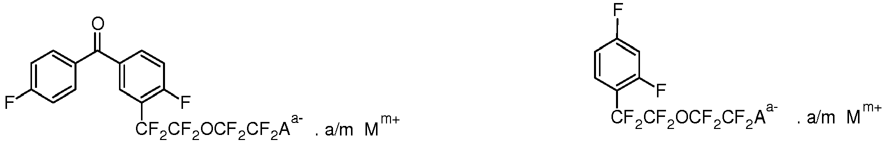 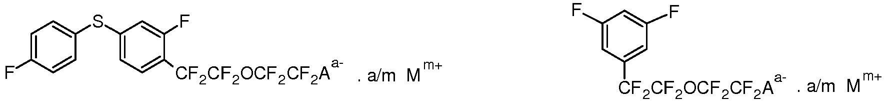 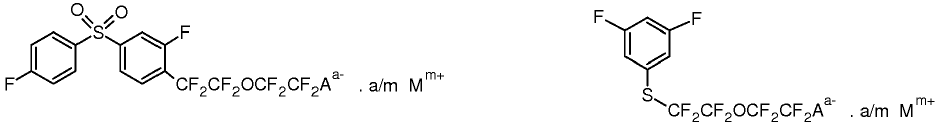 dans lesquelles M, A, a et m sont tels que définis dans la revendication 1.

8. Ionomère, **caractérisé en ce qu'**il est obtenu selon le procédé tel que défini à l'une quelconque des revendications 1 à 7 et qu'il comprend au moins des unités récurrentes UP répondant à la formule (II) suivante : 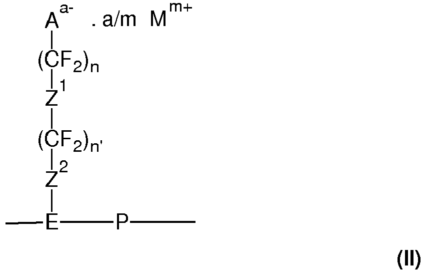 dans laquelle A, n, n', Z¹, Z², E, m, a et M sont tels que définis à l'une quelconque des revendications 1 à 7, et P est une chaîne polymère d'oxyde d'alkylène tel que défini dans la revendication 1.

9. Ionomère selon la revendication 8, **caractérisé en ce que** M est un cation de métal alcalin ou un cation de métal alcalino-terreux.

10. Ionomère selon l'une quelconque des revendications 8 ou 9, **caractérisé en ce qu'**une ou plusieurs des conditions suivantes s'appliquent :
* n = n' = 2,
* Z¹ est un atome d'oxygène,
* Z² est un atome de soufre ou une liaison simple.

11. Composition électrolyte, **caractérisée en ce qu'**elle comprend au moins un ionomère obtenu selon le procédé tel que défini à l'une quelconque des revendications 1 à 7 ou tel que défini à l'une quelconque des revendications 8 à 10.

12. Composition électrolyte selon la revendication 11, **caractérisée en ce qu'**elle comprend en outre un ou plusieurs solvants organiques.

13. Composition électrolyte selon la revendication 11 ou 12, **caractérisée en ce qu'**elle comprend en outre un ou plusieurs additifs choisis parmi les charges minérales, les sels de métal alcalin, les charges organiques, les agents complexants, les agents ignifugeants et un de leurs mélanges.

14. Composition électrolyte selon la revendication 11, **caractérisée en ce qu'**elle est uniquement constituée dudit ionomère.

15. Utilisation d'un ionomère obtenu selon le procédé tel que défini à l'une quelconque des revendications 1 à 7 ou tel que défini à l'une quelconque des revendications 8 à 10, pour la fabrication d'un électrolyte pour un dispositif électrochimique.

16. Utilisation d'un ionomère obtenu selon le procédé tel que défini à l'une quelconque des revendications 1 à 7 ou tel que défini à l'une quelconque des revendications 8 à 10, comme liquide ionique.

17. Utilisation d'un ionomère obtenu selon le procédé tel que défini à l'une quelconque des revendications 1 à 7 ou tel que défini à l'une quelconque des revendications 8 à 10, comme constituant d'une électrode composite.

18. Dispositif électrochimique comprenant au moins une électrode négative et au moins une électrode positive séparées par une composition électrolyte, **caractérisé en ce que** la composition électrolyte est telle que définie à l'une quelconque des revendications 11 à 14.

## Patentansprüche

1. Verfahren zur Herstellung eines Ionomers, mindestens wiederkehrende Einheiten UP umfassend, die der folgenden Formel (II) folgen: 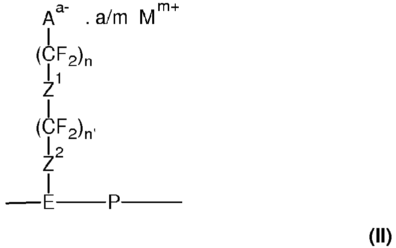 in der
- M ein Alkalimetall-Kation, ein Erdalkalimetall-Kation, ein Übergangsmetall-Kation, ein Kation eines p-Block-Metalls, ein Ammonium, ein Sulfonium oder ein Phosphonium der Wertigkeit m mit 1 ≤ m ≤ 3 ist, wobei m eine ganze Zahl ist,
- A^{a-} ein Anion ist, das ausgewählt ist aus einem Sulfonat-Anion, Sulfonimid-Anion der Formel -SO₂-N⁻-SO₂R und einem Carbanion der Formel -SO₂-C⁻R'R" ist, mit 1 ≤ a ≤ 3, wobei a eine ganze Zahl ist,
* mit R darstellend ein Fluoratom; eine Alkylgruppe, gegebenenfalls fluoriert oder perfluoriert, die 1 bis 10 Kohlenstoffatome aufweist; eine Alkoxygruppe, gegebenenfalls fluoriert oder perfluoriert, die 1 bis 10 Kohlenstoffatome aufweist, eine Phenoxygruppe, gegebenenfalls substituiert durch eine elektronenziehende Gruppe X²; eine Gruppe des Dialkylethers, gegebenenfalls fluoriert oder perfluoriert, die 1 bis 10 Kohlenstoffatome aufweist; eine Thiocyanatgruppe; eine Phenylgruppe, gegebenenfalls substituiert; eine Nitrilgruppe; eine Aminogruppe der Formel -NR¹R², bei der R¹ und R² unabhängig voneinander ausgewählt sind aus den folgenden Gruppen: einer Alkylgruppe, gegebenenfalls fluoriert oder perfluoriert, die 1 bis 5 Kohlenstoffatome aufweist, einer Alkylgruppe, die 1 bis 5 Kohlenstoffatome aufweist und eine elektronenziehende Gruppe X³ trägt, einer Gruppe des Dialkylethers, gegebenenfalls fluoriert oder perfluoriert, die 1 bis 5 Kohlenstoffatome und eine elektronenziehende Gruppe X⁴ aufweist; eine Gruppe -NR³, ausgewählt aus einem gesättigten Heterozyklus mit 3 bis 6 Kohlenstoffatomen und einem ungesättigten Heterozyklus mit 4 bis 6 Kohlenstoffatomen; einer Amidgruppe der Formel -NH-CO-R⁴ oder -N(CH₃)-CO-R⁴, in der R⁴ eine Alkylgruppe mit 1 bis 3 Kohlenstoffatomen ist; eine Sulfonamidgruppe der Formel -NH-SO₂-R⁵ oder - N(CH₃)-SO₂-R⁵, in der R⁵ eine Alkylgruppe mit 1 bis 3 Kohlenstoffatomen ist; eine Urethangruppe der Formel -NH-CO₂-R⁶ oder - N(CH₃)-SO₂-R⁶, in der R⁶ eine Alkylgruppe mit 1 bis 3 Kohlenstoffatomen ist; eine Cyanamidgruppe der Formel -NH-CN oder -N(R₇)-CN, in der R⁷ eine Alkylgruppe mit 1 bis 3 Kohlenstoffatomen ist; eine Dicyanamidgruppe -N(CN)₂; eine Tricyanmethylgruppe - C(CN)₃; oder eine Dicyanmethlengruppe der Formel -CH(CN)₂ oder -CR⁸(CN)₂, in der R⁸ eine Alkylgruppe mit 1 bis 3 Kohlenstoffatomen ist,
* mit R' und R" unabhängig voneinander ausgewählt sind aus den folgenden monovalenten Gruppen: einem Fluoratom; einer Thiocyanatgruppe; einer Nitrilgruppe; einer Nitrogruppe; einer Nitrosogruppe der Formel R⁹NO-, in der R⁹ eine Alkylgruppe mit 1 bis 3 Kohlenstoffatomen ist; einer Carbonylgruppe der Formel -COR¹⁰, in der R¹⁰ eine perfluorierte Alkylgruppe mit 1 bis 5 Kohlenstoffatomen ist; einer Sulfoxidgruppe der Formel -SOR¹¹, in der R¹¹ eine Alkylgruppe, gegebenenfalls fluoriert oder perfluoriert, mit 1 bis 5 Kohlenstoffatomen, oder eine Gruppe des Dialkylethers, gegebenenfalls fluoriert oder perfluoriert, mit 1 bis 5 Kohlenstoffatomen, ist; einer Sulfonylgruppe der Formel -SO₂R¹², in der R¹² ein Fluoratom, eine Thiocyanatgruppe, eine Nitrilgruppe, eine Alkoxygruppe, gegebenenfalls fluoriert oder perfluoriert, mit 1 bis 5 Kohlenstoffatomen, eine Alkylgruppe, gegebenenfalls fluoriert oder perfluoriert, mit 1 bis 5 Kohlenstoffatomen, oder eine Gruppe des Dialkylethers, gegebenenfalls fluoriert oder perfluoriert, mit 1 bis 5 Kohlenstoffatomen ist; einer Carboxylestergruppe der Formel - COOR¹³, in der R¹³ eine Alkylgruppe mit 1 bis 5 Kohlenstoffatomen ist; einer Amidgruppe der Formel CONHR¹⁴, in der R¹⁴ eine Alkylgruppe mit 1 bis 5 Kohlenstoffatomen ist; einer Amidgruppe der Formel -CONR¹⁴R¹⁵, in der R¹⁴ und R¹⁵ unabhängig voneinander ausgewählt sind aus einer Alkylgruppe mit 1 bis 5 Kohlenstoffatomen; einer Phenylgruppe, gegebenenfalls substituiert; oder einer Phenoxygruppe, gegebenenfalls substituiert, oder
* mit R' und R" als divalente Gruppen derart, dass das resultierende Carbanionradikal -C⁻R'R" einen aromatischen Ring bildet, der 5 bis 6 Kohlenstoffatome und optional ein oder mehrere Heteroatome O oder N enthält, wobei der aromatische Ring gegebenenfalls durch ein oder mehrere Nitrilgruppen substituiert ist,
- 1 ≤ n ≤ 4, wobei n eine ganze Zahl ist,
- 0 ≤ n' ≤ 2, wobei n' eine ganze Zahl ist,
- Z¹ ausgewählt ist aus einer einfachen Bindung, einem Sauerstoffatom, einem Schwefelatom, einer Gruppe -S=O, einer Gruppe - S(=O)₂ und einer Phenylgruppe, gegebenenfalls an der ortho-Position substituiert in Bezug auf eine der Funktionen (CF₂)ₙ oder (CF₂)n',
- Z² ausgewählt ist aus einer einfachen Bindung, einem Sauerstoffatom, einem Schwefelatom, einer Gruppe -S=O, einer Gruppe -S(=O)₂ und einer Gruppe -C=O, vorausgesetzt, dass, wenn n' = 0 ist, Z² eine einfache Bindung ist,
- E eine Phenylgruppe, eine Thienylgruppe, eine Pyridylgruppe, eine Furanylgruppe, eine Pyrazolylgruppe, eine Benzophenongruppe, eine Diphenylsulfidgruppe oder eine Diphenylsulfongruppe ist und
- P eine Polymerkette des Alkylenoxids ist, die einer der folgenden Formeln folgt:
* -[O-(CH₂)ₓ]_{y}-O-, wobei 2 ≤ x ≤ 4 und 1 ≤ y ≤ 50 ist,
* -[O-CH₂-CHR¹⁸]_{y}-O-, wobei R¹⁸ eine Alkylgruppe mit 1 bis 8 Kohlenstoffatomen oder eine Alkoxygruppe mit 1 bis 8 Kohlenstoffatomen und 1 ≤ y ≤ 50 ist, oder
* -[O-(CH₂)ₓᵢ-O-(CH₂-CHR¹⁹)ₓᵢᵢ]_{y}-O-, wobei 1 ≤ xi ≤ 4 ist; R¹⁹ ein Wasserstoffatom oder eine Alkylgruppe mit 1 bis 8 Kohlenstoffatomen und 1 ≤ y ≤ 50 ist,
wobei das Verfahren **dadurch gekennzeichnet ist, dass** es mindestens einen Schritt a) der Polykondensation mindestens eines difluorierten ionischen Monomers (I) mit mindestens einem Alkylenoxidpolymer P¹ in einem basischen Medium umfasst, wobei das difluorierte ionische Monomer der folgenden Formel (I) folgt: 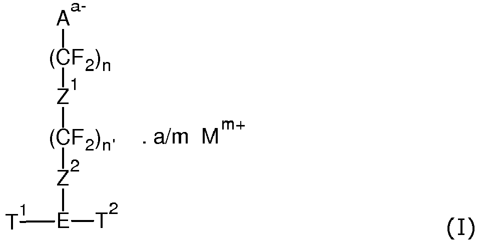
in der:
- A, n, n', Z¹, Z², E, m, a und M so sind, wie oben definiert, und
- T¹ und T² Fluoratome sind,
und das Alkylenoxidpolymer P¹, das in dem Schritt a) verwendet wird, ausgewählt ist aus den Polymeren der folgenden Formeln:
* H-[O-(CH₂)ₓ]_{y}-OH, wobei 2 ≤ x ≤ 4, 1 ≤ y ≤ 50 ist,
* H-[O-CH₂-CHR¹⁸]_{y}-OH, wobei R¹⁸ eine Alkylgruppe mit 1 bis 8 Kohlenstoffatomen und eine Alkoxygruppe mit 1 bis 8 Kohlenstoffatomen ist, und 1 ≤ y ≤ 50, und
* H-[O-(CH₂)ₓᵢ-O-(CH₂-CHR¹⁹)ₓᵢᵢ]_{y}-OH, wobei 1 ≤ xi ≤ 4; 1 ≤ xii ≤ 2; R¹⁹ ein Wasserstoffatom oder eine Alkylgruppe mit 1 bis 8 Kohlenstoffatomen ist; 1 ≤ y ≤ 50.

2. Verfahren nach Anspruch 1, **dadurch gekennzeichnet, dass** es außerdem nach dem Schritt a) mindestens einen Schritt b) des In-Kontakt-Bringens des lonomers mit einer Verbindung G umfasst, die mindestens zwei Funktionen F¹ enthält, die geeignet sind, mit dem lonomer zu polykondensieren, und gegebenenfalls mindestens eine nachpolymerisierbare Funktion F² enthält.

3. Verfahren nach Anspruch 2, **dadurch gekennzeichnet, dass** die Verbindung G eine nachpolymerisierbare Funktion F² enthält und das Verfahren außerdem einen Schritt c) der Nachpolymerisation des Ionomers, das am Ende des Schrittes b) erhalten wird, umfasst.

4. Verfahren nach Anspruch 1, **dadurch gekennzeichnet, dass** es außerdem nach dem Schritt a) mindestens einen Schritt d) des In-Kontakt-Bringens des lonomers mit einer Verbindung H umfasst, die eine Funktion F¹ enthält, die geeignet ist, mit dem lonomer zu kondensieren, und gegebenenfalls mindestens eine nachpolymerisierbare Funktion F² enthält.

5. Verfahren nach Anspruch 1, **dadurch gekennzeichnet, dass** es außerdem nach dem Schritt a) mindestens einen Schritt f) des In-Kontakt-Bringens des lonomers mit einer Verbindung J umfasst, die geeignet ist, mit dem lonomer gemäß einer radikalischen oder ionischen Polymerisation zu reagieren.

6. Verfahren nach einem beliebigen der vorhergehenden Ansprüche, **dadurch gekennzeichnet, dass** das difluorierte ionische Monomer (I) nach einem Verfahren hergestellt wird, das mindestens einen Schritt i) der Reaktion einer Verbindung (I-a) mit einer Verbindung (I-b) nach dem folgenden Reaktionsschema umfasst: 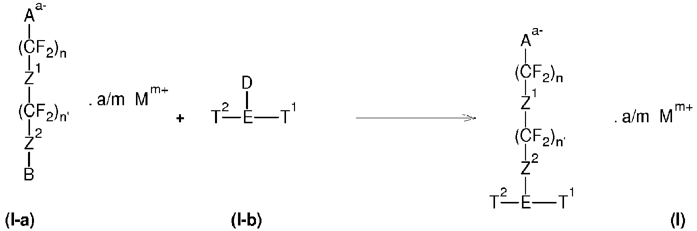 oder mindestens einen Schritt i') der Reaktion einer Verbindung (I'-a) mit einer Verbindung (I'-b) nach dem folgenden Reaktionsschema umfasst: 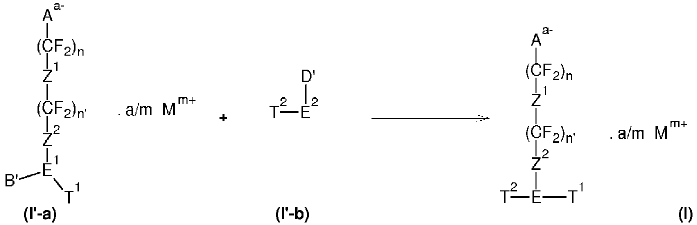 oder mindestens einen Schritt i") der Reaktion einer Verbindung (I"-a) mit einer Verbindung (I"-b) nach dem folgenden Reaktionsschema umfasst: 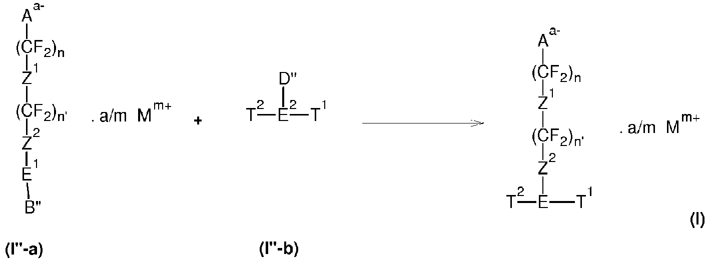
wobei Z¹, Z², n, n', T¹, T², E, A, a, m und M so sind, wie in Anspruch 1 definiert, und
die Gruppen B und D, die Gruppen B' und D' und die Gruppen B" und D" in geeigneter Weise ausgewählt sind, um untereinander reagieren zu können, und E¹ und E² in geeigneter Weise ausgewählt sind, um die aromatische Gruppe E zu bilden.

7. Verfahren nach einem beliebigen der vorhergehenden Ansprüche, **dadurch gekennzeichnet, dass** das difluorierte ionische Monomer (I) einer beliebigen der folgenden Formeln folgt: 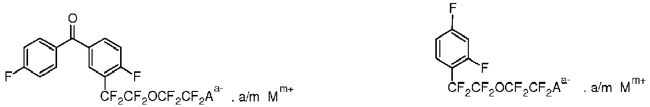 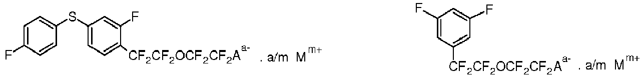 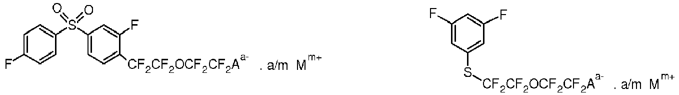 wobei M, A, a und m so sind, wie in Anspruch 1 definiert.

8. Ionomer, **dadurch gekennzeichnet, dass** es nach dem Verfahren nach einem beliebigen der Ansprüche 1 bis 7 erhalten wurde und dass es mindestens die wiederkehrenden Einheiten UP enthält, die der folgenden Formel (II) folgen: 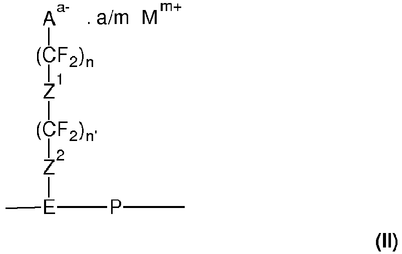 wobei A, n, n', Z¹, Z², E, m, a und M so sind, wie sie in einem beliebigen der Ansprüche 1 bis 7 definiert sind, und P eine Polymerkette des Alkylenoxids, wie im Anspruch 1 definiert, ist.

9. lonomer nach Anspruch 8, **dadurch gekennzeichnet, dass** M ein Alkalimetall-Kation oder ein Erdalkalimetall-Kation ist.

10. lonomer nach einem beliebigen der Ansprüche 8 bis 9, **dadurch gekennzeichnet, dass** eine oder mehrere der folgenden Bedingungen anwendbar sind:
* n = n' = 2,
* Z¹ ist ein Sauerstoffatom,
* Z² ist ein Schwefelatom oder eine einfache Bindung.

11. Elektrolytzusammensetzung, **dadurch gekennzeichnet, dass** sie mindestens ein lonomer enthält, das nach dem Verfahren, wie in einem beliebigen der Ansprüche 1 bis 7 definiert, erhalten wurde, oder wie in einem beliebigen der Ansprüche 8 bis 10 definiert ist.

12. Elektrolytzusammensetzung nach Anspruch 11, **dadurch gekennzeichnet, dass** sie außerdem ein oder mehrere organische Lösungsmittel enthält.

13. Elektrolytzusammensetzung nach Anspruch 11 oder 12, **dadurch gekennzeichnet, dass** sie außerdem ein oder mehrere Additive ausgewählt aus mineralischen Füllstoffen, Alkalimetallsalzen, organischen Füllstoffen, komplexbildenden Stoffen, Flammschutzmitteln und einer ihrer Mischungen, enthält.

14. Elektrolytzusammensetzung nach Anspruch 11, **dadurch gekennzeichnet, dass** sie alleinig aus dem lonomer gebildet ist.

15. Verwendung eines Ionomers,das nach dem Verfahren, wie es in einem beliebigen der Ansprüche 1 bis 7 definiert ist, erhalten wurde, oder wie es in einem beliebigen der Ansprüche 8 bis 10 definiert ist, zur Herstellung eines Elektrolyts für eine elektrochemische Vorrichtung.

16. Verwendung eines Ionomers, das nach dem Verfahren, wie es in einem beliebigen der Ansprüche 1 bis 7 definiert ist, erhalten wurde, oder wie es in einem beliebigen der Ansprüche 8 bis 10 definiert ist, als ionische Flüssigkeit.

17. Verwendung eines Ionomers, das nach dem Verfahren, wie es in einem beliebigen der Ansprüche 1 bis 7 definiert ist, erhalten wurde, oder wie es in einem beliebigen der Ansprüche 8 bis 10 definiert ist, als Bestandteil einer Kompositelektrode.

18. Elektrochemische Vorrichtung, mindestens eine negative Elektrode und eine positive Elektrode umfassend, die von einer Elektrolytzusammensetzung getrennt sind, **dadurch gekennzeichnet, dass** die Elektrolytzusammensetzung so ist, wie sie in einem beliebigen der Ansprüche 11 bis 14 definiert ist.

## Claims

1. Process for preparing an ionomer comprising at least repeating units RU corresponding to the following formula (II): 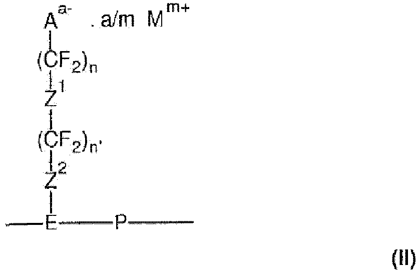 wherein
- M is an alkali metal cation, an alkaline earth metal cation, a transition metal cation, a poor metal cation, an ammonium, a sulfonium or a phosphonium of valence m, where 1 ≤ m ≤ 3, m being an integer,
- A^{a-} is an anion chosen from a sulfonate anion, a sulfonimide anion of formula -SO₂-N⁻-SO₂R and a carbanion of formula -SO₂C⁻R'R", where 1 ≤ a ≤ 3, a being an integer,
* where R represents a fluorine atom; an optionally fluorinated or perfluorinated alkyl group having from 1 to 10 carbon atoms; an optionally fluorinated or perfluorinated alkoxy group having from 1 to 10 carbon atoms; a phenoxy group optionally substituted by an electroattractive group X²; an optionally fluorinated or perfluorinated dialkyl ether group having from 1 to 10 carbon atoms; a thiocyanate group; an optionally substituted phenyl group; a nitrile group; an amino group of formula -NR¹R² wherein R¹ and R² are chosen, independently of one another, from the following groups: an optionally fluorinated or perfluorinated alkyl group having from 1 to 5 carbon atoms, an alkyl group having from 1 to 5 carbon atoms and carrying an electroattractive group X³, an optionally fluorinated or perfluorinated dialkyl ether group having from 1 to 5 carbon atoms, and an electroattractive group X⁴; a group -NR³ chosen from a saturated heterocycle having from 3 to 6 carbon atoms and an unsaturated heterocycle having from 4 to 6 carbon atoms; an amide group of formula -NH-CO-R⁴ or -N(CH₃)-CO-R⁴ wherein R⁴ is an alkyl group having from 1 to 3 carbon atoms; a sulfonamide group of formula -NH-SO₂-R⁵ or -N(CH₃)-SO₂-R⁵ wherein R⁵ is an alkyl group having from 1 to 3 carbon atoms; a urethane group of formula -NH-CO₂-R⁶ or -N(CH₃)-CO₂-R⁶ wherein R⁶ is an alkyl group having from 1 to 3 carbon atoms; a cyanamide group of formula -NH-CN or -N(R⁷)-CN wherein R⁷ is an alkyl group having from 1 to 3 carbon atoms; a dicyanamide group -N(CN)₂; a tricyanomethyl group -C(CN)₃; or a dicyanomethylene group of formula -CH(CN)₂ or -CR⁸(CN)₂ wherein R⁸ is an alkyl group having from 1 to 3 carbon atoms,
* where R' and R" are chosen, independently of one another, from the following monovalent groups: a fluorine atom; a thiocyanate group; a nitrile group; a nitro group; a nitroso group of formula R⁹NO- wherein R⁹ is an alkyl group having from 1 to 3 carbon atoms; a carbonyl group of formula -COR¹⁰ wherein R¹⁰ is a perfluorinated alkyl group having from 1 to 5 carbon atoms; a sulfoxide group of formula -SOR¹¹ wherein R¹¹ is an optionally fluorinated or perfluorinated alkyl group having from 1 to 5 carbon atoms or an optionally fluorinated or perfluorinated dialkyl ether group having from 1 to 5 carbon atoms; a sulfonyl group of formula -SO₂R¹² wherein R¹² is a fluorine atom, a thiocyanate group, a nitrile group, an optionally fluorinated or perfluorinated alkoxy group having from 1 to 5 carbon atoms, an optionally fluorinated or perfluorinated alkyl group having from 1 to 5 carbon atoms or an optionally fluorinated or perfluorinated dialkyl ether group having from 1 to 5 carbon atoms; a carboxylic ester group of formula -COOR¹³ wherein R¹³ is an alkyl group having from 1 to 5 carbon atoms; an amide group of formula -CONHR¹⁴ wherein R¹⁴ is an alkyl group having from 1 to 5 carbon atoms; an amide group of formula -CONR¹⁴R¹⁵ wherein R¹⁴ and R¹⁵ are chosen independently of one another and R¹⁵ is an alkyl group having from 1 to 5 carbon atoms; an optionally substituted phenyl group; or an optionally substituted phenoxy group, or
* where R' and R" are divalent groups such that the resulting carbanion radical -C⁻R'R" forms an aromatic ring comprising from 5 to 6 carbon atoms and optionally one or more hetero atoms O or N, said aromatic ring being optionally substituted by one or more nitrile groups,
- 1 ≤ n ≤ 4, n being an integer,
- 0 ≤ n' ≤ 2, n' being an integer,
- Z¹ is chosen from a single bond, an oxygen atom, a sulfur atom, a group -S=O, a group -S(=O)₂ and a phenyl group optionally substituted in the ortho position relative to one of the functional groups (CF₂)ₙ or (CF₂)_{n'},
- Z² is chosen from a single bond, an oxygen atom, a sulfur atom, a group -S=O, a group -S(=O)₂ and a group -C=O, it being understood that, when n' = 0, Z² is a single bond,
- E is a phenyl group, a thienyl group, a pyridyl group, a furanyl group, a pyrazolyl group, a benzophenone group, a diphenyl sulfide group or a diphenylsulfone group, and
- P is an alkylene oxide polymer chain corresponding to one of the following formulae:
* -[O-(CH₂)ₓ]_{y}-O- wherein 2 ≤ x ≤ 4 and 1 ≤ y ≤ 50,
* -[O-CH₂-CHR¹⁸]_{y}-O- wherein R¹⁸ is an alkyl group having from 1 to 8 carbon atoms or an alkoxy group having from 1 to 8 carbon atoms and 1 ≤ y ≤ 50, or
* -[O-(CH₂)ₓᵢ-O-(CH₂-CHR¹⁹)ₓᵢᵢ]_{y}-O- wherein 1 ≤ xi ≤ 4; R¹⁹ is a hydrogen atom or an alkyl group having from 1 to 8 carbon atoms and 1 ≤ y ≤ 50,
said process being **characterised in that** it comprises at least a step a) of polycondensation of at least one difluorinated ionic monomer (I) with at least one alkylene oxide polymer P¹ in a basic medium, said difluorinated ionic monomer corresponding to the following formula (I): 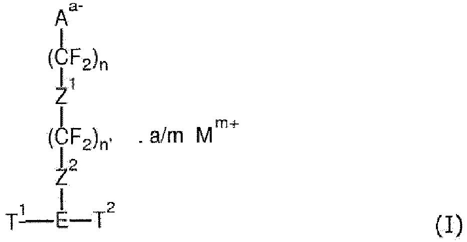
wherein:
- A, n, n', Z¹, Z², E, m, a and M are as defined hereinbefore, and
- T¹ and T² are fluorine atoms,
and said alkylene oxide polymer P¹ used in step a) is chosen from the polymers of the following formulae:
* H-[O-(CH₂)ₓ]_{y}-OH wherein 2 ≤ x ≤ 4, 1 ≤ y ≤ 50,
* H-[O-CH₂-CHR¹⁸]_{y}-OH wherein R¹⁸ is an alkyl group having from 1 to 8 carbon atoms or an alkoxy group having from 1 to 8 carbon atoms and 1 ≤ y ≤ 50, and
* H-[O-(CH₂)ₓᵢ-O-(CH₂-CHR¹⁹)ₓᵢᵢ]_{y}-OH wherein 1 ≤ xi ≤ 4; 1 ≤ xii ≤ 2; R¹⁹ is a hydrogen atom or an alkyl group having from 1 to 8 carbon atoms; 1 ≤ y ≤ 50.

2. Process according to claim 1, **characterised in that** it further comprises, after step a), at least a step b) of bringing the ionomer into contact with a compound G comprising at least two functional groups F¹ capable of polycondensing with said ionomer and optionally at least one post-polymerisable functional group F².

3. Process according to claim 2, **characterised in that** the compound G comprises a post-polymerisable functional group F² and the process further comprises a step c) of post-polymerisation of the ionomer obtained at the end of step b).

4. Process according to claim 1, **characterised in that** it further comprises, after step a), at least a step d) of bringing the ionomer into contact with a compound H comprising a functional group F¹ capable of condensing with said ionomer and optionally at least one post-polymerisable functional group F².

5. Process according to claim 1, **characterised in that** it further comprises, after step a), at least a step f) of bringing the ionomer into contact with a compound J capable of reacting with said ionomer according to a radical or ionic polymerisation.

6. Process according to any one of the preceding claims, **characterised in that** the difluorinated ionic monomer (I) is prepared by a process comprising at least a step i) of reacting a compound (I-a) with a compound (I-b) according to the following reaction scheme: 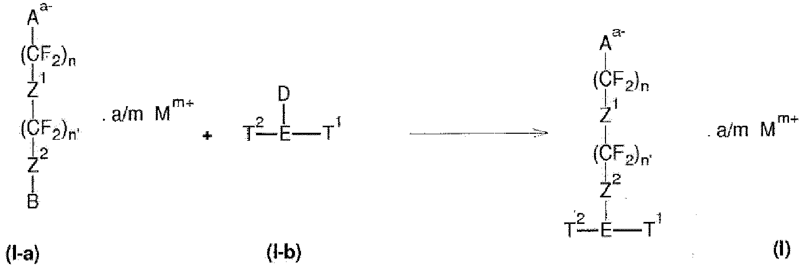 or at least a step i') of reacting a compound (I'-a) with a compound (I'-b) according to the following reaction scheme: 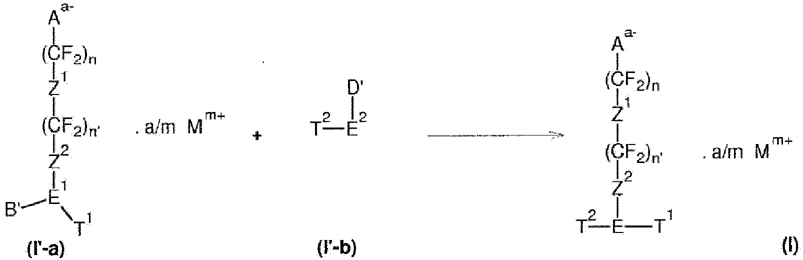 or at least a step i") of reacting a compound (I"-a) with a compound (I"-b) according to the following reaction scheme: 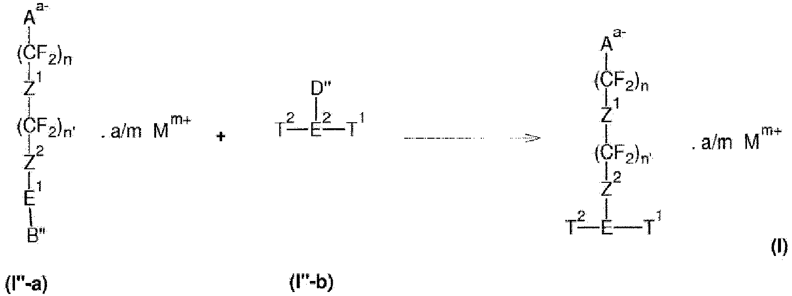
where Z¹, Z², n, n', T¹, T², E, A, a, m and M are as defined in claim 1, and
the groups B and D, the groups B' and D' and the groups B" and D" are chosen in an appropriate manner to be able to react with one another, and E¹ and E² are chosen in an appropriate manner to be able to form the aromatic group E.

7. Process according to any one of the preceding claims, **characterised in that** the difluorinated ionic monomer (I) corresponds to any one of the following formulae: 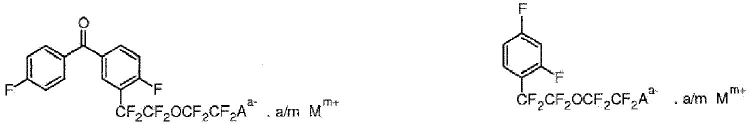 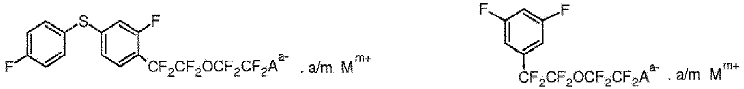 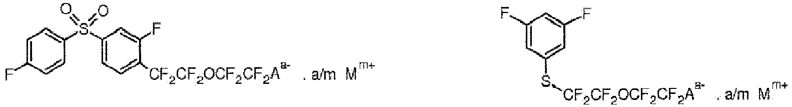 wherein M, A, a and m are as defined in claim 1.

8. lonomer, **characterised in that** it is obtained by the process as defined in any one of claims 1 to 7, and **in that** it comprises at least repeating units RU corresponding to the following formula (II): 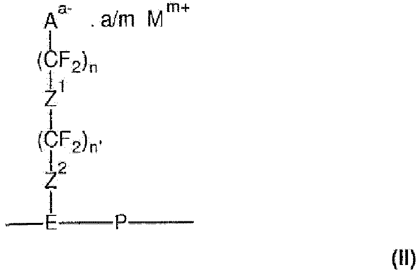 wherein A, n, n', Z¹, Z², E, m, a and M are as defined in any one of claims 1 to 7 and P is an alkylene oxide polymer chain as defined in claim 1.

9. lonomer according to claim 8, **characterised in that** M is an alkali metal cation or an alkaline earth metal cation.

10. lonomer according to either claim 8 or claim 9, **characterised in that** one or more of the following conditions apply:
* n = n'= 2,
* Z¹ is an oxygen atom,
* Z² is a sulfur atom or a single bond.

11. Electrolyte composition, **characterised in that** it comprises at least one ionomer obtained by the process as defined in any one of claims 1 to 7 or as defined in any one of claims 8 to 10.

12. Electrolyte composition according to claim 11, **characterised in that** it further comprises one or more organic solvents.

13. Electrolyte composition according to claim 11 or 12, **characterised in that** it further comprises one or more additives chosen from mineral fillers, alkali metal salts, organic fillers, complexing agents, flame retardants, and a mixture thereof.

14. Electrolyte composition according to claim 11, **characterised in that** it is composed only of said ionomer.

15. Use of an ionomer obtained by the process as defined in any one of claims 1 to 7 or as defined in any one of claims 8 to 10 in the manufacture of an electrolyte for an electrochemical device.

16. Use of an ionomer obtained by the process as defined in any one of claims 1 to 7 or as defined in any one of claims 8 to 10 as an ionic liquid.

17. Use of an ionomer obtained by the process as defined in any one of claims 1 to 7 or as defined in any one of claims 8 to 10 as a constituent of a composite electrode.

18. Electrochemical device comprising at least one negative electrode and at least one positive electrode separated by an electrolyte composition, **characterised in that** the electrolyte composition is as defined in any one of claims 11 to 14.
